(19) Europäisches Patentamt
European Patent Office
Office européen des brevets

(11) EP 3 630 764 B1

(12) EUROPÄISCHE PATENTSCHRIFT

(45) Veröffentlichungstag und Bekanntmachung des Hinweises auf die Patenterteilung:
**18.08.2021 Patentblatt 2021/33**

(21) Anmeldenummer: **18724256.5**

(22) Anmeldetag: **18.05.2018**

(51) Int Cl.:
***C07D 471/22*** *(2006.01)* ***C07D 491/22*** *(2006.01)*
***C07D 493/22*** *(2006.01)* ***C07D 497/22*** *(2006.01)*
***C07D 498/22*** *(2006.01)* ***H01L 51/50*** *(2006.01)*

(86) Internationale Anmeldenummer:
**PCT/EP2018/063031**

(87) Internationale Veröffentlichungsnummer:
**WO 2018/215318 (29.11.2018 Gazette 2018/48)**

# (54) HEXAZYKLISCHE HETEROAROMATISCHE VERBINDUNGEN FÜR ELEKTRONISCHE VORRICHTUNGEN

HEXACYCLIC HETEROAROMATIC COMPOUNDS FOR USE IN ELEKTRONIC DEVICES

COMPOSES HEXACYQULIQUES ET HETEROAROMATIQUES POUR DES DISPOSITIFS ELECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.05.2017 EP 17172234**

(43) Veröffentlichungstag der Anmeldung:
**08.04.2020 Patentblatt 2020/15**

(73) Patentinhaber: **Merck Patent GmbH**
**64293 Darmstadt (DE)**

(72) Erfinder:
- **PARHAM, Amir**
  **60486 Frankfurt am Main (DE)**
- **KROEBER, Jonas**
  **60311 Frankfurt am Main (DE)**
- **JOOSTEN, Dominik**
  **64372 Obert-Ramstadt (DE)**
- **LUDEMANN, Aurélie**
  **60322 Frankfurt am Main (DE)**
- **GROSSMANN, Tobias**
  **64297 Darmstadt (DE)**

(56) Entgegenhaltungen:
**WO-A1-2013/183327 WO-A2-2005/048315**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung beschreibt heteroaromatische Verbindungen, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

In organischen Elektrolumineszenzvorrichtungen (OLEDs) werden als emittierende Materialien häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host-/Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden als Matrixmaterialien für phosphoreszierende Verbindungen unter anderem Indolizinderivate verwendet. Beispielsweise sind in J. Am. Chem. Soc. Vol, 126, No. 51, 2004, 16793-16803, J. Mater. Chem., 2009, 19, 5826-5836, Eur. J. Org. Chem. 2007, 3718-3726, WO 2013/183327 und WO 2005/048315 entsprechende Verbindungen beschrieben. Allerdings umfassen die beschriebenen Indolizinderivate keine weitere Ringstruktur, die an den Fünfring der Indolizinstruktur kondensiert ist.

Generell besteht bei diesen Materialien, beispielsweise für die Verwendung als Matrixmaterialien, Lochleitermaterialien oder Elektronentransportmaterialien noch Verbesserungsbedarf, insbesondere in Bezug auf die Lebensdauer, aber auch in Bezug auf die Effizienz und die Betriebsspannung der Vorrichtung. Ferner sollten die Verbindungen eine hohe Farbreinheit aufweisen.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung, eignen und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung, Verbindungen zur Verfügung zu stellen, die zu hoher Lebensdauer, guter Effizienz und geringer Betriebsspannung führen. Gerade auch die Eigenschaften der Matrixmaterialien, der Lochleitermaterialien oder der Elektronentransportmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs, insbesondere als Matrixmaterial für eine rot phosphoreszierende Verbindung eignen.

Weiterhin sollten die Verbindungen, insbesondere bei ihrem Einsatz als Matrixmaterialien, als Lochleitermaterialien oder als Elektronentransportmaterialien in organischen Elektrolumineszenzvorrichtung zu Vorrichtungen führen, die eine ausgezeichnete Farbreinheit aufweisen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausführungsformen sind daher Gegenstand der vorliegenden Erfindung.

Gegenstand der vorliegenden Erfindung ist daher eine Verbindung, umfassend mindestens eine Struktur gemäß der folgenden Formel (I),

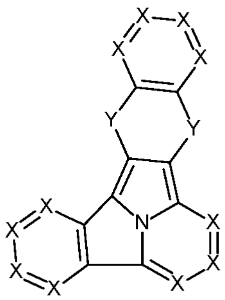

Formel (I)

wobei für die verwendeten Symbole gilt:

Y ist bei jedem Auftreten gleich oder verschieden eine Bindung oder $NR^1$, NAr, O, S, $C(R^1)_2$, $CArR^1$, $C(Ar)_2$, $Si(Ar)_2$, $SiArR^1$ oder $Si(R^1)_2$, wobei mindestens eine Gruppe Y ausgewählt ist aus $NR^1$, NAr, O, S, $C(R^1)_2$, $CArR^1$, $C(Ar)_2$, $Si(Ar)_2$, $SiArR^1$ oder $Si(R^1)_2$, vorzugsweise aus $NR^1$ oder NAr, besonders bevorzugt NAr;

X ist bei jedem Auftreten gleich oder verschieden N oder $CR^1$, vorzugsweise $CR^1$;

Ar ist bei jedem Auftreten gleich oder verschieden ein aromatisches oder heteroaromatisches Ringsystem mit 5 bis 60 aromatischen Ringatomen, das mit einem oder mehreren Resten $R^1$ substituiert sein kann, dabei können zwei Reste Ar, welche an dasselbe C-Atom oder Si-Atom binden, auch durch eine Einfachbindung oder eine Brücke, ausgewählt aus $B(R^1)$, $C(R^1)_2$, $Si(R^1)_2$, C=O, $C=NR^1$, $C=C(R^1)_2$, O, S, S=O, $SO_2$, $N(R^1)$, $P(R^1)$ und $P(=O)R^1$, miteinander verbrückt sein;

$R^1$ ist bei jedem Auftreten gleich oder verschieden H, D, OH, $OR^2$, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder einer Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C≡C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen, die mit einem oder mehreren Resten $R^2$ substituiert sein kann, oder eine Kombination dieser Systeme; dabei können zwei oder mehrere, vorzugsweise benachbarte Reste $R^1$ miteinander ein Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^2$ substituiert sein kann, dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C≡C-, $Si(R^3)_2$, $Ge(R^3)_2$, $Sn(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere, vorzugsweise benachbarte Substituenten $R^2$

auch miteinander ein Ringsystem bilden;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehrere, vorzugsweise benachbarte Substituenten $R^3$ auch miteinander ein Ringsystem bilden.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

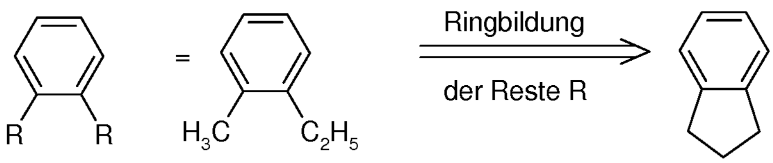


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

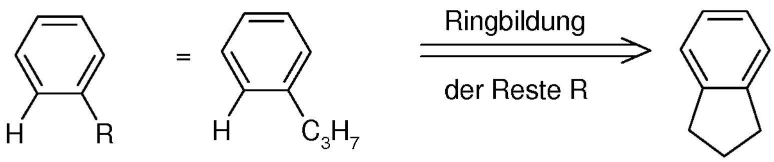


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindestens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 60 C-Atome, vorzugsweise 6 bis 40 C-Atome, besonders bevorzugt 6 bis 30 C-Atome; eine Heteroarylgruppe im Sinne dieser Erfindung enthält 2 bis 60 C-Atome, vorzugsweise 2 bis 40 C-Atome, besonders bevorzugt 2 bis 30 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome, vorzugsweise 6 bis 40 C-Atome, besonders bevorzugt 6 bis 30 C-Atome im Ringsystem. Ein heteroaromatisches Ringsystem im Sinne dieser Erfindung enthält 1 bis 60 C, vorzugsweise 1 bis 40 C-Atome, besonders bevorzugt 1 bis 30 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 bis 60, vorzugsweise 5 - 40 aromatischen Ringatomen, besonders bevorzugt 5 bis 30 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausführungsform der vorliegenden Erfindung stellt eine der Gruppen Y in Formel (I) NAr oder $NR^1$ und eine der Gruppen Y O, S oder $C(R^1)_2$ dar. In einer weiteren Ausführungsform stellt eine der Gruppen Y in Formel (I) NAr oder $NR^1$ und eine der Gruppen Y eine Bindung dar, wobei die Ausführungsform, gemäß der eine der Gruppen Y eine Bindung darstellt gegenüber der Ausführungsform, gemäß der beide Gruppen Y ausgewählt sind aus $NR^1$, NAr, O, S, $C(R^1)_2$, $CArR^1$, $C(Ar)_2$, $Si(Ar)_2$, $SiArR^1$ oder $Si(R^1)_2$, bevorzugt ist.

In einer bevorzugten Ausgestaltung können die erfindungsgemäßen Verbindungen mindestens eine Struktur der Formel (IIa) oder (IIb) umfassen,

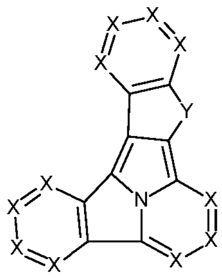

Formel (IIa)

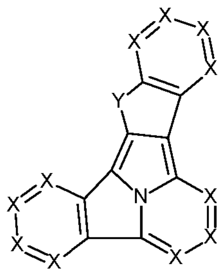

Formel (IIb)

wobei die verwendeten Symbole Y und X die zuvor, insbesondere für Formel (I) genannte Bedeutung haben.

Vorzugsweise können die erfindungsgemäßen Verbindungen mindestens eine Struktur der Formel (IIIa) oder (IIIb) umfassen,

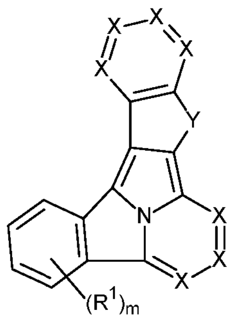

Formel (IIIa)

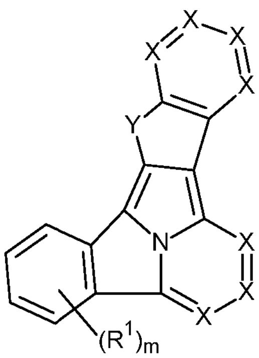

Formel (IIIb)

wobei die Symbole $R^1$, Y und X die zuvor, insbesondere für Formel (I) genannte Bedeutung aufweisen und m 0, 1, 2, 3, oder 4, vorzugsweise 0, 1, 2 oder 3, besonders bevorzugt 0, 1 oder 2 ist.

Vorzugsweise können die erfindungsgemäßen Verbindungen mindestens eine Struktur der Formel (IVa) oder (IVb) umfassen,

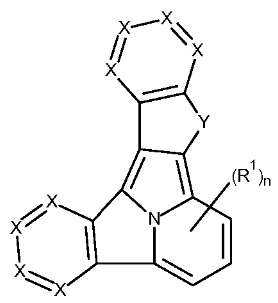

Formel (IVa)

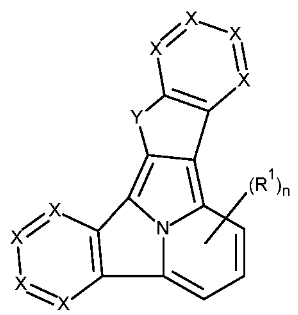

Formel (IVb)

wobei die Symbole $R^1$, Y und X die zuvor, insbesondere für Formel (I) genannte Bedeutung aufweisen und n 0, 1, 2 oder 3, bevorzugt 0, 1 oder 2, besonders bevorzugt 0 oder 1 ist.

Gemäß einer weiteren bevorzugten Ausführungsform können die erfindungsgemäßen Verbindungen mindestens eine Struktur der Formel (Va) oder (Vb) umfassen,

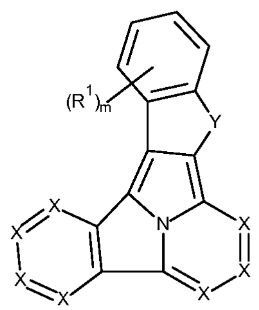

Formel (Va)

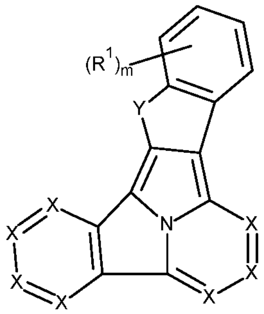

Formel (Vb)

wobei die Symbole $R^1$, Y und X die zuvor, insbesondere für Formel (I) genannte Bedeutung aufweisen und m 0, 1, 2, 3, oder 4, vorzugsweise 0, 1, 2 oder 3, besonders bevorzugt 0, 1 oder 2 ist.

Weiterhin kann vorgesehen sein, dass in Strukturen gemäß Formel (I), (IIa), (IIb), (Iila), (IIIb), (IVa), (IVb), (Va) und/oder (Vb) mindestens eine der Gruppen Y beziehungsweise die Gruppe Y NAr oder $NR^1$, vorzugsweise NAr ist und die in para-Stellung zu dieser Gruppe Y stehende Gruppe X des Sechsrings für eine Gruppe $CR^1$ steht, wobei der Rest $R^1$ in dieser Gruppe $CR^1$ ungleich H oder D ist und vorzugsweise für ein aromatisches oder heteroaromatisches Ringsystem mit 5 bis 40 aromatischen Ringatomen steht, das jeweils durch einen oder mehrere Reste $R^2$ substituiert sein kann.

Ferner kann vorgesehen sein, dass in Strukturen gemäß Formel (I), (IIa), (IIb), (IIIa), (IIIb), (IVa), (IVb), (Va) und/oder (Vb) die in para-Stellung zum Stickstoffatom stehende Gruppe X des Stickstoff-enthaltenden Sechsrings für eine Gruppe $CR^1$ steht, wobei der Rest $R^1$ in dieser Gruppe $CR^1$ ungleich H oder D ist und vorzugsweise für ein aromatisches oder heteroaromatisches Ringsystem mit 5 bis 40 aromatischen Ringatomen steht, das jeweils durch einen oder mehrere Reste $R^2$ substituiert sein kann.

Ferner sind Verbindungen mit Strukturen bevorzugt, in denen höchstens zwei Gruppen X pro Ring für N stehen und bevorzugt mindestens eine, besonders bevorzugt mindestens zwei der Gruppen X pro Ring ausgewählt sind aus C-H und C-D.

Darüber hinaus sind Verbindungen mit Strukturen gemäß Formel (I), (IIa), (IIb), (IIIa), (IIIb), (IVa), (IVb), (Va) und/oder (Vb) bevorzugt, in denen nicht mehr als vier, vorzugsweise nicht mehr als zwei Gruppen X für N stehen, und besonders bevorzugt alle Gruppen X für $CR^1$ stehen, wobei vorzugsweise höchstens vier, besonders bevorzugt höchstens drei und speziell bevorzugt höchstens zwei der Gruppen $CR^1$, für die X steht, ungleich der Gruppe CH ist.

Vorzugsweise können die erfindungsgemäßen Verbindungen mindestens eine Struktur der Formel (VIa) oder (VIb) umfassen,

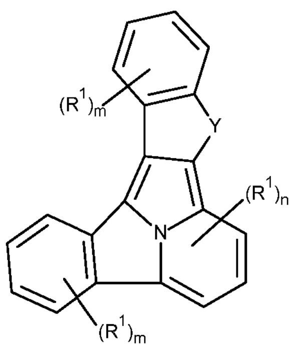

Formel (VIa)

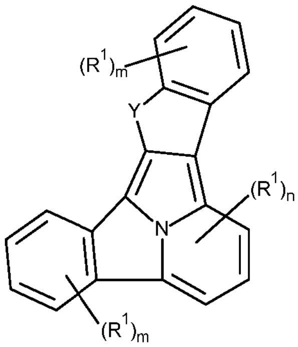

Formel (VIb)

wobei die Symbole $R^1$ und Y die zuvor, insbesondere für Formel (I) genannte Bedeutung aufweisen, m gleich oder verschieden bei jedem Auftreten 0, 1, 2, 3, oder 4, vorzugsweise 0, 1, 2 oder 3, besonders bevorzugt 0, 1 oder 2 und n 0, 1, 2 oder 3, bevorzugt 0, 1 oder 2, besonders bevorzugt 0 oder 1 ist.

Ferner kann vorgesehen sein, dass in Strukturen der Formeln (VIa) und/oder (VIb) die Summe der Indices m und n höchstens 6, vorzugsweise höchstens 4 und besonders bevorzugt höchstens 2 beträgt.

Ferner kann vorgesehen sein, dass in den Formeln (I), (IIa), (IIb), (IIc), (IId), (Iila), (IIIb), (IVa), (IVb), (Va), (Vb), (VIa) und/oder (VIb) genau ein Rest $R^1$ oder genau zwei Reste $R^1$ vorhanden sind, der beziehungsweise die ungleich H oder D ist/sind, so dass die anderen Gruppen X für N, CH oder CD stehen, beziehungsweise die Summe der Indices m und n genau eins beziehungsweise zwei ist. Vorzugsweise ist zumindest einer dieser Reste $R^1$ an den Stickstoff enthaltenden aromatischen Sechsring in diesen Formeln gebunden. Falls die Gruppe Y durch einen Rest NAr darstellbar ist, kann vorzugsweise zumindest einer dieser Reste $R^1$ an den aromatischen Sechsring gebunden sein, der an die Gruppe Y gebunden ist. Dieser Substituent $R^1$ steht vorzugsweise in para-Position zum Stickstoffatom des Sechsrings oder zur Bindungsstelle der Gruppe Y.

In einer weiterhin bevorzugten Ausführungsform können die erfindungsgemäßen Verbindungen mindestens eine Struktur der Formel (VIIa) oder (VIIb) aufweisen,

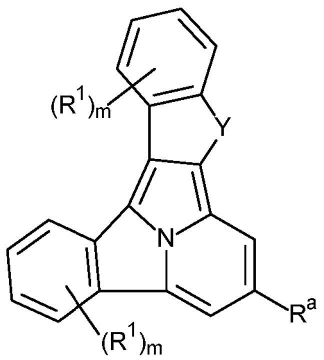

Formel (VIIa)

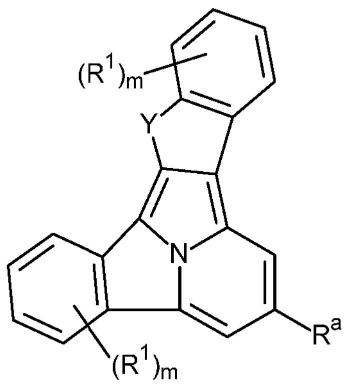

Formel (VIIb)

wobei die verwendeten Symbole $R^1$ und Y die zuvor, insbesondere für Formel (I) genannte Bedeutung aufweisen, wobei die Symbole $R^1$ und Y die zuvor, insbesondere für Formel (I) genannte Bedeutung aufweisen, $R^a$ OH, $OR^2$, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder einer Alkenylgruppe mit 2 bis 40 C-Atomen, die jeweils mit einem oder mehreren Resten $R^2$ substituiert sein kann, wobei eine oder mehrere nicht benachbarte $CH_2$-Gruppen durch $-R^2C=CR^{2-}$, -C≡C-, $Si(R^2)_{2,}$ $Ge(R^2)_2$, $Sn(R^2)_2$, C=O, C=S, C=Se, $C=NR^2$, -C(=O)O-, $-C(=O)NR^{2}$-, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen ist, die mit einem oder mehreren Resten $R^2$ substituiert sein kann, oder eine Kombination dieser Systeme; dabei kann $R^a$ mit einem oder mehreren, vorzugsweise benachbarten Resten $R^1$ ein Ringsystem bilden; m ist gleich oder verschieden bei jedem Auftreten 0, 1, 2, 3, oder 4, vorzugsweise 0, 1, 2 oder 3, besonders bevorzugt 0, 1 oder 2, wobei $R^2$ die zuvor, insbesondere für Formel (I) genannte Bedeutung aufweist. Dabei steht Y bevorzugt für NAr.

Gemäß einer weiteren bevorzugten Ausführungsform können die erfindungsgemäßen Verbindungen mindestens eine Struktur der Formel (VIIIa) oder (VIIIb) umfassen,

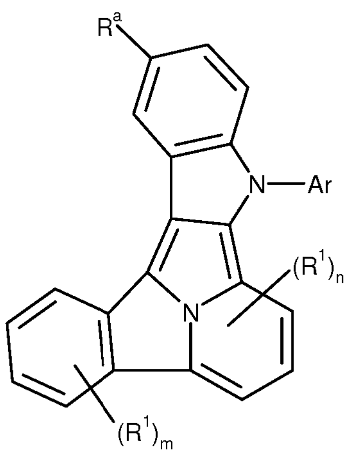

Formel (VIIIa)

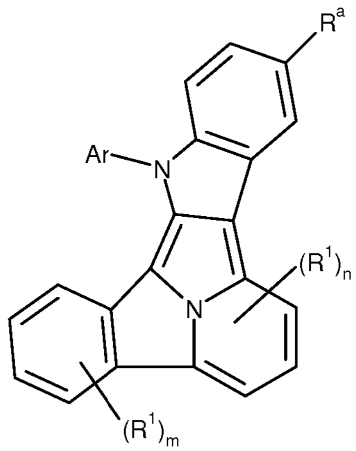

Formel (VIIIb)

wobei die Symbole $R^1$ und Ar die zuvor, insbesondere für Formel (I) genannte Bedeutung aufweisen, $R^a$ die zuvor, insbesondere für Formel (VIIa) oder (VIIb) genannte Bedeutung aufweist, m 0, 1, 2, 3, oder 4, vorzugsweise 0, 1, 2 oder 3, besonders bevorzugt 0, 1 oder 2 und n 0, 1, 2 oder 3, bevorzugt 0, 1 oder 2, besonders bevorzugt 0 oder 1 ist.

Vorzugsweise können die erfindungsgemäßen Verbindungen mindestens eine Struktur der Formel (IXa) oder (IXb) aufweisen,

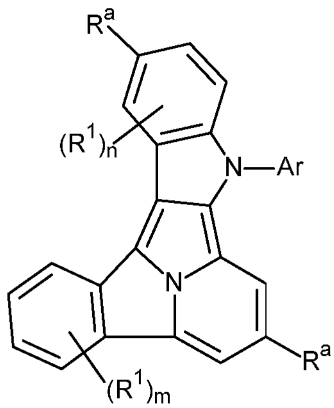

Formel (IXa)

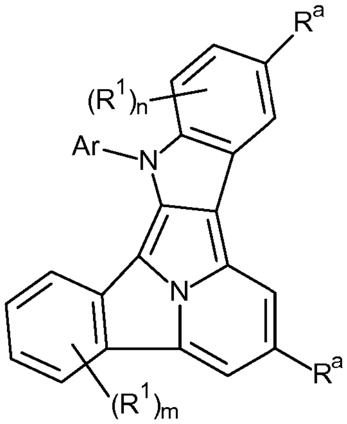

Formel (IXb)

wobei die verwendeten Symbole $R^1$ und Ar die zuvor, insbesondere für Formel (I) genannte Bedeutung aufweisen, $R^a$ die zuvor, insbesondere für Formel (VIIa) oder (VIIb) genannte Bedeutung aufweist, m 0, 1, 2, 3, oder 4, vorzugsweise

0, 1, 2 oder 3, besonders bevorzugt 0, 1 oder 2 und n 0, 1, 2 oder 3, bevorzugt 0, 1 oder 2, besonders bevorzugt 0 oder 1, ganz besonders bevorzugt 0 ist.

Ferner kann vorgesehen sein, dass in Strukturen der Formeln (VIIa), (VIIb), (VIIIa), (VIIIb), (IXa) und/oder (IXb) die Summe der Indices m und n höchstens 6, vorzugsweise höchstens 4 und besonders bevorzugt höchstens 2 beträgt. Speziell bevorzugt ist die Summe der Indices m und n in Strukturen der Formeln (VIIa), (VIIb), (VIIIa), (VIIIb), (IXa) und/oder (IXb) 0, so dass diese Strukturen außer der Gruppe $R^a$ keine weiteren Substituenten $R^1$ aufweist.

Vorzugsweise steht der Rest $R^a$ für ein aromatisches oder heteroaromatisches Ringsystem mit 5 bis 24 aromatischen Ringatomen, vorzugsweise mit 6 bis 13 aromatischen Ringatomen, das jeweils mit einem oder mehreren Resten $R^2$ substituiert sein kann, bevorzugt aber unsubstituiert ist. Zu den bevorzugten Gruppen, für die der Rest $R^a$ stehen kann, gehören insbesondere Phenyl, ortho-, meta- oder para-Biphenyl, Terphenyl, insbesondere verzweigtes Terphenyl, Quaterphenyl, insbesondere verzweigtes Quaterphenyl, 1- oder 2-Naphthyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, 1-, 2-, 3-, 4- oder N-Carbazolyl und Indenocarbazolyl, die jeweils durch einen oder mehrere Reste $R^2$ substituiert sein können, bevorzugt aber unsubstituiert sind

In einer weiteren Ausführungsform umfassen die zuvor genannten Strukturen der Formeln (I), (IIa), (IIb), (IIc), (IId), (IIIa), (IIIb), (IVa), (IVb), (Va), (Vb), (VIa) und/oder (VIb) keine Substituenten am Grundgerüst, so dass X für N oder CH beziehungsweise CD steht, besonders bevorzugt für CH, oder die Summe der Indices n und m 0 ist.

In einer weiterhin bevorzugten Ausführungsform können die erfindungsgemäßen Verbindungen mindestens eine Struktur der Formel (Xa) oder (Xb) umfassen,

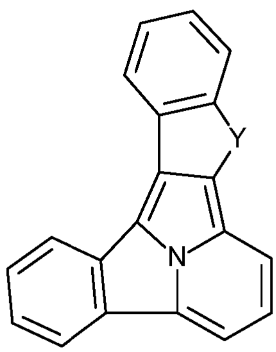

Formel (Xa)

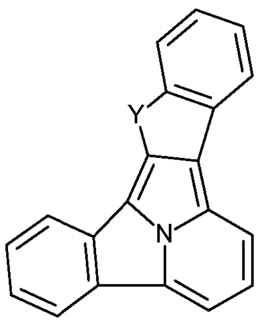

Formel (Xb)

wobei das Symbol Y die zuvor, insbesondere für Formel (I) genannte Bedeutung aufweist und vorzugsweise für $NR^1$ oder NAr, besonders bevorzugt für NAr steht.

Weiterhin kann vorgesehen sein, dass die Substituenten $R^1$ und/oder $R^a$ des heteroaromatischen Ringssystems gemäß den Formeln (I), (IIa), (IIb), (IIc), (IId), (IIIa), (IIIb), (IVa), (IVb), (Va), (Vb), (VIa), (VIb), (VIIa), (VIIb), (VIIIa), (VIIIb), (IXa) und/oder (IXb) mit den Ringatomen des heteroaromatischen Ringssystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können.

Gemäß einer bevorzugten Ausgestaltung sind erfindungsgemäße Verbindungen durch Strukturen der Formel (I), (IIa), (IIb), (IIc), (IId), (lila), (IIIb), (IVa), (IVb), (Va), (Vb), (VIa), (VIb), (VIIa), (VIIb), (VIIIa), (VIIIb), (IXa), (IXb), (Xa)

und/oder (Xb) darstellbar. Vorzugsweise weisen Verbindungen, umfassend Strukturen gemäß Formel (I), (IIa), (IIb), (IIc), (IId), (IIIa), (IIIb), (IVa), (IVb), (Va), (Vb), (VIa), (VIb), (VIIa), (VIIb), (VIIIa), (VIIIb), (IXa), (IXb), (Xa) und/oder (Xb), ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Falls eine erfindungsgemäße Verbindung eine Gruppe Ar und/oder $Ar^1$ enthält, so ist die aromatische oder heteroaromatische Gruppe des durch das Symbol Ar beziehungsweise $Ar^1$ dargestellten aromatischen oder heteroaromatischen Ringsystems vorzugsweise direkt, d.h. über ein Atom der aromatischen oder heteroaromatischen Gruppe, an das jeweilige Atom der weiteren Gruppe gebunden. Bevorzugt stellt das Symbol Ar beziehungsweise $Ar^1$ eine Aryl- oder Heteroarylgruppe dar, wobei für die weiteren Eigenschaften die zuvor und nachfolgend dargelegten Ausgestaltungen dieser Gruppen gelten.

Gemäß einer weiteren Ausführungsform kann die erfindungsgemäße Verbindung eine Lochtransportgruppe umfassen, wobei vorzugsweise die Gruppe Ar oder eine Gruppe $R^1$, die in einer Gruppe Y enthalten ist, oder eine Gruppe $R^1$, die am Grundgerüst gebunden ist, eine Lochtransportgruppe umfasst, vorzugsweise darstellt. Lochtransportgruppen sind in der Fachwelt bekannt, wobei diese vorzugsweise Triarylamin- oder Carbazolgruppen umfassen.

Vorzugsweise kann vorgesehen sein, dass die Lochtransportgruppe eine Gruppe umfasst, bevorzugt für eine Gruppe steht, die ausgewählt ist aus den Formeln (H-1) bis (H-3),

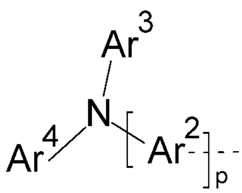

Formel (H-1)

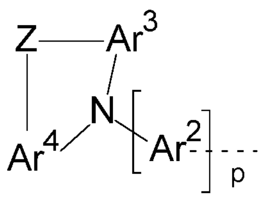

Formel (H-2)

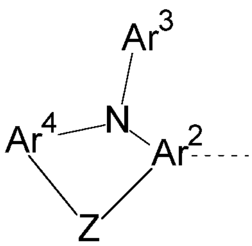

Formel (H-3)

wobei die gestrichelte Bindung die Anbindungsposition an das Stickstoffatom markiert und weiterhin gilt:

| | |
|---|---|
| $Ar^2$, $Ar^3$, $Ar^4$ | ist jeweils unabhängig ein aromatisches Ringsystem mit 6 bis 40 C-Atomen oder ein heteroaromatisches Ringsystem mit 3 bis 40 C-Atomen, welches jeweils durch einen oder mehrere Reste $R^1$ substituiert sein kann; |
| Z | steht für $C(R^1)_2$, $Si(R^1)_2$, C=O, N-$Ar^1$, $BR^1$, $PR^1$, $PO(R^1)$, SO, $SO_2$, Se, O oder S, vorzugsweise $C(R^1)_2$, N-$Ar^1$, O oder S, wobei die Symbole $Ar^1$ und $R^1$ die zuvor, insbesondere für Formel (I) genannte Bedeutung aufweisen, wobei das Vorhandensein einer N-N-Bindung vorzugsweise ausgeschlossen ist, so dass im Falle Y = NAr der Index p = 1 ist. |

Demgemäß kann die Gruppe Ar einen Rest der Formeln (H-1), (H-2) und/oder (H-3) umfassen, bevorzugt für einen Rest der Formeln (H-1), (H-2) oder (H-3) stehen.

Weiterhin kann vorgesehen sein, dass die Gruppe Ar eine Gruppe umfasst, bevorzugt für eine Gruppe steht, die ausgewählt ist aus den Formeln (H-4) bis (H-26),

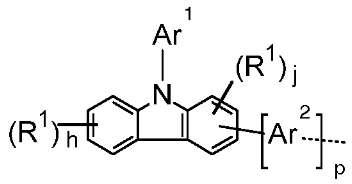

Formel (H-4)

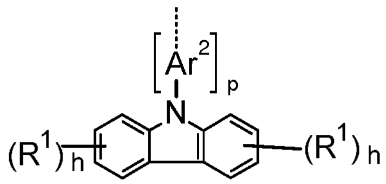

Formel (H-5)

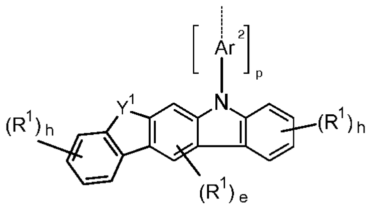

Formel (H-6)

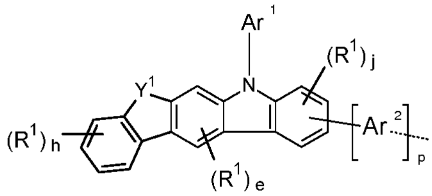

Formel (H-7)

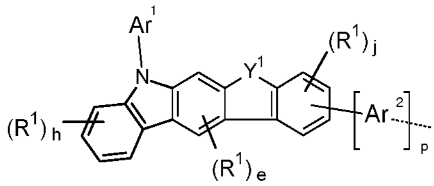

Formel (H-8)

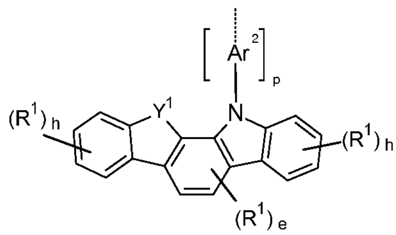

Formel (H-9)

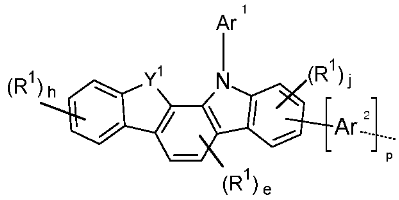

Formel (H-10)

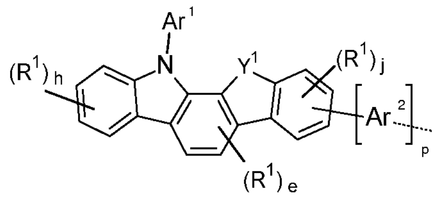

Formel (H-11)

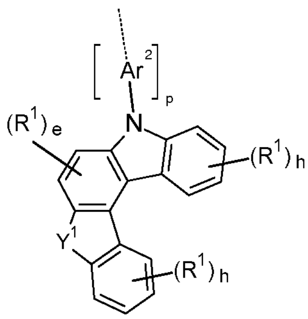

Formel (H-12)

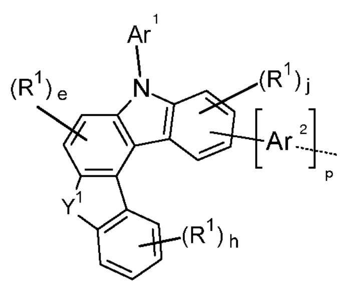

Formel (H-13)

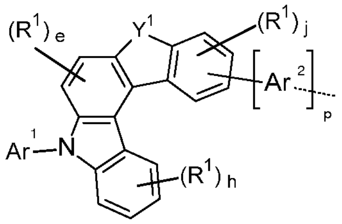

Formel (H-14)

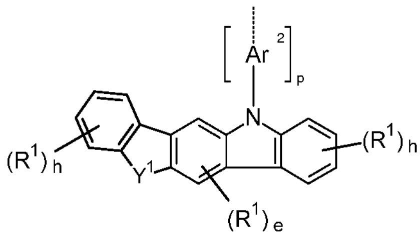

Formel (H-15)

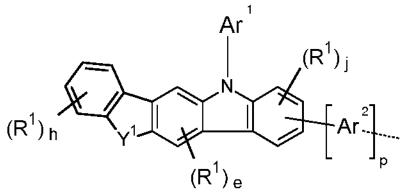

Formel (H-16)

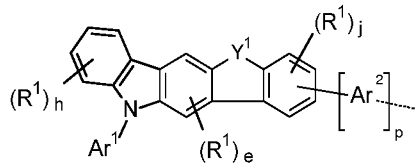

Formel (H-17)

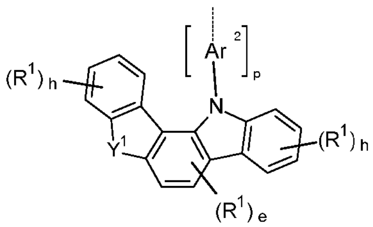

Formel (H-18)

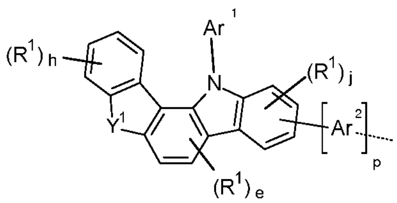

Formel (H-19)

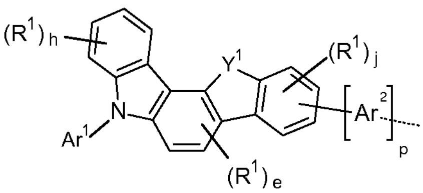

Formel (H-20)

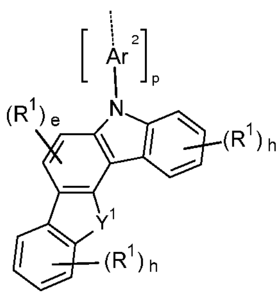

Formel (H-21)

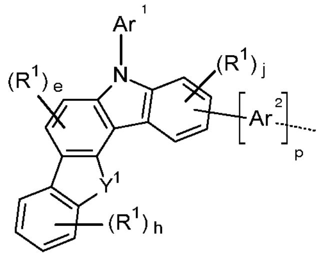

Formel (H-22)

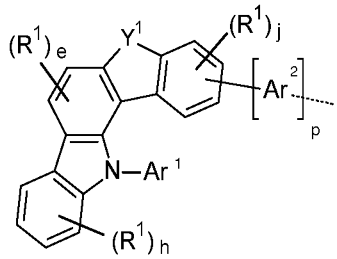

Formel (H-23)

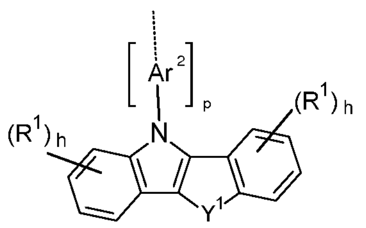

Formel (H-24)

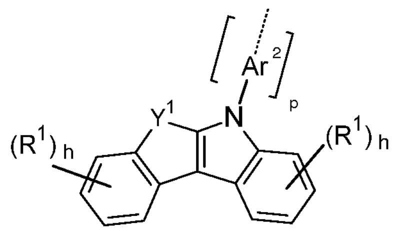

Formel (H-25)

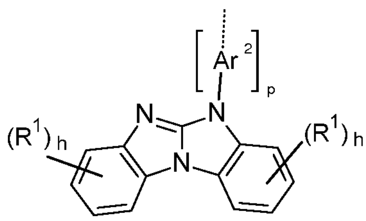

Formel (H-26)

wobei $Y^1$ O, S, $C(R^1)_2$ oder $NAr^1$ darstellt, die gestrichelte Bindung die Anbindungsposition an das Stickstoffatom markiert, e 0, 1 oder 2 ist, j 0, 1, 2 oder 3 ist, h gleich oder verschieden bei jedem Auftreten 0, 1, 2, 3 oder 4 ist, p 0 oder 1 ist, $Ar^1$ und $R^1$ die zuvor, insbesondere für Formel (I) und $Ar^2$ die zuvor, insbesondere für Formel (H-1) oder (H-2) genannte Bedeutung aufweist, wobei das Vorhandensein einer N-N-Bindung vorzugsweise ausgeschlossen ist, so dass im Falle Y = NAr in den Formeln (H-5), (H-6), (H-9), (H-12), (H-15), (H-18), (H-21), (H-24), (H-25) und (H-26) der Index p = 1 ist.

Die zuvor dargelegten Lochtransportgruppen der Formeln (H-1) bis (H-26) stellen bevorzugte Reste $R^1$ gemäß Formel (I) oder bevorzugte Ausführungsformen dieser Formel dar, wobei in diesem Fall die in den Formeln (H-1) bis (H-26) dargelegten Gruppen $R^1$ durch Reste $R^2$ zu ersetzen sind. Die Gruppen Z, $Ar^2$, $Ar^3$, $Ar^4$ können im Fall, dass der Reste $R^1$ gemäß Formel (I) eine Lochtransportgruppe darstellt, Reste $R^2$ als Substituenten anstatt den genannten Resten $R^1$ aufweisen. Das Vorhandensein einer N-N-Bindung ist vorzugsweise ausgeschlossen, so dass im Falle Y = $NR^1$ in den Formeln (H-1), (H-2), (H-5), (H-6), (H-9), (H-12), (H-15), (H-18), (H-21), (H-24), (H-25) und (H-26) der Index p = 1 ist.

Aus der obigen Formulierung ist ersichtlich, dass, falls der Index p = 0 ist, die entsprechende Gruppe $Ar^2$ nicht vorhanden ist und eine Bindung gebildet wird.

Bevorzugt kann die Gruppe $Ar^2$ mit dem aromatischen oder heteroaromatischen Rest oder dem Stickstoffatom, an den die Gruppe $Ar^2$ gemäß den Formeln (H-1) bis (H-26) gebunden sein kann, eine durchgängige Konjugation ausbilden.

In einer weiteren bevorzugten Ausführungsform der Erfindung steht $Ar^2$ für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formel (I) genannte Bedeutung aufweisen kann. Besonders bevorzugt steht $Ar^2$ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^1$ substituiert sein kann, bevorzugt aber unsubstituiert ist, wobei $R^1$ die zuvor, insbesondere für Formel (I) genannte Bedeutung aufweisen kann.

Weiterhin bevorzugt steht das unter anderem in Formeln (H-1) bis (H-26) dargelegte Symbol $Ar^2$ für einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formeln (H-1) bis (H-26) dargelegte Gruppe $Ar^2$ ein aromatisches oder heteroaromatisches Ringsystem mit höchstens zwei kondensierten aromatischen und/oder heteroaromatischen 6-Ringen, vorzugsweise kein kondensiertes aromatisches oder heteroaromatisches Ringsystem mit kondensierten 6-Ringen umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Ferner kann vorgesehen sein, dass die unter anderem in Formeln (H-1) bis (H-26) dargelegte Gruppe $Ar^2$ höchstens 1 Stickstoffatom, bevorzugt höchstens 2 Heteroatome, insbesondere bevorzugt höchstens ein Heteroatom und besonders bevorzugt kein Heteroatom aufweist.

In einer weiteren bevorzugten Ausführungsform der Erfindung steht $Ar^3$ und/oder $Ar^4$ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formel (I) dargestellte Bedeutung aufweisen kann.

In einer weiteren bevorzugten Ausführungsform kann die erfindungsgemäße Verbindung eine Elektronentransportgruppe umfassen, wobei vorzugsweise die Gruppe Ar oder eine Gruppe $R^1$, die in einer Gruppe Y enthalten ist, oder eine Gruppe $R^1$, die am Grundgerüst gebunden ist, oder die Gruppe $R^a$ eine Elektronentransportgruppe umfasst, vorzugsweise darstellt. Elektronentransportgruppen sind in der Fachwelt weithin bekannt und fördern die Fähigkeit von Verbindungen, Elektronen zu transportieren und/oder zu leiten.

Weiterhin zeigen Verbindungen, umfassend mindestens eine Struktur gemäß Formel (I) oder deren bevorzugte Ausführungsformen überraschende Vorteile, bei denen die Gruppe Y mindestens einen Rest Ar oder $R^1$ enthält oder das Grundgerüst mindestens einen Rest $R^1$ oder $R^a$ umfasst, der eine Struktur aus der Gruppe der Pyridine, Pyrimidine, Pyrazine, Pyridazine, Triazine, Chinazoline, Chinoxaline, Chinoline, Isochinoline, Imidazole und/oder Benzimidazole umfasst oder aus den genannten Strukturen ausgewählt ist, wobei Pyrimidine, Triazine und Chinazoline besonders bevorzugt sind.

In einer bevorzugten Ausgestaltung der vorliegenden Erfindung kann vorgesehen sein, dass die Gruppe Y mindestens einen Rest Ar enthält, der für eine Gruppe steht, die durch die Formel (QL) darstellbar ist,

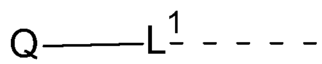

Formel (QL)

worin $L^1$ eine Bindung oder ein aromatisches oder heteroaromatisches Ringsystem mit 5 bis 40, bevorzugt 5 bis 30 aromatischen Ringatomen darstellt, welches durch einen oder mehrere Reste $R^1$ substituiert sein kann, und Q eine Elektronentransportgruppe ist, wobei $R^1$ die zuvor, insbesondere für Formel (I) genannte Bedeutung aufweist.

Bevorzugt kann die Gruppe $L^1$ mit der Gruppe Q und dem Atom, an das die Gruppe $L^1$ gemäß Formel (QL) gebunden ist, eine durchgängige Konjugation ausbilden. Eine durchgängige Konjugation der aromatischen beziehungsweise heteroaromatischen Systeme wird ausgebildet, sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$-hybridisierte Kohlenstoffatom in Position 9 zwar eine Kondensation dieser Ringe unterbindet, jedoch eine Konjugation erfolgen kann, da dieses $sp^3$-hybridisierte Kohlenstoffatom in Position 9 nicht zwingend zwischen der elektronentransportierenden Gruppe Q und dem Atom, an das die Gruppe $L^1$ gemäß Formel (QL) gebunden ist, liegt. Im Gegensatz hierzu kann bei einer Spirobifluorenstruktur eine durchgängige Konjugation ausgebildet werden, falls die Verbindung zwischen der Gruppe Q und dem Atom, an das die Gruppe $L^1$ gemäß Formel (QL) gebunden ist, über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen der Gruppe Q und dem Atom, an das die Gruppe $L^1$ gemäß Formel (QL) gebunden ist, über verschiedene Phenylgruppen der Spirobifluorenstruktur erfolgt, die über das $sp^3$-hybridisierte Kohlenstoffatom in Position 9 verbunden sind, ist die Konjugation unterbrochen.

In einer weiteren bevorzugten Ausführungsform der Erfindung steht $L^1$ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formel (I) genannte Bedeutung aufweisen kann. Besonders bevorzugt steht $L^1$ für eine Bindung oder ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^1$ substituiert sein kann, bevorzugt aber unsubstituiert ist, wobei $R^1$ die zuvor, insbesondere für Formel (I) genannte Bedeutung aufweisen kann.

Weiterhin bevorzugt steht das unter anderem in Formel (QL) dargelegte Symbol $L^1$ gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (QL) dargelegte Gruppe $L^1$ ein aromatisches oder heteroaromatisches Ringsystem mit höchstens zwei kondensierten aromatischen und/oder heteroaromatischen 6-Ringen, vorzugsweise kein kondensiertes aromatisches oder heteroaromatisches Ringsystem mit direkt aneinander kondensierten Sechsringen umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt. Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme $L^1$ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste $R^1$ substituiert sein können, bevorzugt aber unsubstituiert sind.

Ferner kann vorgesehen sein, dass die unter anderem in Formel (QL) dargelegte Gruppe $L^1$ höchstens 1 Stickstoffatom, bevorzugt höchstens 2 Heteroatome, insbesondere bevorzugt höchstens ein Heteroatom und besonders bevorzugt kein Heteroatom aufweist.

Vorzugsweise kann die unter anderem in der Formel (QL) dargelegte Gruppe Q beziehungsweise die Elektronentransportgruppe ausgewählt sein aus Strukturen der Formeln (Q-1), (Q-2), (Q-3), (Q-4), (Q-5), (Q-6), (Q-7), (Q-8), (Q-9) und/oder (Q-10),

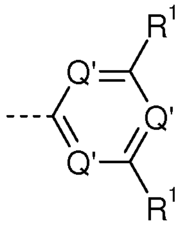

Formel (Q-1)

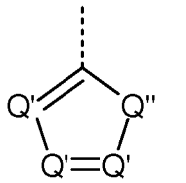

Formel (Q-2)

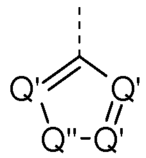

Formel (Q-3)

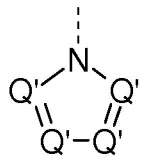

Formel (Q-4)

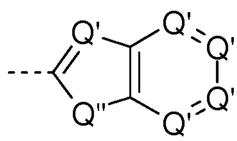

Formel (Q-5)

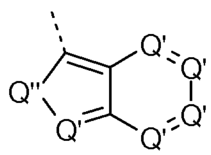

Formel (Q-6)

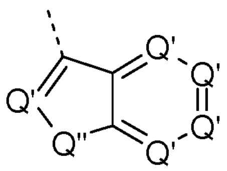

Formel (Q-7)

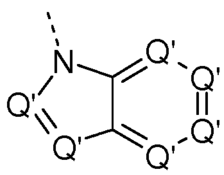

Formel (Q-8)

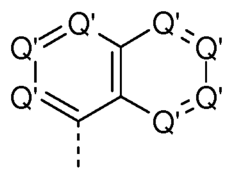

Formel (Q-9)

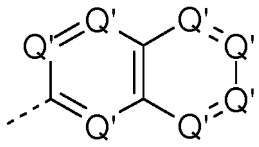

Formel (Q-10),

wobei die gestrichelte Bindung die Anbindungsposition markiert,

Q’ bei jedem Auftreten gleich oder verschieden $CR^1$ oder N darstellt, wobei wenigstens ein Q’ gleich N ist;
Q" $NR^1$, O oder S darstellt; und
$R^1$ wie zuvor, insbesondere in Formel (I) definiert ist.

Weiterhin kann die unter anderem in der Formel (QL) dargelegte Gruppe Q beziehungsweise die Elektronentransportgruppe vorzugsweise ausgewählt sein aus einer Struktur der Formeln (Q-11), (Q-12), (Q-13), (Q-14) und/oder (Q-15),

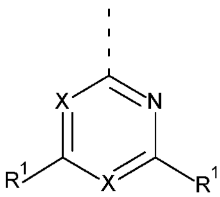

Formel (Q-11)

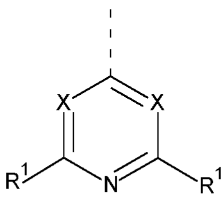

Formel (Q-12)

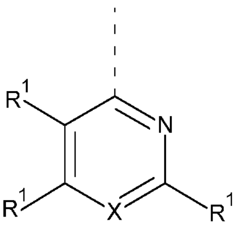

Formel (Q-13)

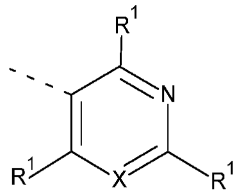

Formel (Q-14)

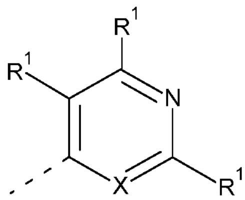

Formel (Q-15)

wobei das Symbol $R^1$ die zuvor unter anderem für Formel (I) genannte Bedeutung aufweist, X N oder $CR^1$ ist und die gestrichelte Bindung die Anbindungsposition markiert, wobei X vorzugsweise ein Stickstoffatom darstellt.

In einer weiteren Ausführungsform kann die unter anderem in der Formel (QL) dargelegte Gruppe Q beziehungsweise die Elektronentransportgruppe ausgewählt sein aus Strukturen der Formeln (Q-16), (Q-17), (Q-18), (Q-19), (Q-20), (Q-21) und/oder (Q-22),

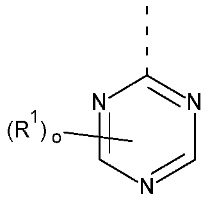

Formel (Q-16)

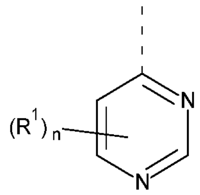

Formel (Q-17)

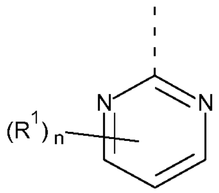

Formel (Q-18)

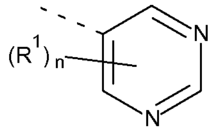

Formel (Q-19)

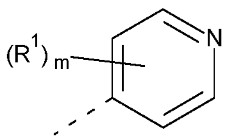

Formel (Q-20)

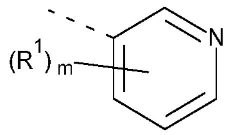

Formel (Q-21)

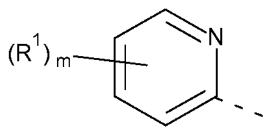

Formel (Q-22)

worin das Symbol $R^1$ die zuvor unter anderem für Formel (I) dargelegte Bedeutung aufweist, die gestrichelte Bindung die Anbindungsposition markiert und m 0, 1, 2, 3 oder 4, vorzugsweise 0, 1 oder 2, n 0, 1, 2 oder 3, vorzugsweise 0, 1 oder 2 und o 0, 1 oder 2, vorzugsweise 1 oder 2 ist. Hierbei sind die Strukturen der Formeln (Q-16), (Q-17), (Q-18) und (Q-19) bevorzugt.

In einer weiteren Ausführungsform kann die unter anderem in der Formel (QL) dargelegte Gruppe Q beziehungsweise die Elektronentransportgruppe ausgewählt sein aus Strukturen der Formeln (Q-23), (Q-24) und/oder (Q-25),

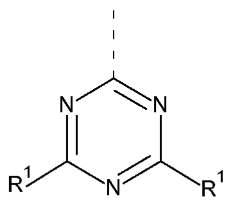

Formel (Q-23)

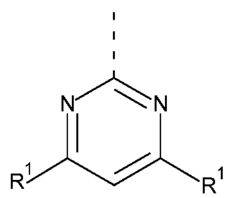

Formel (Q-24)

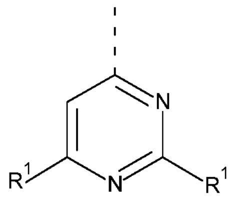

Formel (Q-25)

worin das Symbol $R^1$ die zuvor unter anderem für Formel (I) dargelegte Bedeutung aufweist und die gestrichelte Bindung die Anbindungsposition markiert.

In einer weiteren Ausführungsform kann die unter anderem in der Formel (QL) dargelegte Gruppe Q beziehungsweise die Elektronentransportgruppe ausgewählt sein aus Strukturen der Formeln (Q-26), (Q-27), (Q-28), (Q-29) und/oder (Q-30),

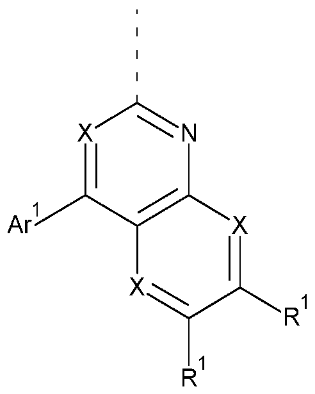

Formel (Q-26)

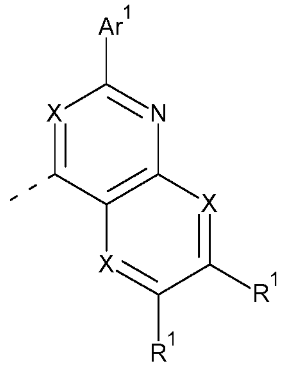

Formel (Q-27)

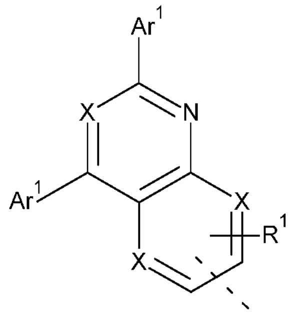

Formel (Q-28)

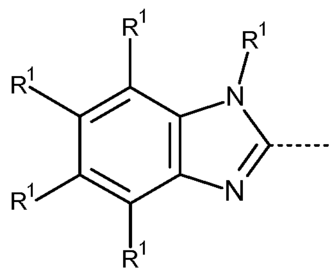

Formel (Q-29)

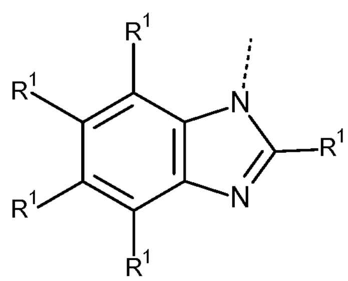

Formel (Q-30)

wobei Symbole X, $Ar^1$ und $R^1$ die zuvor zuvor unter anderem für Formel (I) genannte Bedeutung aufweisen und die gestrichelte Bindung die Anbindungsposition markiert. Vorzugsweise stellt in den Strukturen der Formeln (Q-26), (Q-27) und (Q-28) genau ein X ein Stickstoffatom dar.

Vorzugsweise kann die unter anderem in der Formel (QL) dargelegte Gruppe Q beziehungsweise die Elektronentransportgruppe ausgewählt sein aus Strukturen der Formeln (Q-31), (Q-32), (Q-33), (Q-34), (Q-35), (Q-36), (Q-37), (Q-38), (Q-39), (Q-40), (Q-41), (Q-42), (Q-43) und/oder (Q-44),

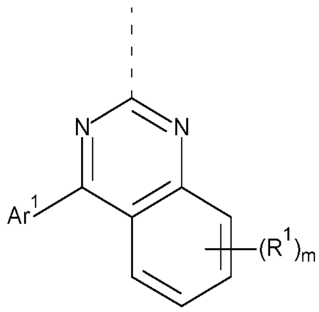

Formel (Q-31)

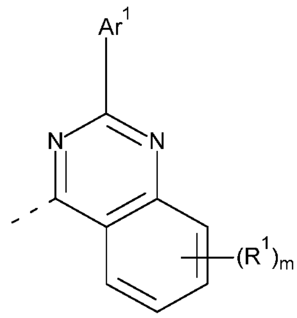

Formel (Q-32)

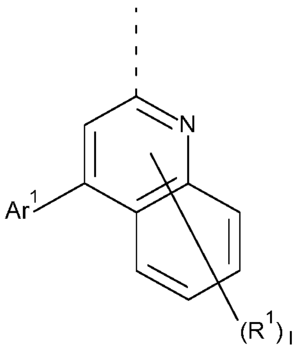

Formel (Q-33)

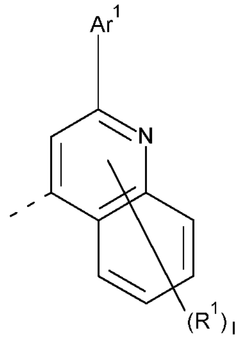

Formel (Q-34)

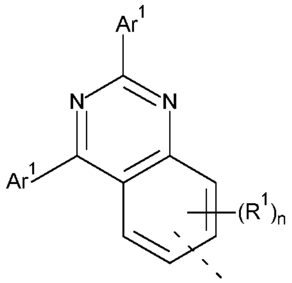

Formel (Q-35)

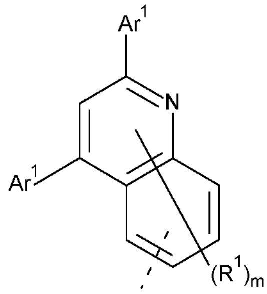

Formel (Q-36)

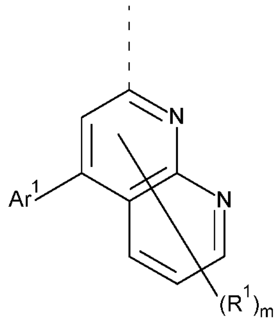

Formel (Q-37)

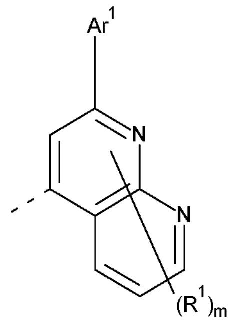

Formel (Q-38)

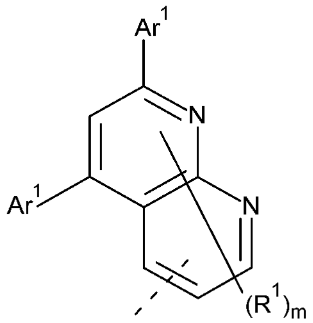

Formel (Q-39)

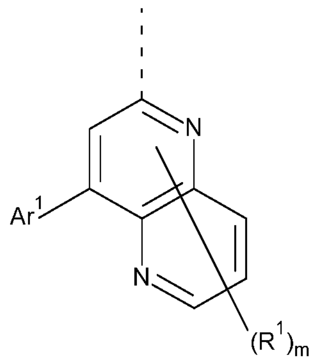

Formel (Q-40)

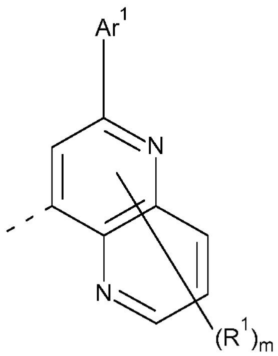

Formel (Q-41)

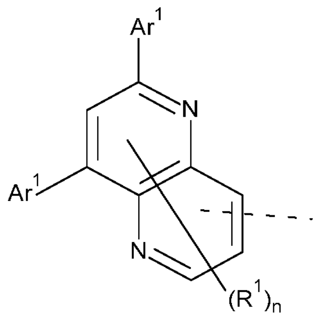

Formel (Q-42)

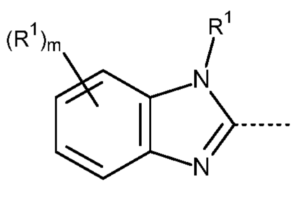

Formel (Q-43)

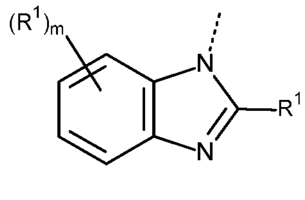

Formel (Q-44)

worin die Symbole $Ar^1$ und $R^1$ die zuvor unter anderem für Formel (I) dargelegte Bedeutung aufweisen, die gestrichelte Bindung die Anbindungsposition markiert und m 0, 1, 2, 3 oder 4, vorzugsweise 0, 1 oder 2, n 0, 1, 2 oder 3, vorzugsweise 0 oder 1, n 0, 1, 2 oder 3, vorzugsweise 0, 1 oder 2 und l 0, 1, 2, 3, 4 oder 5, vorzugsweise 0, 1 oder 2 ist.

Die zuvor dargelegten Elektronentransportgruppen der Formeln (Q-1) bis (Q-44) stellen auch bevorzugte Reste

$R^1$ gemäß Formel (I) oder bevorzugten Ausführungsformen dieser Formel dar, wobei in diesem Fall die in den Formeln (Q-1) bis (Q-44) dargelegten Gruppen $R^1$ durch Reste $R^2$ zu ersetzen sind. Ferner kann der Rest $R^1$ eine oder mehrere Elektronentransportgruppen enthalten, wobei bevorzugte Ausführungsformen entsprechend der Formel (QL) darstellbar sind. In diesem Fall kann die verbindende Gruppe $L^1$ Reste $R^2$ als Substituenten anstatt den genannten Resten $R^1$ aufweisen. Das Vorhandensein einer N-N-Bindung ist vorzugsweise ausgeschlossen.

Ferner kann vorgesehen sein, dass die Gruppe Ar eine Lochtransport- und eine Elektronentransportgruppe umfasst. Je nach Ausgestaltung können bevorzugte Gruppen aus den zuvor dargelegten Formeln (H-1) bis (H-26) beziehungsweise (Q-1) bis (Q-44) gebildet werden, wobei beispielsweise die Gruppen $R^1$ eine Lochtransport- oder eine Elektronentransportgruppe darstellen kann, wobei die in den Formeln (H-1) bis (H-26) beziehungsweise (Q-1) bis (Q-44) dargelegten Reste $R^1$ durch entsprechende Reste $R^2$ ersetzt werden können.

In einer weiteren bevorzugten Ausführungsform der Erfindung steht $Ar^1$ gleich oder verschieden bei jedem Auftreten für ein aromatisches oder heteroaromatisches Ringsystem, vorzugsweise einen Aryl- oder Heteroarylrest mit 5 bis 24 aromatischen Ringatomen, bevorzugt mit 6 bis 18 aromatischen Ringatomen, besonders bevorzugt für ein aromatisches Ringsystem, vorzugsweise einen Arylrest mit 6 bis 12 aromatischen Ringatomen bzw. ein heteroaromatisches Ringsystem, vorzugsweise eine Heteroarylgruppe mit 5 bis 13 aromatischen Ringatomen, das jeweils durch einen oder mehrere Reste $R^2$ substituiert sein kann, bevorzugt aber unsubstituiert ist, wobei $R^2$ die zuvor, insbesondere in Formel (I) dargestellte Bedeutung aufweisen kann. Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist, beispielweise ein C- oder N-Atom der zuvor dargestellten Gruppen (H-1) bis (H-26) oder (Q-26) bis (Q-44).

Mit Vorteil stellt $Ar^1$ in den Formeln (H-1) bis (H-26) oder (Q-26) bis (Q-44) ein aromatisches Ringsystem mit 6 bis 12 aromatischen Ringatomen dar, welches mit einem oder mehreren Resten $R^2$ substituiert sein kann, vorzugsweise aber unsubstituiert ist, wobei $R^2$ die zuvor, insbesondere für Formel (I) dargestellte Bedeutung aufweisen kann.

Bevorzugt bilden die Reste $R^1$ oder $R^2$ in den Formeln (H-1) bis (H-26) oder (Q-1) bis (Q-44) mit den Ringatomen der Arylgruppe oder Heteroarylgruppe $Ar^1$, $Ar^2$, $Ar^3$ und/oder $Ar^4$, an die die Reste $R^1$ oder $R^2$ gebunden sind, kein kondensiertes Ringsystem. Dies schließt die Bildung eines kondensierten Ringsystems mit möglichen Substituenten $R^2$, $R^3$ ein, die an die Reste $R^1$ oder $R^2$ gebunden sein können.

Ferner kann vorgesehen sein, dass die Gruppe Ar, $Ar^1$, $Ar^2$, $Ar^3$ und/oder $Ar^4$ ausgewählt ist aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imidazolyl, Benzimidazolyl, Benzoxazolyl, Benzthiazolyl, 1-, 2-, 3- oder 4-Carbazolyl, 1- oder 2-Naphthyl, Anthracenyl, vorzugsweise 9-Anthracenyl, Phenanthrenyl und/oder Triphenylenyl, die jeweils durch einen oder mehrere Reste $R^2$ substituiert sein können, bevorzugt aber unsubstituiert sind, wobei Phenyl, Spirobifluoren-, Fluoren-, Dibenzofuran-, Dibenzothiophen-, Phenanthren-, Triphenylengruppen besonders bevorzugt sind.

Wenn X für $CR^1$ steht bzw. wenn die aromatische und/oder heteroaromatische Gruppen durch Substituenten $R^1$ und/oder $R^a$ substituiert sind, dann sind diese Substituenten $R^1$ bevorzugt gewählt aus der Gruppe bestehend aus H, D, F, CN, $N(Ar^1)_2$, $C(=O)Ar^1$, $P(=O)(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nichtbenachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die vorzugsweise an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann, wobei die Gruppe $Ar^1$ die zuvor, insbesondere für Formel (I) genannte Bedeutung aufweist. Dabei ist $R^a$ ungleich H oder D.

Besonders bevorzugt sind diese Substituenten $R^1$ und/oder $R^a$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 5 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^2$ substituiert sein kann, bevorzugt aber unsubstituiert ist; dabei können optional zwei Substituenten $R^1$, vorzugsweise die an benachbarte Kohlenstoffatome gebunden sind, ein monocyclisches oder polycyclisches, aliphatisches Ringsystem bilden, das

mit einem oder mehreren Resten $R^2$ substituiert sein kann, bevorzugt aber unsubstituiert ist, wobei $Ar^1$ die zuvor dargelegte Bedeutung aufweisen kann. Dabei ist $R^a$ ungleich H oder D.

Ganz besonders bevorzugt sind die Substituenten $R^1$ und/oder $R^a$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei ist $R^a$ ungleich H oder D.

Weiterhin kann vorgesehen sein, dass in einer Struktur gemäß Formel Formel (I), (IIa), (IIb), (IIc), (IId), (IIIa), (IIIb), (IVa), (IVb), (Va), (Vb), (VIa), (VIb), (VIIa), (VIIb), (VIIIa), (VIIIb), (IXa), (IXb), (Xa) und/oder (Xb) mindestens ein Rest $R^1$ oder $Ar^1$ für eine Gruppe steht, die ausgewählt ist aus den Formeln ($R^1$-1) bis ($R^1$- 92), beziehungsweise in einer Struktur gemäß Formel (H-1) bis (H-26), (Q-1) bis (Q-44) mindestens ein Rest $Ar^1$ oder $R^1$ für eine Gruppe steht, die ausgewählt ist aus den Formeln ($R^1$-1) bis ($R^1$-92),

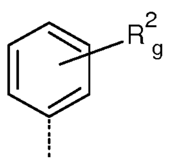

Formel ($R^1$-1)

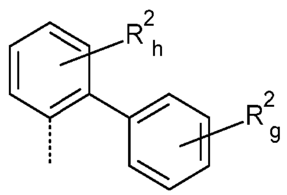

Formel ($R^1$-2)

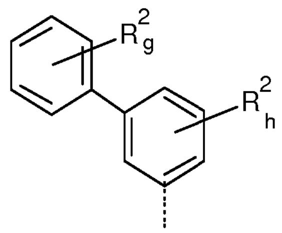

Formel ($R^1$-3)

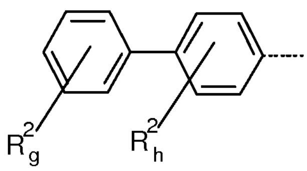

Formel ($R^1$-4)

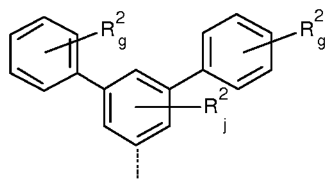

Formel ($R^1$-5)

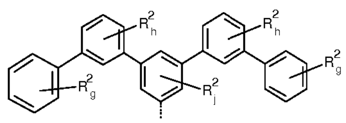

Formel ($R^1$-6)

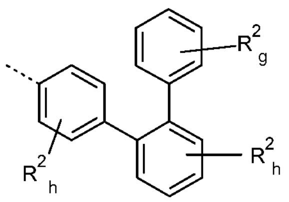

Formel ($R^1$-7)

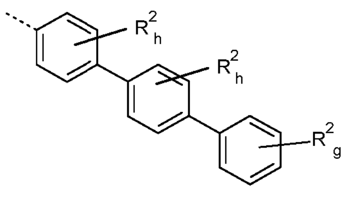

Formel ($R^1$-8)

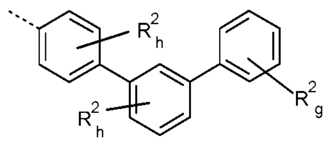

Formel ($R^1$-9)

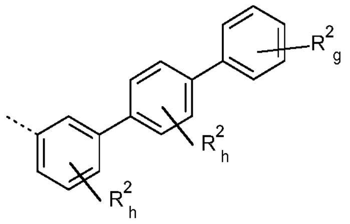

Formel ($R^1$-10)

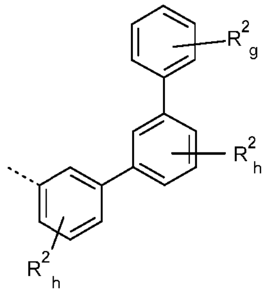

Formel ($R^1$-11)

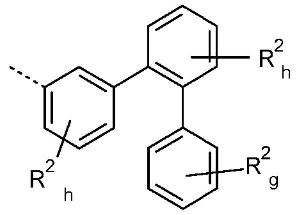

Formel ($R^1$-12)

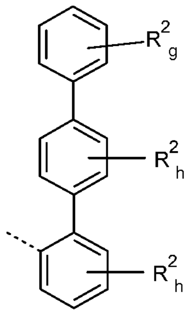

Formel ($R^1$-13)

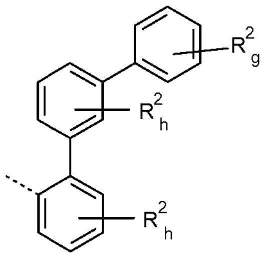

Formel ($R^1$-14)

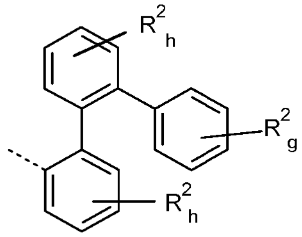

Formel ($R^1$-15)

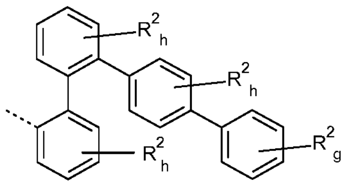

Formel ($R^1$-16)

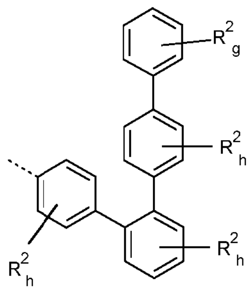

Formel ($R^1$-17)

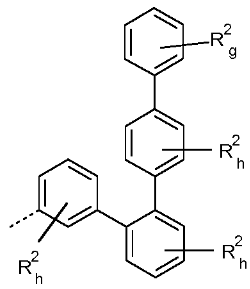

Formel ($R^1$-18)

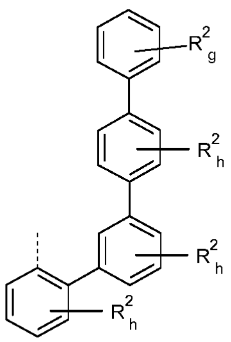

Formel ($R^1$-19)

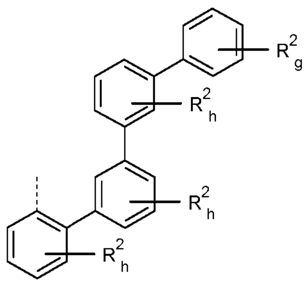

Formel ($R^1$-20)

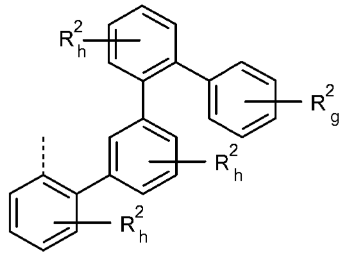

Formel ($R^1$-21)

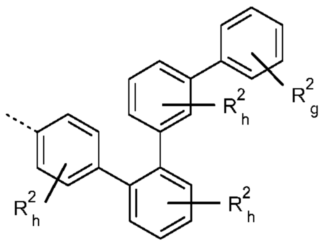

Formel ($R^1$-22)

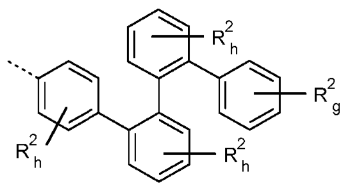

Formel ($R^1$-23)

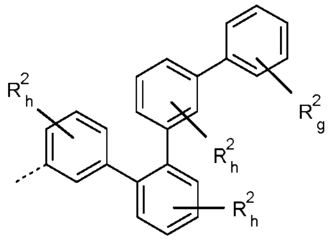

Formel ($R^1$-24)

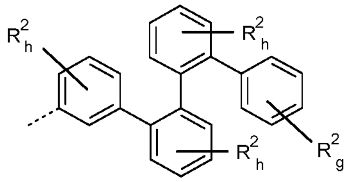

Formel ($R^1$-25)

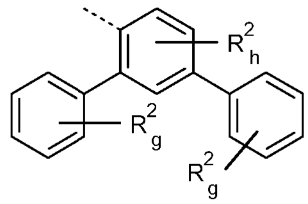

Formel ($R^1$-26)

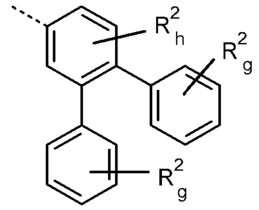

Formel ($R^1$-27)

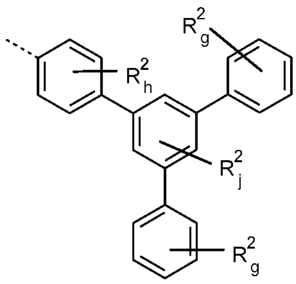

Formel ($R^1$-28)

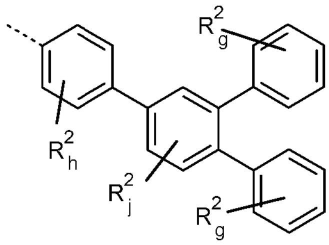

Formel ($R^1$-29)

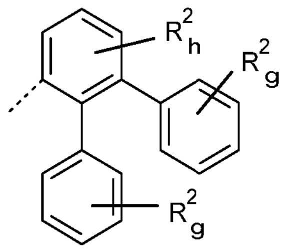

Formel ($R^1$-30)

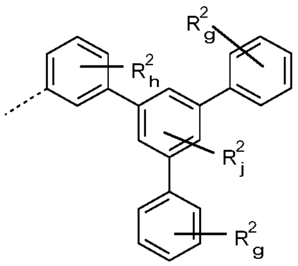

Formel ($R^1$-31)

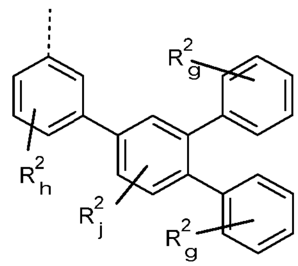

Formel ($R^1$-32)

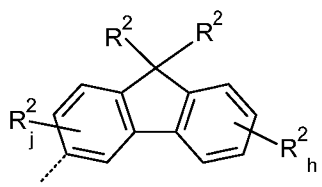

Formel ($R^1$-33)

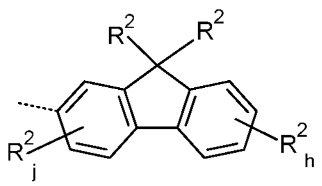

Formel ($R^1$-34)

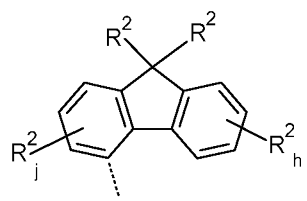

Formel ($R^1$-35)

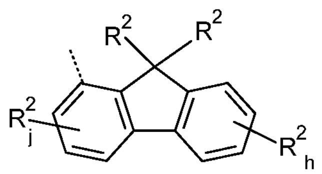

Formel ($R^1$-36)

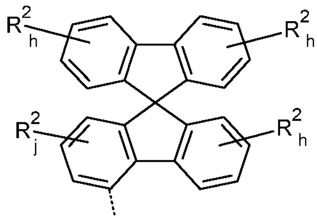
Formel ($R^1$-37)

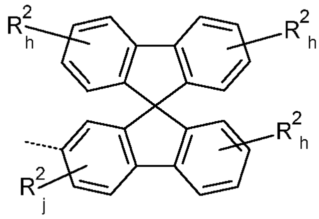
Formel ($R^1$-38)

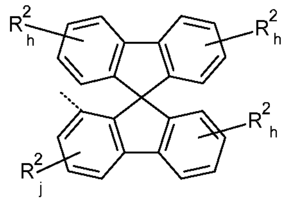
Formel ($R^1$-39)

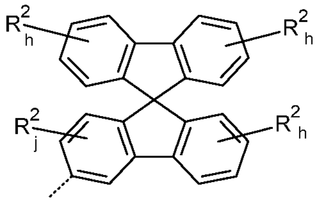
Formel ($R^1$-40)

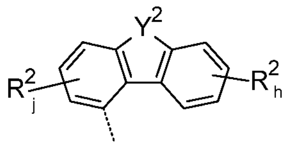
Formel ($R^1$-41)

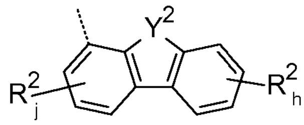
Formel ($R^1$-42)

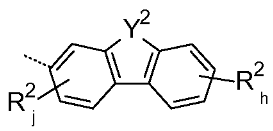
Formel ($R^1$-43)

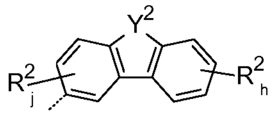
Formel ($R^1$-44)

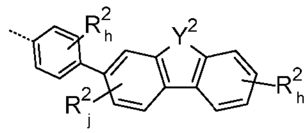
Formel ($R^1$-45)

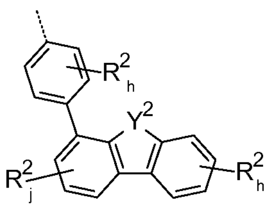
Formel ($R^1$-46)

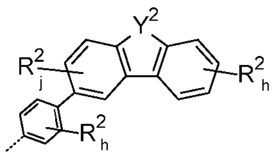
Formel ($R^1$-47)

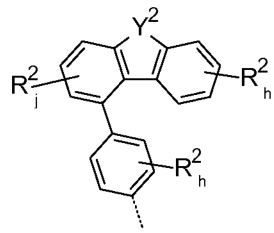
Formel ($R^1$-48)

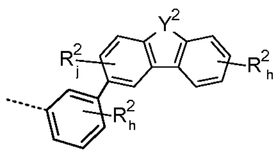
Formel ($R^1$-49)

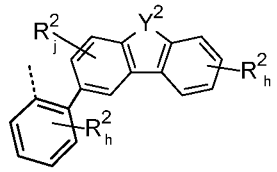
Formel ($R^1$-50)

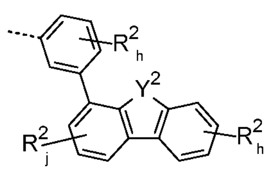
Formel ($R^1$-51)

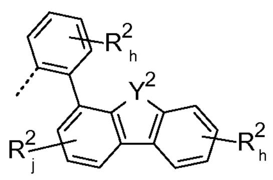
Formel ($R^1$-52)

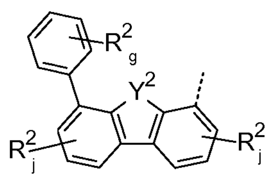
Formel ($R^1$-53)

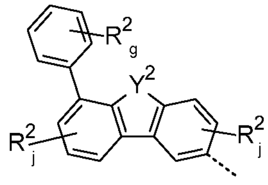
Formel ($R^1$-54)

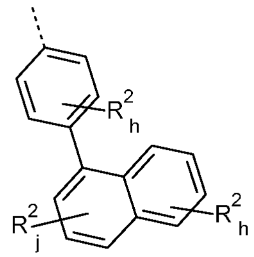

Formel ($R^1$-55)

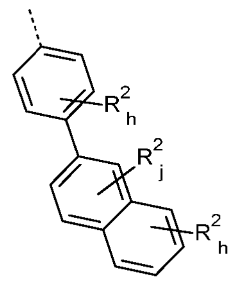

Formel ($R^1$-56)

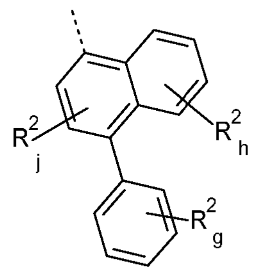

Formel ($R^1$-57)

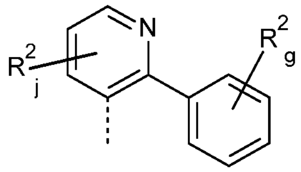

Formel ($R^1$-58)

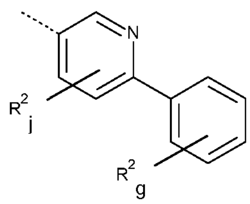

Formel ($R^1$-59)

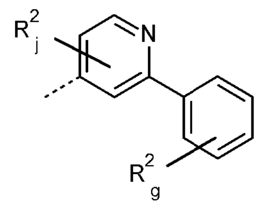

Formel ($R^1$-60)

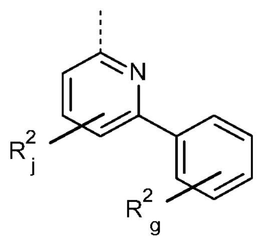

Formel ($R^1$-61)

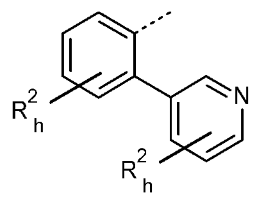

Formel ($R^1$-62)

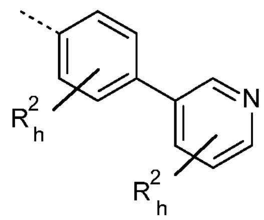

Formel ($R^1$-63)

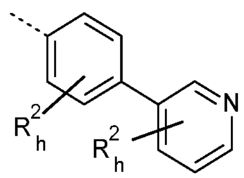

Formel ($R^1$-64)

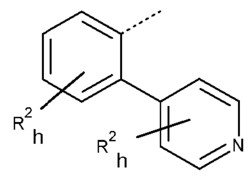

Formel ($R^1$-65)

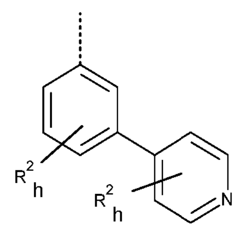

Formel ($R^1$-66)

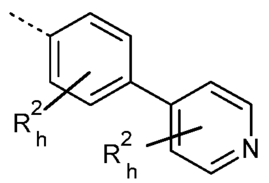

Formel ($R^1$-67)

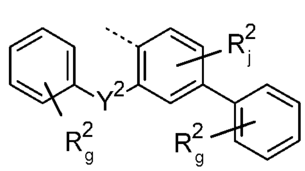

Formel ($R^1$-68)

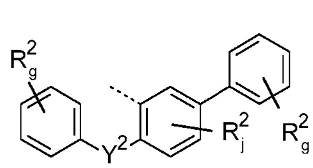

Formel ($R^1$-69)

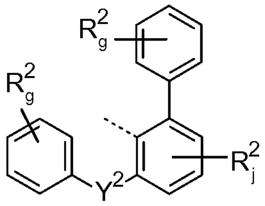

Formel ($R^1$-70)

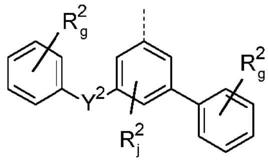

Formel ($R^1$-71)

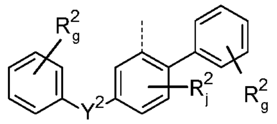

Formel ($R^1$-72)

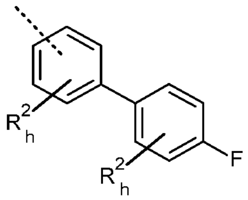

Formel ($R^1$-73)

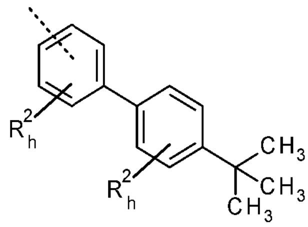

Formel ($R^1$-74)

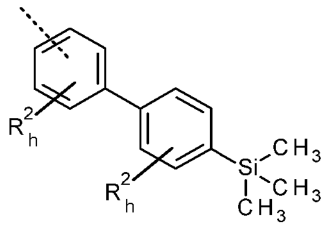

Formel ($R^1$-75)

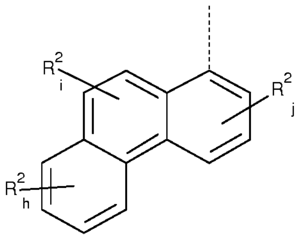

Formel ($R^1$-76)

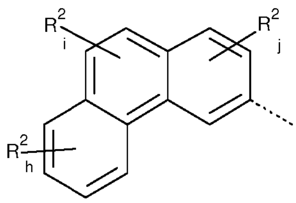

Formel ($R^1$-77)

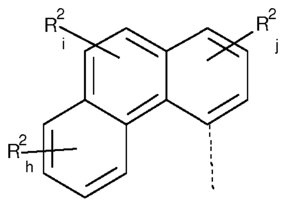

Formel ($R^1$-78)

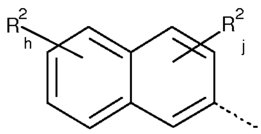

Formel ($R^1$-79)

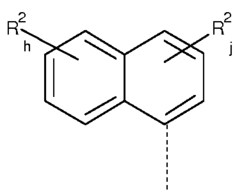

Formel ($R^1$-80)

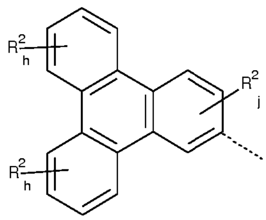

Formel ($R^1$-81)

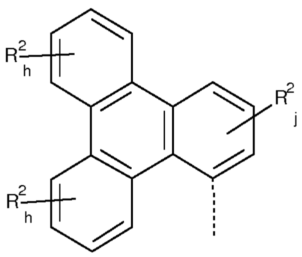

Formel ($R^1$-82)

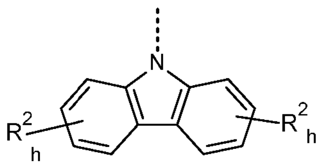

Formel ($R^1$-83)

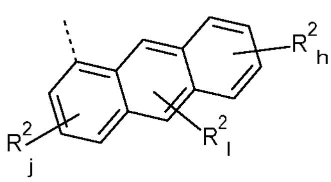

Formel ($R^1$-84)

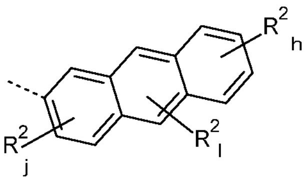
Formel ($R^1$-85)

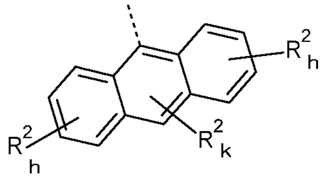
Formel ($R^1$-86)

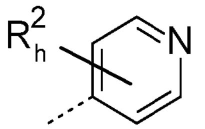
Formel ($R^1$-87)

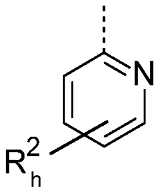
Formel ($R^1$-88)

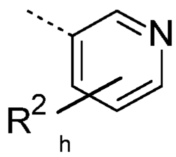
Formel ($R^1$-89)

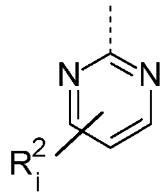
Formel ($R^1$-90)

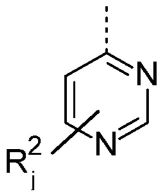
Formel ($R^1$-91)

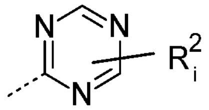
Formel ($R^1$-92)

wobei für die verwendeten Symbole gilt:

- $Y^2$ ist O, S oder $NR^2$, vorzugsweise O oder S;
- k ist bei jedem Auftreten unabhängig 0 oder 1;
- i ist bei jedem Auftreten unabhängig 0, 1 oder 2;
- j ist bei jedem Auftreten unabhängig 0, 1, 2 oder 3;
- h ist bei jedem Auftreten unabhängig 0, 1, 2, 3 oder 4;
- g ist bei jedem Auftreten unabhängig 0, 1, 2, 3, 4 oder 5;
- $R^2$ kann die zuvor genannte, insbesondere für Formel (I) genannte Bedeutung aufweisen;

die gestrichelte Bindung markiert die Anbindungsposition.

Hierbei sind die Gruppen der Formeln $R^1$-1 bis $R^1$-56 bevorzugt, wobei die Gruppen $R^1$-1, $R^1$-3, $R^1$-5, $R^1$-6, $R^1$-15, $R^1$-29, $R^1$-30, $R^1$-31, $R^1$-32, $R^1$-33, $R^1$-38, $R^1$-39, $R^1$-40, $R^1$-41, $R^1$-42, $R^1$-43, $R^1$-44 und/oder $R^1$-45 besonders bevorzugt sind.

Vorzugsweise kann vorgesehen sein, dass die Summe der Indices k, i, j, h und g in den Strukturen der Formel ($R^1$-1) bis ($R^1$-92) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-92) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Die zuvor dargelegten Reste der Formeln ($R^1$-1) bis ($R^1$-92) stellen bevorzugte Reste Ar gemäß Formel (I) beziehungsweise $Ar^3$, $Ar^4$ gemäß Formeln (H-1) bis (H-3) oder bevorzugte Ausführungsformen dieser Formeln dar, wobei in diesem Fall die in den Formeln ($R^1$-1) bis ($R^1$-92) dargelegten Gruppen $R^2$ durch Reste $R^1$ zu ersetzen sind. Die zuvor dargelegten Bevorzugungen hinsichtlich der Formeln ($R^1$-1) bis ($R^1$-92) gelten entsprechend.

Bevorzugt sind Verbindungen, umfassend mindestens eine Struktur der Formeln (H-1) bis (H-26), in denen die Gruppe $Ar^2$ für eine Gruppe steht, die ausgewählt ist aus den Formeln ($L^1$-1) bis ($L^1$-108), und/oder Verbindungen, umfassend Strukturen der Formel (QL), in denen die Gruppe $L^1$ für eine Bindung oder für eine Gruppe steht, die ausgewählt ist aus den Formeln ($L^1$-1) bis ($L^1$-108),

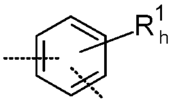

Formel ($L^1$-1)

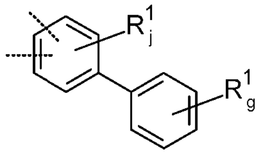

Formel ($L^1$-2)

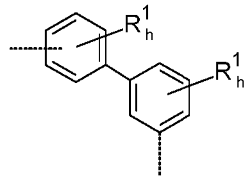

Formel ($L^1$-3)

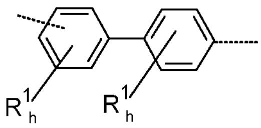

Formel ($L^1$-4)

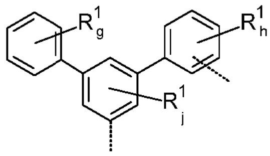

Formel ($L^1$-5)

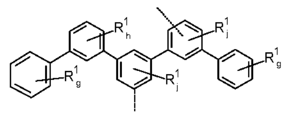

Formel ($L^1$-6)

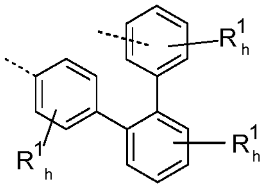

Formel ($L^1$-7)

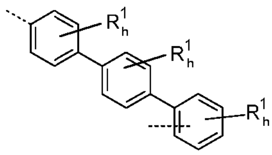

Formel ($L^1$-8)

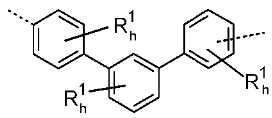

Formel ($L^1$-9)

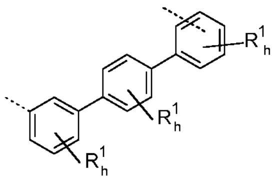

Formel ($L^1$-10)

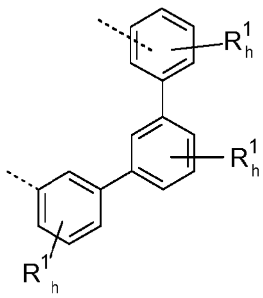

Formel ($L^1$-11)

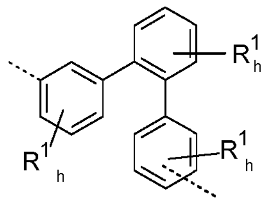

Formel ($L^1$-12)

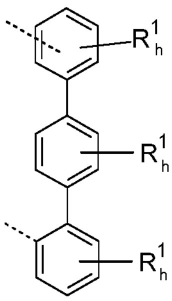

Formel ($L^1$-13)

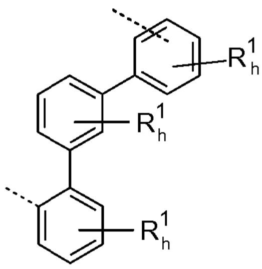

Formel ($L^1$-14)

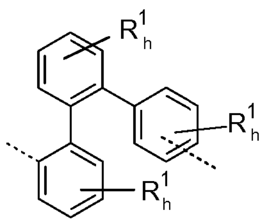

Formel ($L^1$-15)

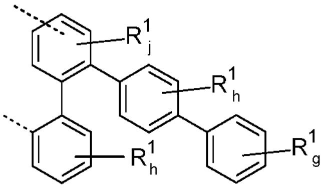

Formel ($L^1$-16)

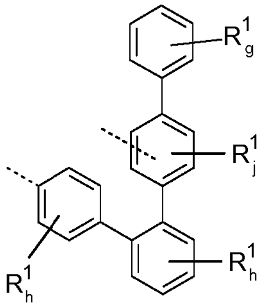

Formel ($L^1$-17)

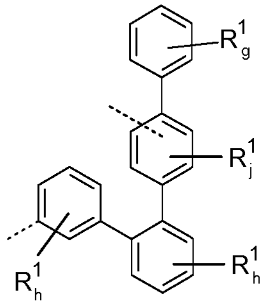

Formel ($L^1$-18)

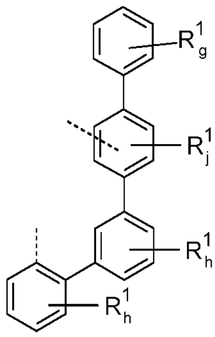

Formel ($L^1$-19)

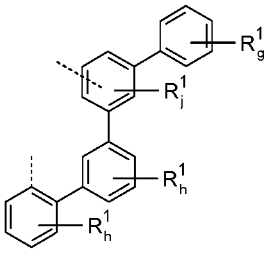

Formel ($L^1$-20)

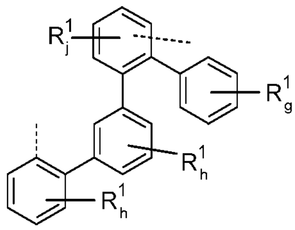

Formel ($L^1$-21)

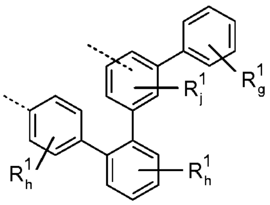

Formel ($L^1$-22)

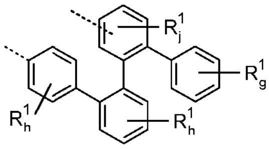

Formel ($L^1$-23)

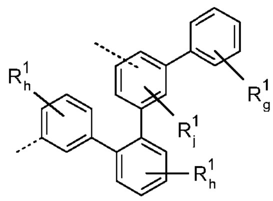

Formel ($L^1$-24)

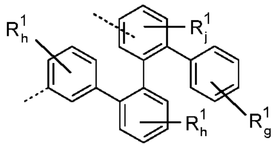

Formel ($L^1$-25)

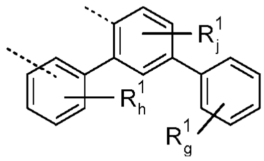

Formel ($L^1$-26)

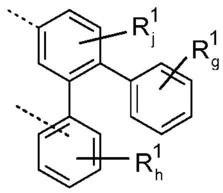

Formel ($L^1$-27)

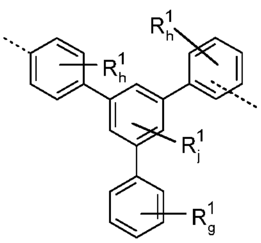

Formel ($L^1$-28)

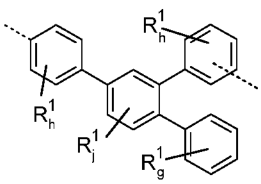

Formel ($L^1$-29)

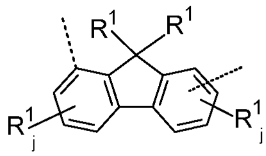

Formel ($L^1$-30)

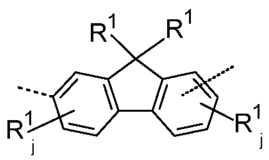

Formel ($L^1$-31)

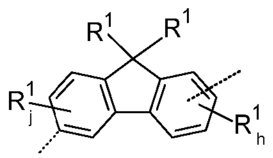

Formel ($L^1$-32)

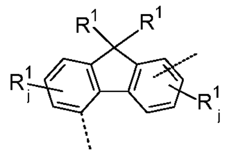

Formel ($L^1$-33)

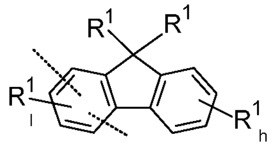

Formel ($L^1$-34)

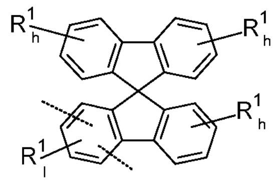

Formel ($L^1$-35)

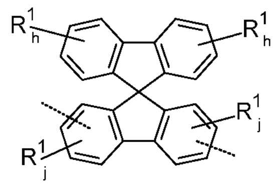

Formel ($L^1$-36)

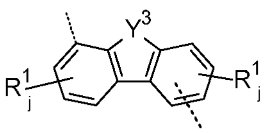

Formel ($L^1$-37)

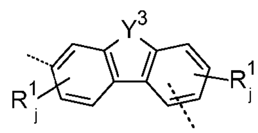

Formel ($L^1$-38)

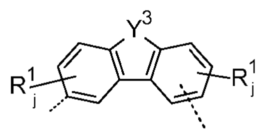

Formel ($L^1$-39)

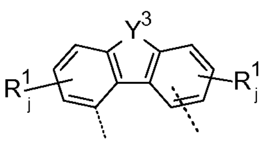

Formel ($L^1$-40)

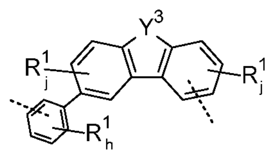

Formel ($L^1$-41)

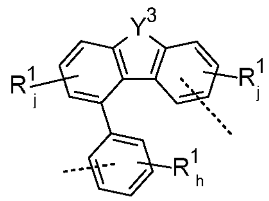

Formel ($L^1$-42)

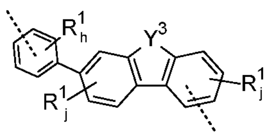

Formel ($L^1$-43)

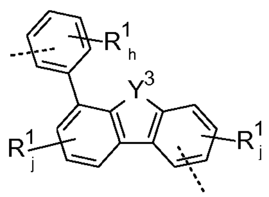

Formel ($L^1$-44)

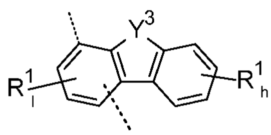

Formel ($L^1$-45)

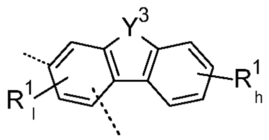

Formel ($L^1$-46)

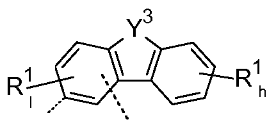

Formel ($L^1$-47)

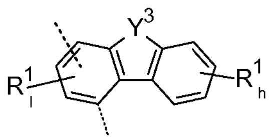

Formel ($L^1$-48)

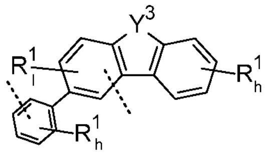
Formel ($L^1$-49)

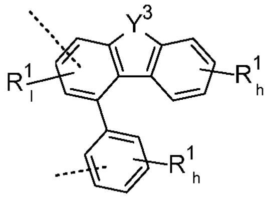
Formel ($L^1$-50)

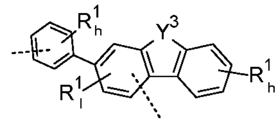
Formel ($L^1$-51)

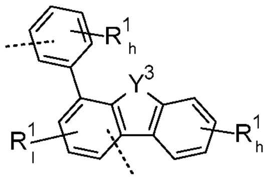
Formel ($L^1$-52)

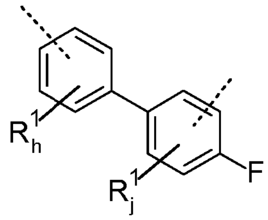
Formel ($L^1$-53)

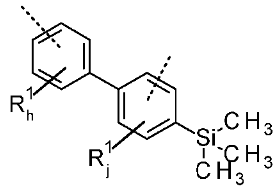
Formel ($L^1$-54)

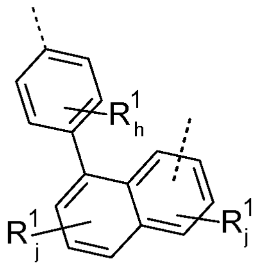
Formel ($L^1$-55)

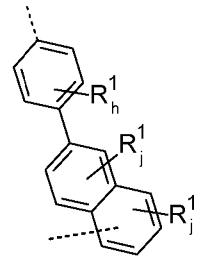
Formel ($L^1$-56)

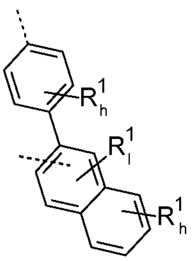
Formel ($L^1$-57)

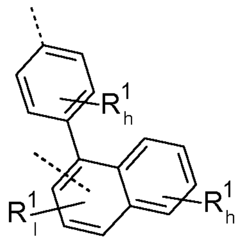
Formel ($L^1$-58)

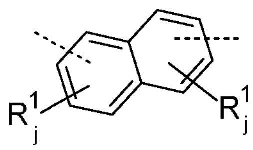
Formel ($L^1$-59)

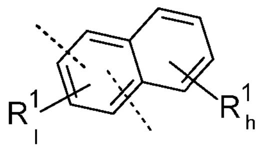
Formel ($L^1$-60)

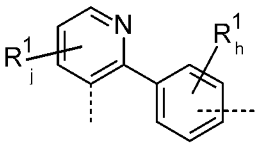
Formel ($L^1$-61)

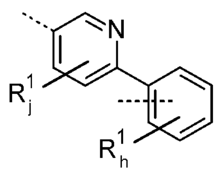
Formel ($L^1$-62)

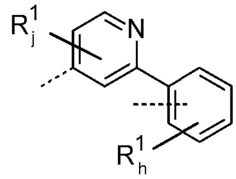
Formel ($L^1$-63)

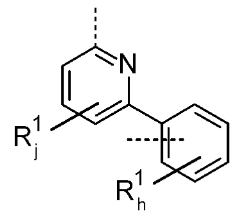

Formel ($L^1$-64)

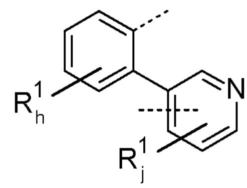

Formel ($L^1$-65)

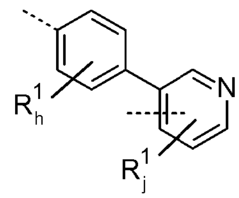

Formel ($L^1$-66)

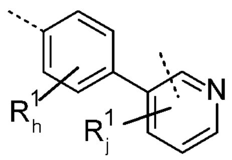

Formel ($L^1$-67)

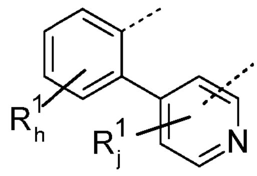

Formel ($L^1$-68)

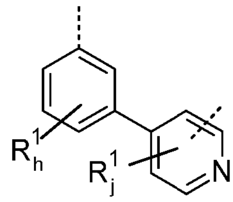

Formel ($L^1$-69)

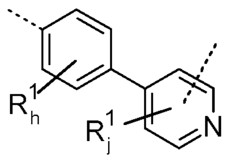

Formel ($L^1$-70)

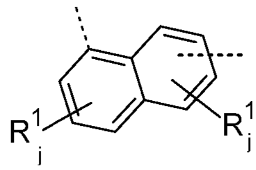

Formel ($L^1$-71)

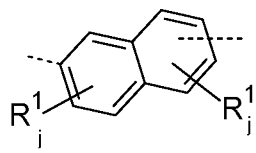

Formel ($L^1$-72)

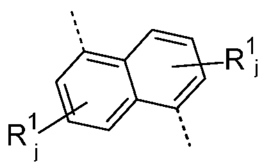

Formel ($L^1$-73)

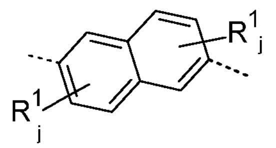

Formel ($L^1$-74)

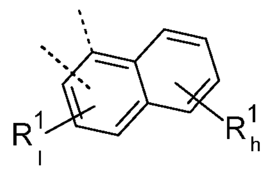

Formel ($L^1$-75)

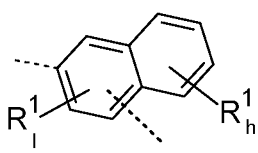

Formel ($L^1$-76)

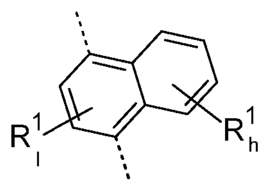

Formel ($L^1$-77)

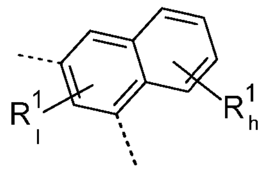

Formel ($L^1$-78)

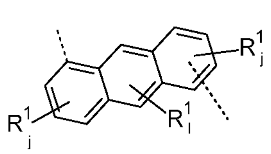

Formel ($L^1$-79)

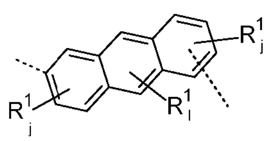

Formel ($L^1$-80)

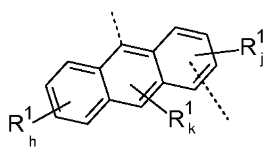

Formel ($L^1$-81)

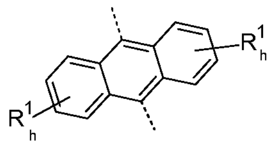

Formel ($L^1$-82)

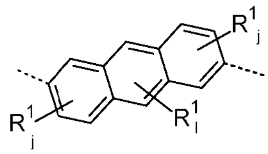

Formel ($L^1$-83)

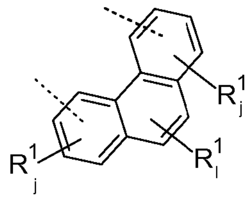

Formel ($L^1$-84)

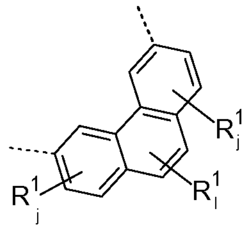

Formel ($L^1$-85)

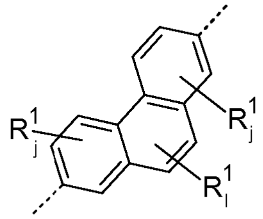

Formel ($L^1$-86)

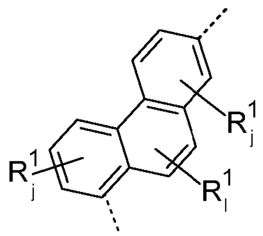

Formel ($L^1$-87)

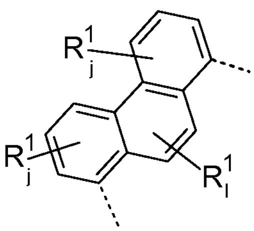

Formel ($L^1$-88)

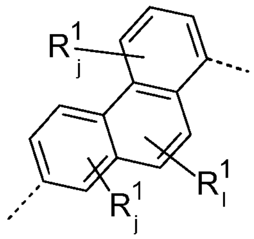

Formel ($L^1$-89)

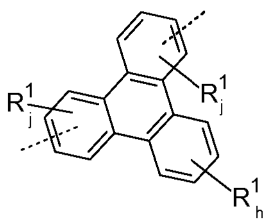

Formel ($L^1$-90)

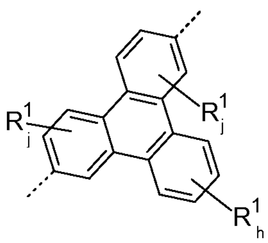

Formel ($L^1$-91)

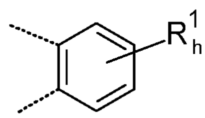

Formel ($L^1$-92)

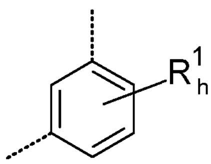

Formel ($L^1$-93)

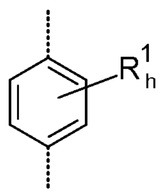

Formel ($L^1$-94)

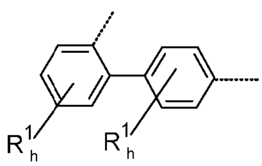

Formel ($L^1$-95)

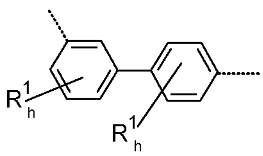

Formel ($L^1$-96)

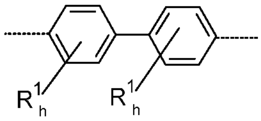
Formel ($L^1$-97)

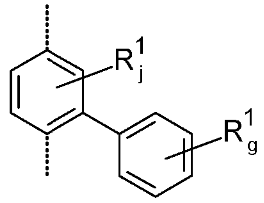
Formel ($L^1$-98)

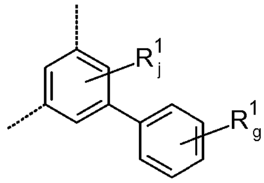
Formel ($L^1$-99)

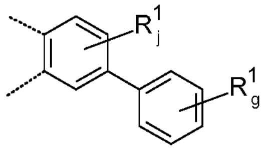
Formel ($L^1$-100)

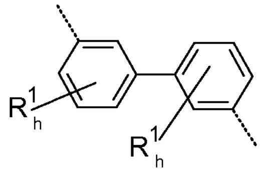
Formel ($L^1$-101)

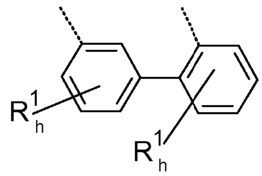
Formel ($L^1$-102)

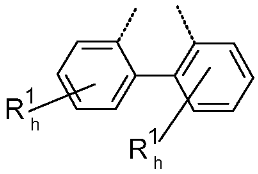
Formel ($L^1$-103)

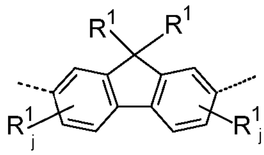
Formel ($L^1$-104)

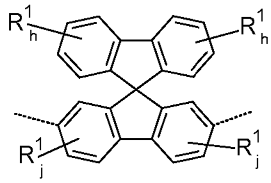
Formel ($L^1$-105)

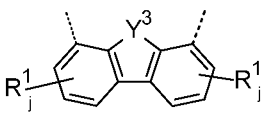
Formel ($L^1$-106)

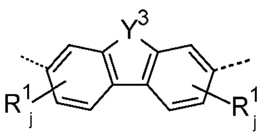
Formel ($L^1$-107)

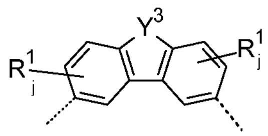
Formel ($L^1$-108)

wobei die gestrichelten Bindungen jeweils die Anbindungspositionen markieren, der Index k 0 oder 1 ist, der Index l 0, 1 oder 2 ist, der Index j bei jedem Auftreten unabhängig 0, 1, 2 oder 3 ist, der Index h bei jedem Auftreten unabhängig 0, 1, 2, 3 oder 4 ist, der Index g 0, 1, 2, 3, 4 oder 5 ist, das Symbol $Y^3$ O, S oder $NR^1$, vorzugsweise O oder S ist, und das Symbol $R^1$ die zuvor, insbesondere für Formel (I) genannte Bedeutung aufweist.

Vorzugsweise kann vorgesehen sein, dass die Summe der Indices k, l, g, h und j in den Strukturen der Formel ($L^1$-1) bis ($L^1$-108) jeweils höchstens 3, vorzugsweise höchstens 2 und besonders bevorzugt höchstens 1 beträgt.

Bevorzugte erfindungsgemäße Verbindungen mit einer Gruppe der Formeln (H-1) bis (H-26) umfassen eine Gruppe $Ar^2$, die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugte erfindungsgemäße Verbindungen mit einer Gruppe der Formel (QL) umfassen eine Gruppe $L^1$, die eine Bindung darstellt oder die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugt bilden die Reste $R^1$ in den Formeln ($L^1$-1) bis ($L^1$-108) mit den Ringatomen der Arylgruppe oder Heteroarylgruppe, an die die Reste $R^1$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^2$ und $R^3$ ein, die an die Reste $R^1$ beziehungsweise $R^2$ gebunden sein können.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf.

Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

In einer weiteren bevorzugten Ausführungsform der Erfindung ist $R^2$, beispielsweise bei einer Struktur gemäß Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Bevorzugt bilden die Reste $R^2$ mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren bevorzugten Ausführungsform der Erfindung ist $R^3$, beispielsweise bei einer Struktur gemäß Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch eine oder mehrere Alkylgruppen mit jeweils 1 bis 4 Kohlenstoffatomen substituiert sein kann, bevorzugt aber unsubstituiert ist.

Beispiele für geeignete erfindungsgemäße Verbindungen sind die nachstehend gezeigten Strukturen gemäß den folgenden Formeln 1 bis 102:

| | | |
|---|---|---|
| 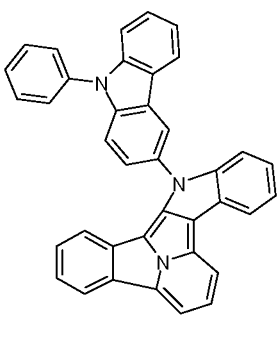 | 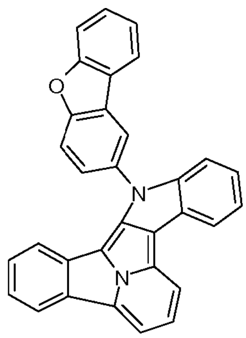 | 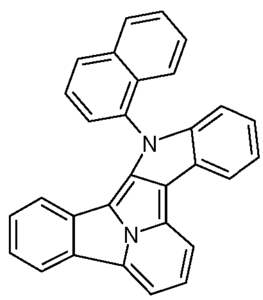 |
| Formel 1 | Formel 2 | Formel 3 |
| 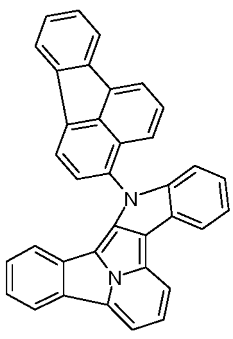 | 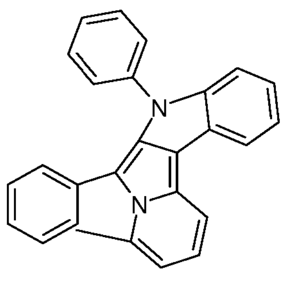 | 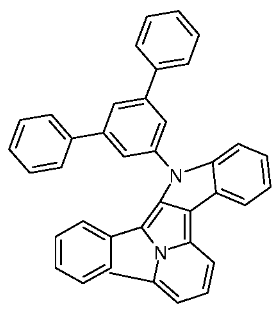 |
| Formel 4 | Formel 5 | Formel 6 |

(fortgesetzt)

| | | |
|---|---|---|
| 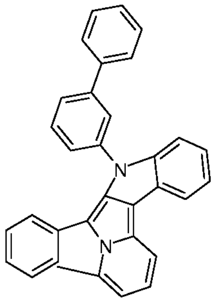 | 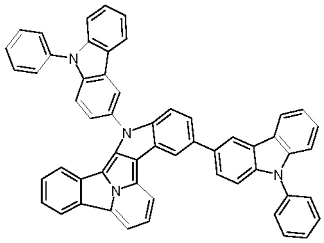 | 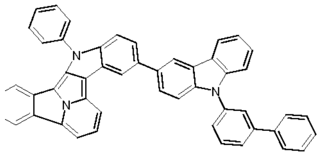 |
| Formel 7 | Formel 8 | Formel 9 |
| 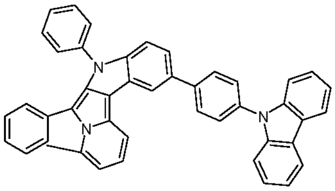 | 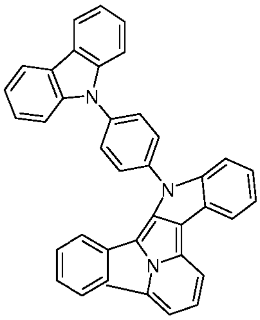 | 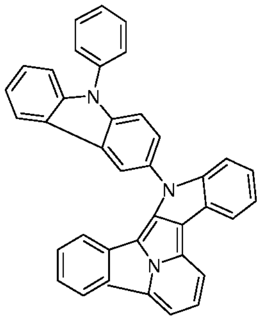 |
| Formel 10 | Formel 11 | Formel 12 |
| 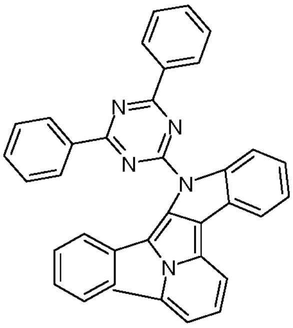 | 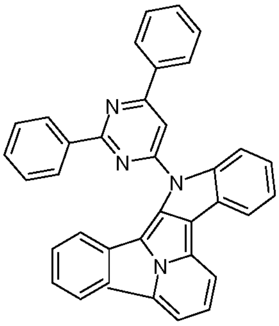 | 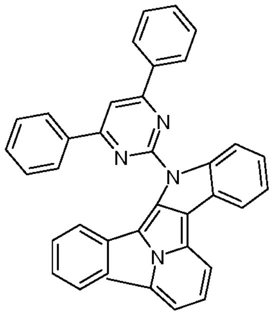 |
| Formel 13 | Formel 14 | Formel 15 |
| 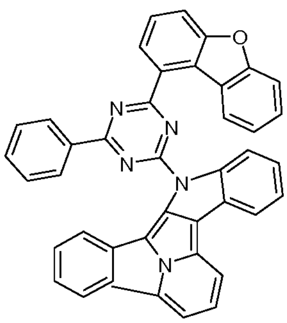 | 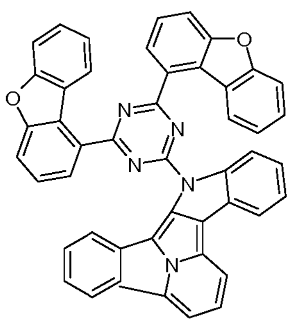 | 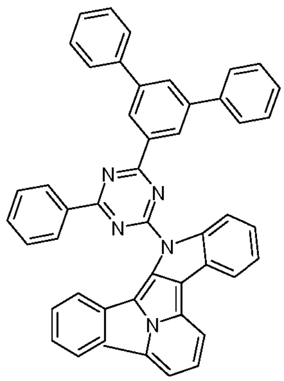 |
| Formel 16 | Formel 17 | Formel 18 |

(fortgesetzt)

| | | |
|---|---|---|
| 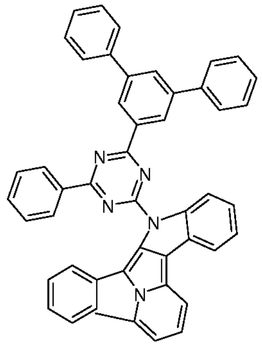 | 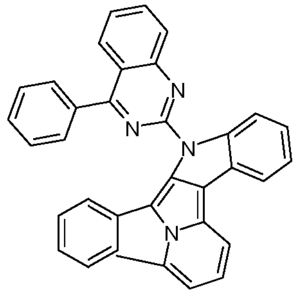 | 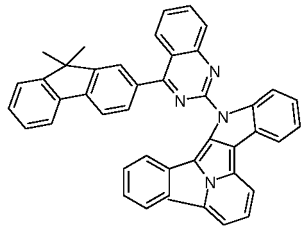 |
| Formel 19 | Formel 20 | Formel 21 |
| 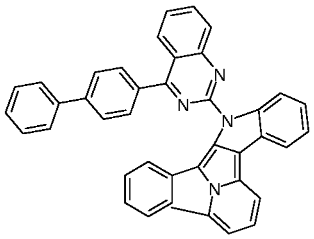 | 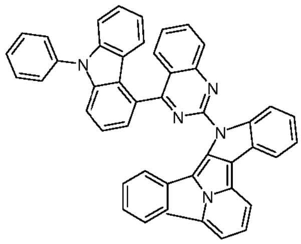 | 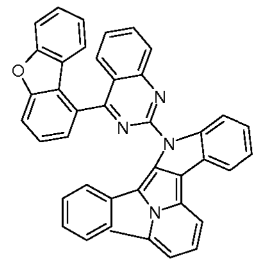 |
| Formel 22 | Formel 23 | Formel 24 |
| 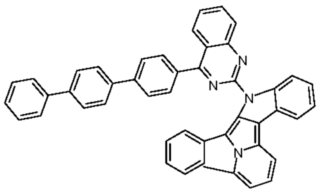 | 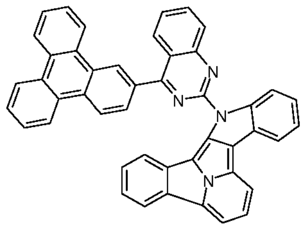 | 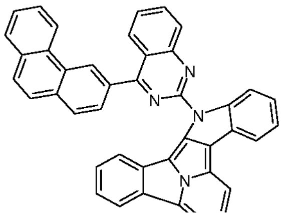 |
| Formel 25 | Formel 26 | Formel 27 |
| 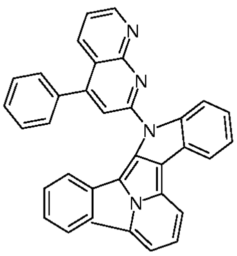 | 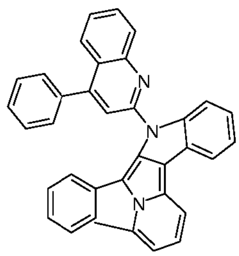 | 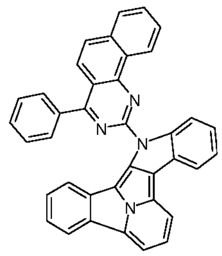 |
| Formel 28 | Formel 29 | Formel 30 |
| 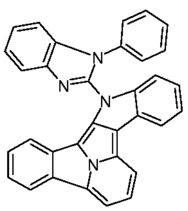 | 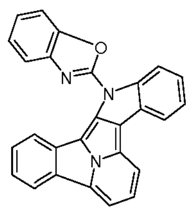 | 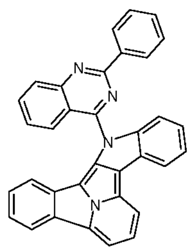 |
| Formel 31 | Formel 32 | Formel 33 |

(fortgesetzt)

| | | |
|---|---|---|
| 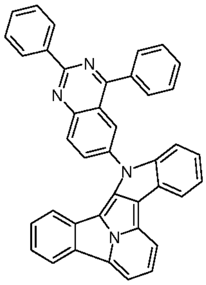 | 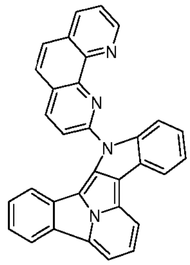 | 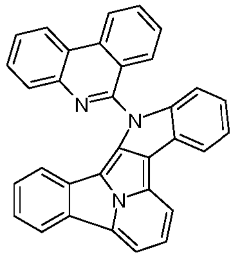 |
| Formel 34 | Formel 35 | Formel 36 |
| 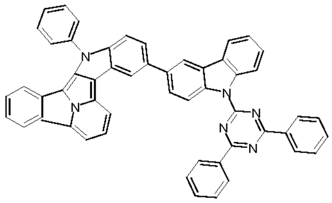 | 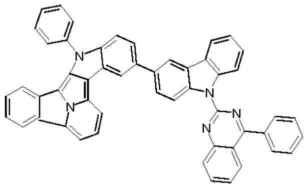 | 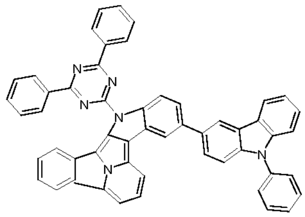 |
| Formel 37 | Formel 38 | Formel 39 |
| 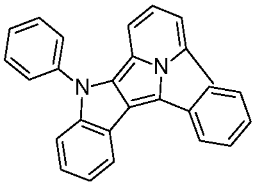 | 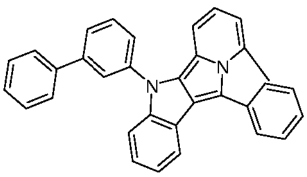 | 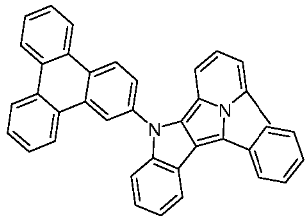 |
| Formel 40 | Formel 41 | Formel 42 |
| 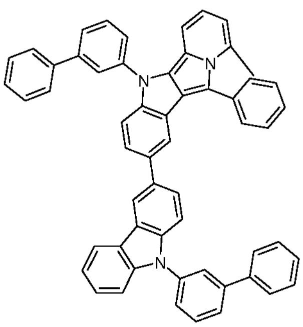 | 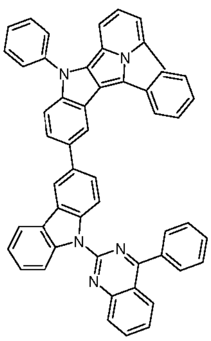 | 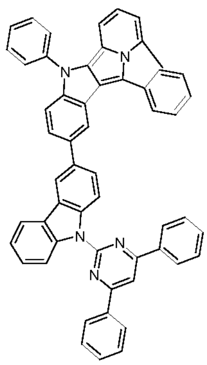 |
| Formel 43 | Formel 44 | Formel 45 |
| 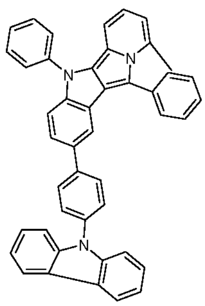 | 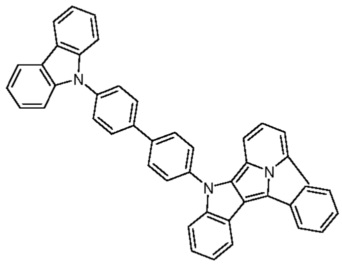 | 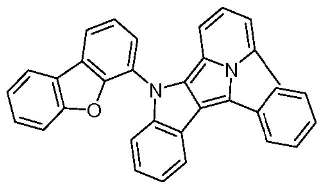 |
| Formel 46 | Formel 47 | Formel 48 |

(fortgesetzt)

| | | |
|---|---|---|
| 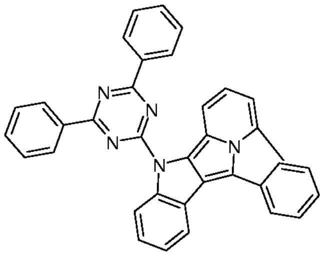 | 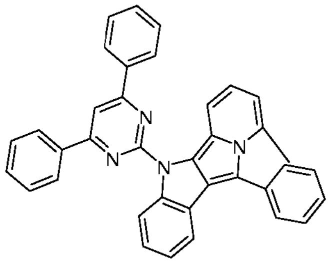 | 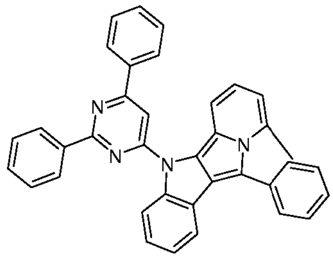 |
| Formel 49 | Formel 50 | Formel 51 |
| 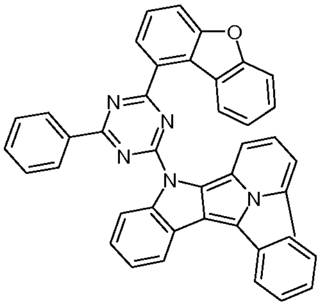 | 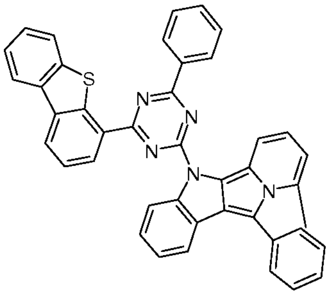 | 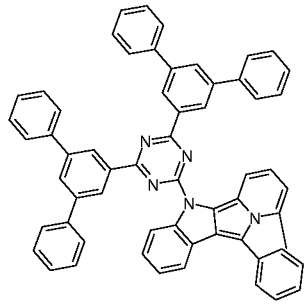 |
| Formel 52 | Formel 53 | Formel 54 |
| 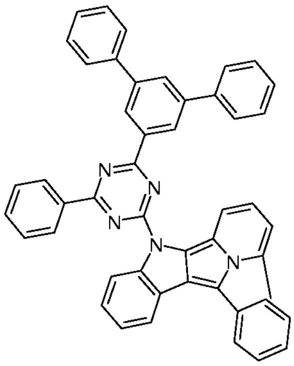 | 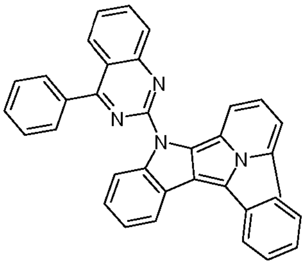 | 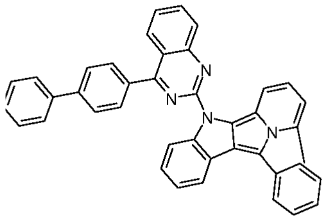 |
| Formel 55 | Formel 56 | Formel 57 |
| 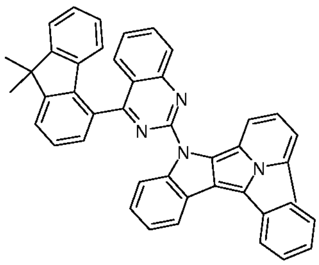 | 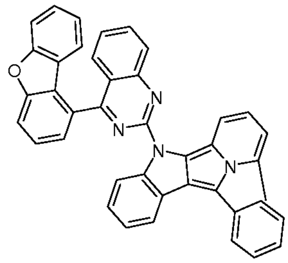 | 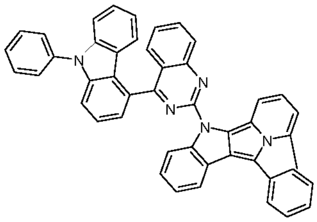 |
| Formel 58 | Formel 59 | Formel 60 |

(fortgesetzt)

| | | |
|---|---|---|
| 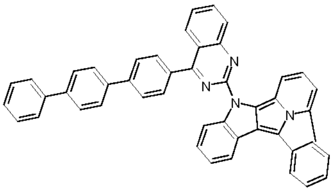 | 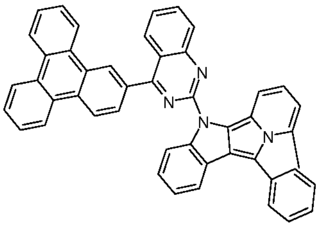 | 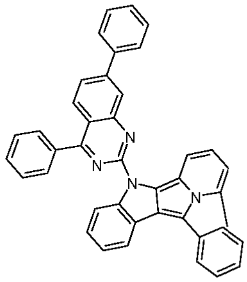 |
| Formel 61 | Formel 62 | Formel 63 |
| 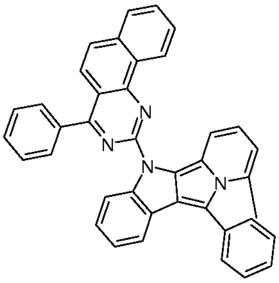 | 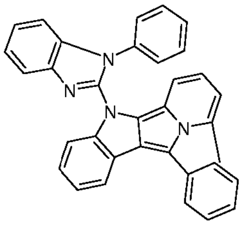 | 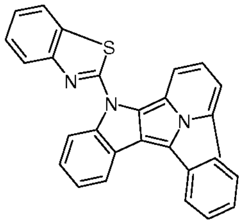 |
| Formel 64 | Formel 65 | Formel 66 |
| 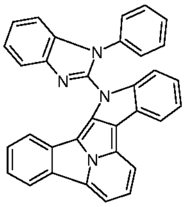 | 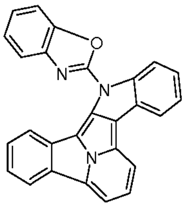 | 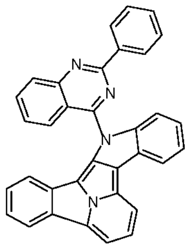 |
| Formel 67 | Formel 68 | Formel 69 |
| 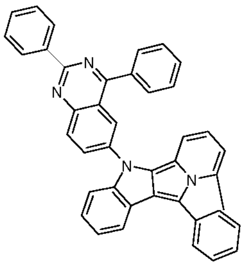 | 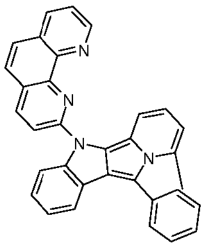 | 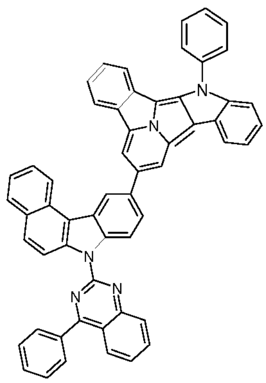 |
| Formel 70 | Formel 71 | Formel 72 |

(fortgesetzt)

| | | |
|---|---|---|
| 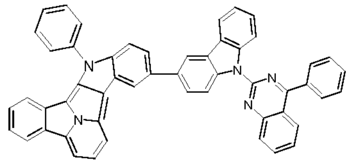 | 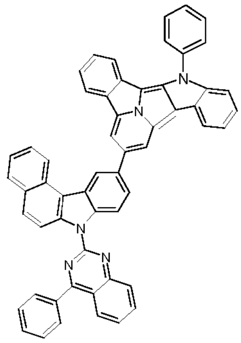 | 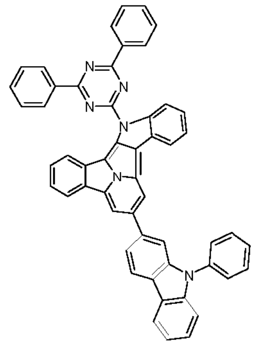 |
| Formel 73 | Formel 74 | Formel 75 |
| 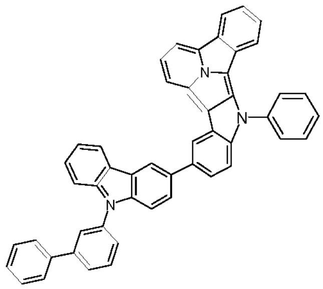 | 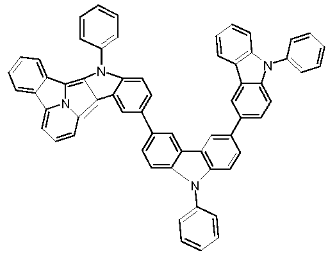 | 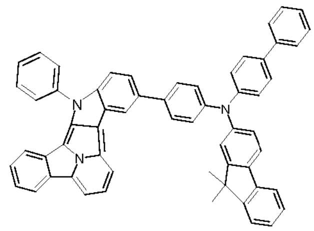 |
| Formel 76 | Formel 77 | Formel 78 |
| 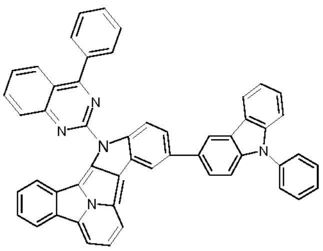 | 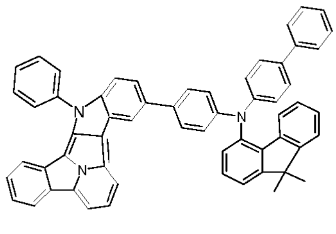 | 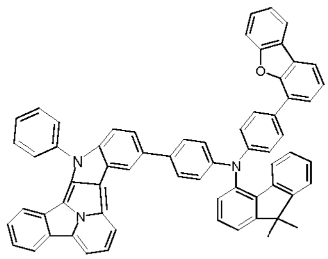 |
| Formel 79 | Formel 80 | Formel 81 |
| 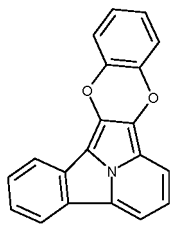 | 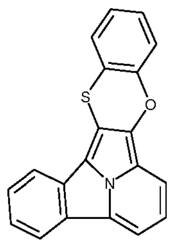 | 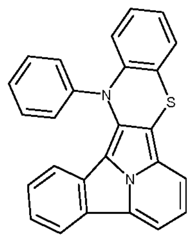 |
| Formel 82 | Formel 83 | Formel 84 |

(fortgesetzt)

| | | |
|---|---|---|
| 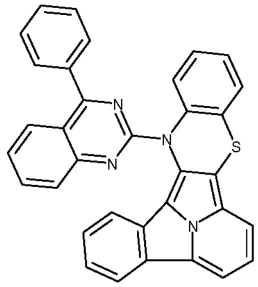 | 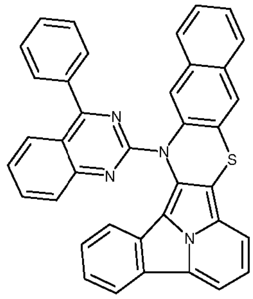 | 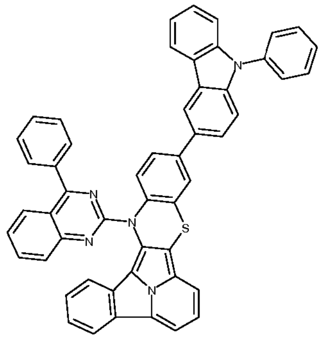 |
| Formel 85 | Formel 86 | Formel 87 |
| 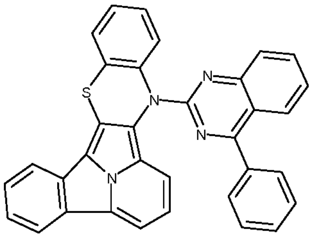 | 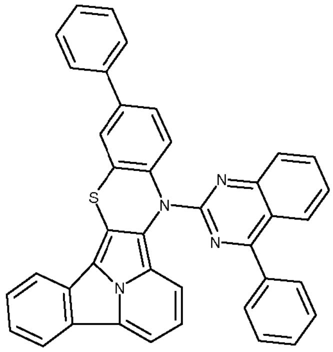 | 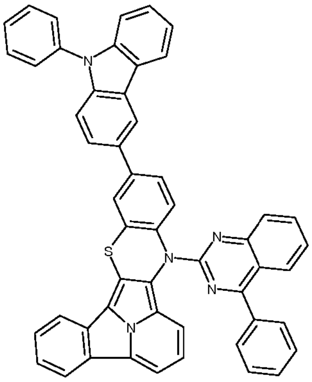 |
| Formel 88 | Formel 89 | Formel 90 |
| 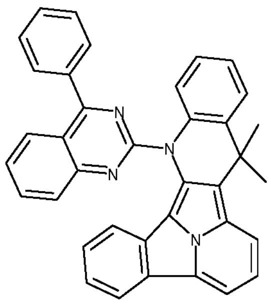 | 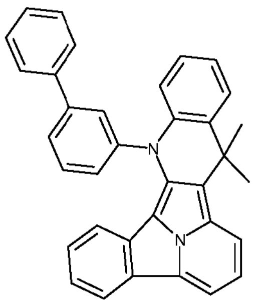 | 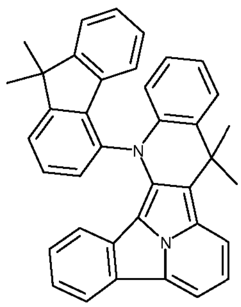 |
| Formel 91 | Formel 92 | Formel 93 |
| 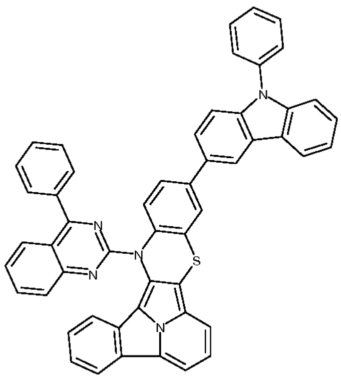 | 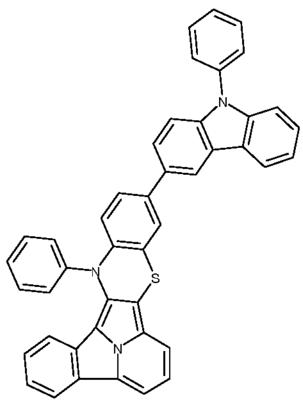 | 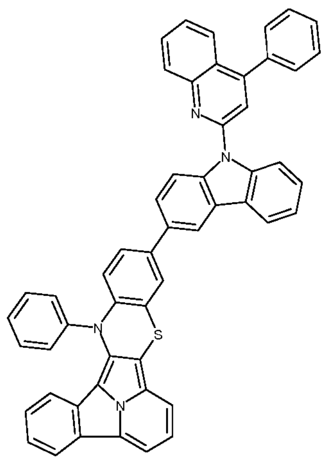 |
| Formel 94 | Formel 95 | Formel 96 |

(fortgesetzt)

| | | |
|---|---|---|
| 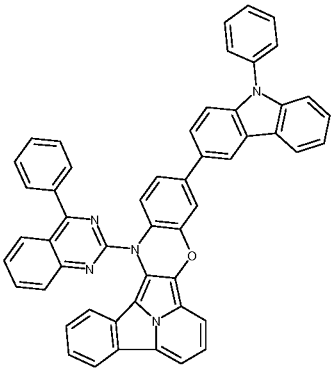 | 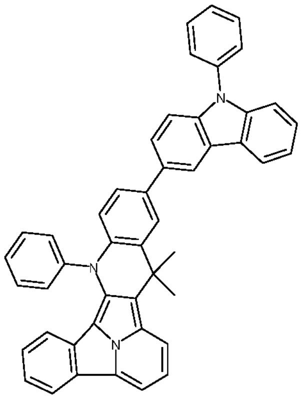 | 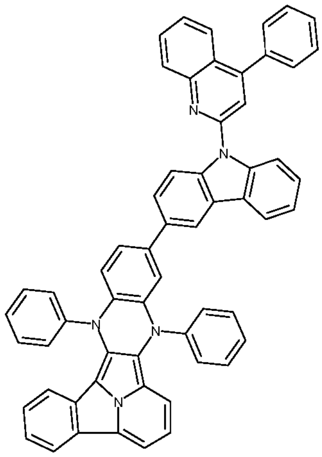 |
| Formel 97 | Formel 98 | Formel 99 |
| 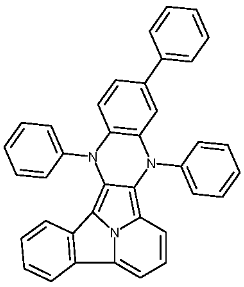 | 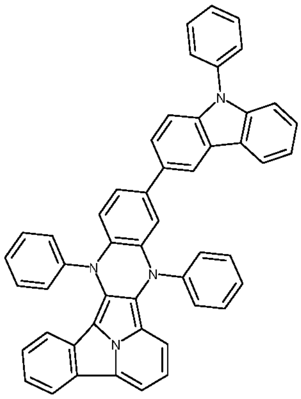 | 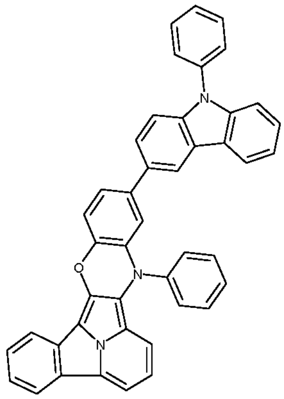 |
| Formel 100 | Formel 101 | Formel 102 |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (I), bei dem in einer Kupplungsreaktion eine Verbindung, umfassend mindestens eine stickstoffhaltige heterocyclische Gruppe, mit einer Verbindung, umfassend mindestens eine aromatische oder heteroaromatische Gruppe, verbunden wird.

Geeignete Indolizine können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen. Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

In allen folgenden Syntheseschemata sind die Verbindungen zur Vereinfachung der Strukturen mit einer geringen Anzahl an Substituenten gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

Schema 1

a)

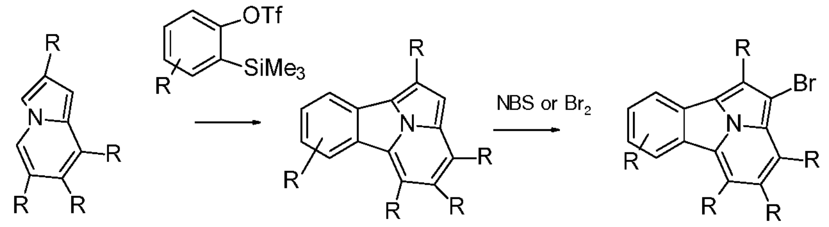


Eur.J. Org. Chem. 2007, 3718-3726

b)

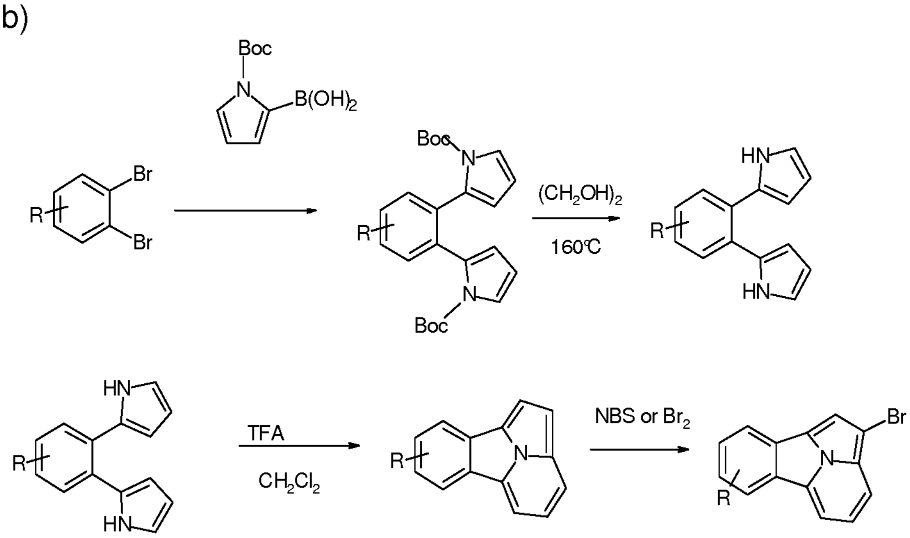


WO 2013/183327

Schema 2

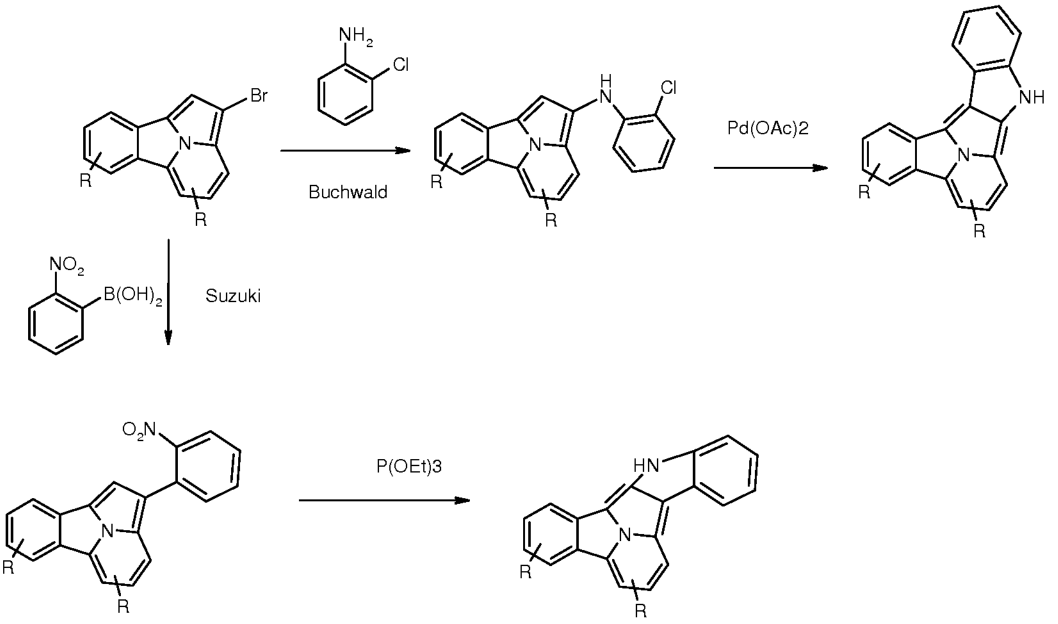


Schema 3

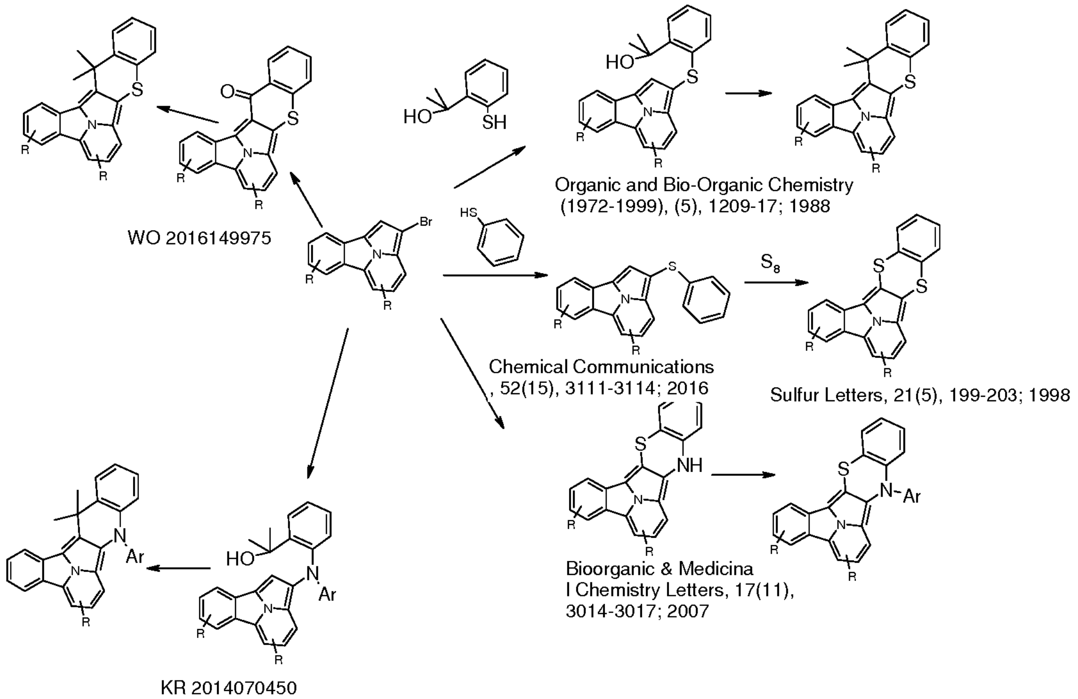


Die Bedeutung der in den Schemata 1, 2 und 3 verwendeten Symbole entspricht im Wesentlichen denen, die für Formel (I) definiert wurden, wobei aus Gründen der Übersichtlichkeit auf eine Nummerierung verzichtet wurde. Ferner finden sich Literaturangaben über weitere Einzelheiten der schematisch gezeigten Umsetzungen.

In Bezug auf die Veröffentlichungen WO 2016/149975; Organic and BioOrganic Chemistry, (1972-1999), (5), 1209-17, 1988; Sulfur Letters, 21 (5), 199-203; 1998, Bioorganic & Medicinal Chemistry Letters, 17(11), 3014-3017, 2007; und KR 20140070450 ist festzuhalten, dass diese Dokumente keine Verbindungen mit einer Indolizinstruktur beschreiben, sondern die zuvor näher beschriebenen Reaktionen an anderen aromatischen oder heteroaromatischen Verbindungen darlegen.

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, so dass die Verbindungen beispielsweise in Toluol oder Xylol bei Raumtemperatur in ausreichender Konzentration löslich sind, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (I) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) oder erfindungsgemäße Verbindungen, wobei ein oder mehrere Bindungen der erfindungsgemäßen Verbindungen oder der Strukturen der Formel (I) zum Polymer, Oligomer oder Dendrimer vorhanden sind. Je nach Verknüpfung der Strukturen der Formel (I) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (I) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mi ndestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, beispielsweise ein fluoreszierender Dotand, ein phosphoreszierender Dotand oder eine Verbindung, die TADF (thermally activated delayed fluorescence) zeigt, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, Matrixmaterialien, Elektronentransportmaterialien, Elektroneninjektionsmaterialien, Lochleitermaterialien, Lochinjektionsmaterialien, Elektronenblockiermaterialien, Lochblockiermaterialien, Exzitonenblockiermaterialien, Wide-Band-Gap-Materialien und n-Dotanden.

Die vorliegenden Erfindung betrifft daher auch eine Zusammensetzung enthaltend wenigstens eine Verbindung umfassend Strukuren gemäß Formel (I) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden Erfindung stellt eine Zusammensetzung dar, enthaltend wenigstens eine Verbindung, umfassend mindestens eine Struktur gemäß Formel (I) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$\text{HOMO(eV)} = ((\text{HEh}*27.212)-0.9899)/1.1206$$

$$\text{LUMO(eV)} = ((\text{LEh}*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formel (I) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintettzustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011 /066898, WO 2011 /157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186 und der noch nicht offen gelegten Anmeldung EP 16186313.9 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

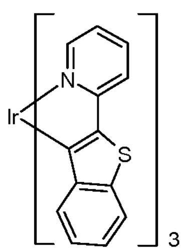

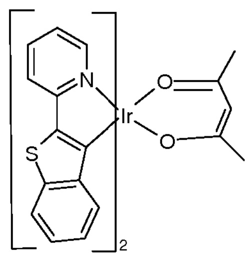

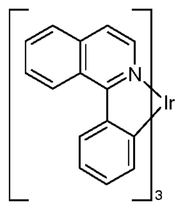

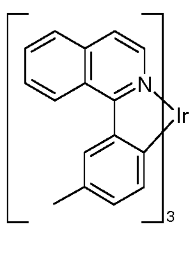

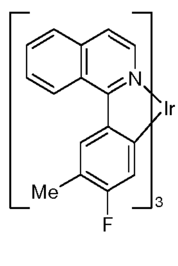

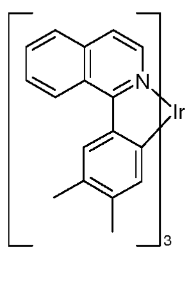

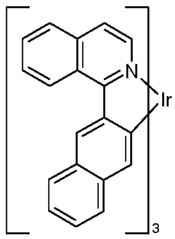
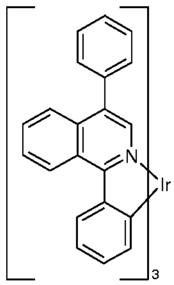
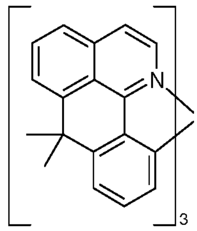

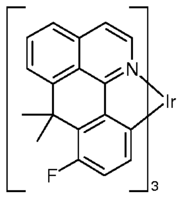
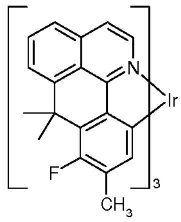
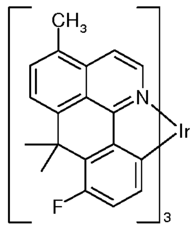

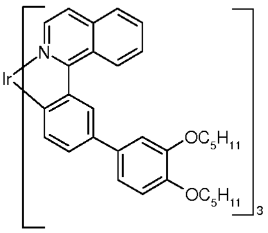
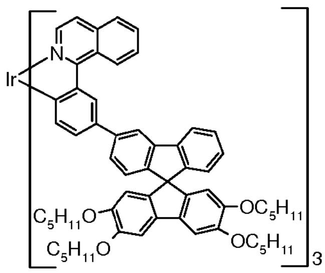
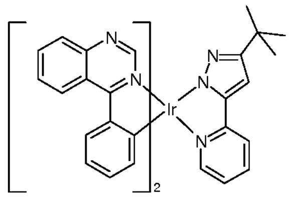

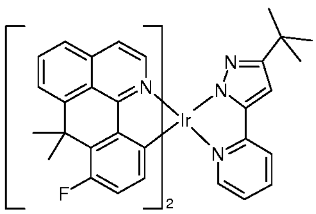
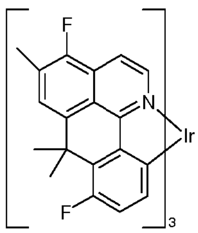
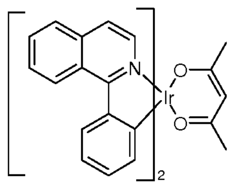

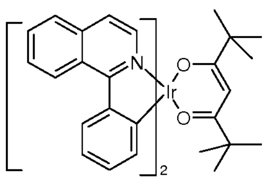
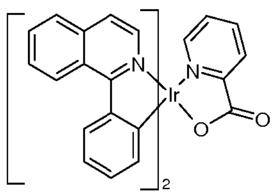
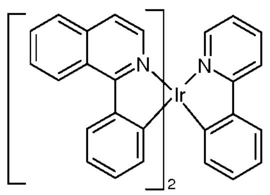

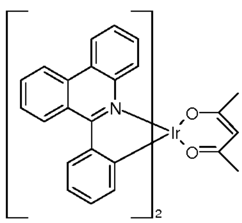
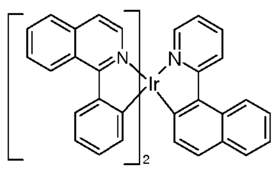
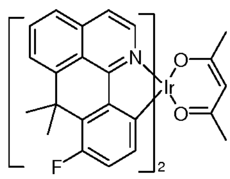

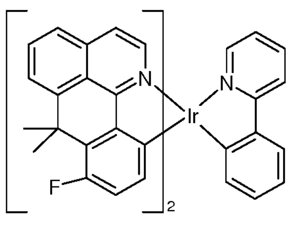
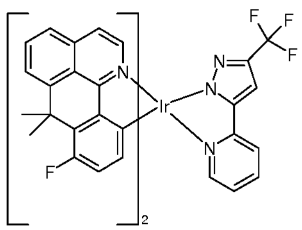
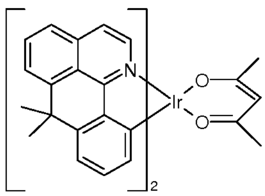

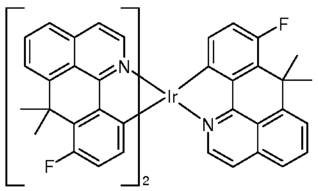
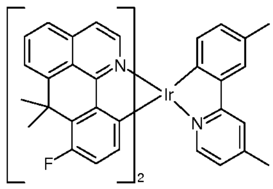
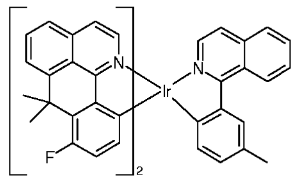

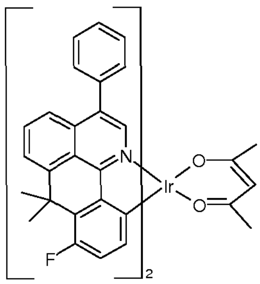
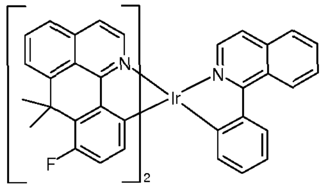
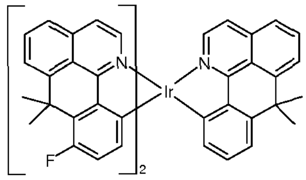

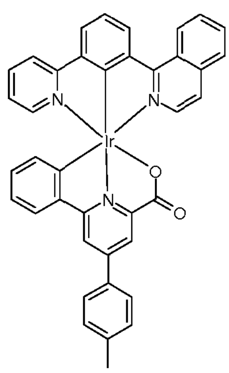
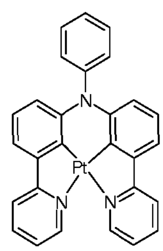
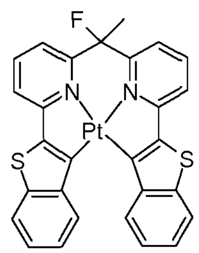

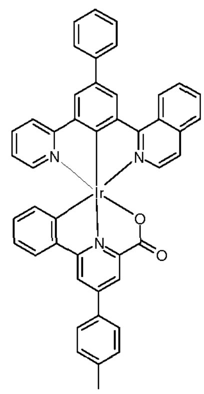
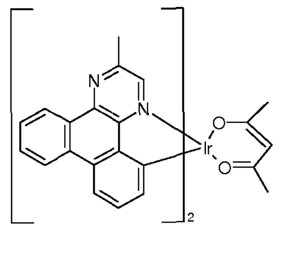
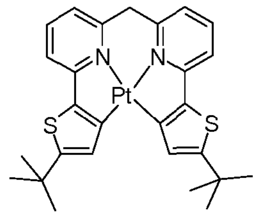

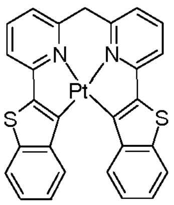
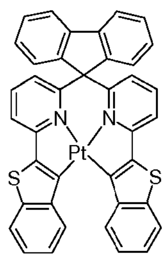
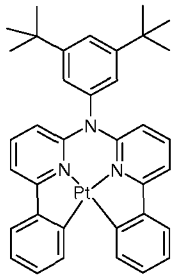
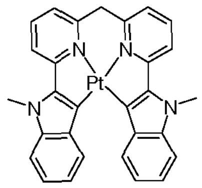
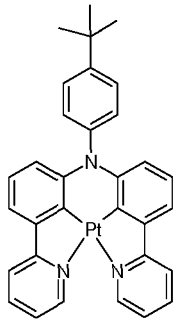
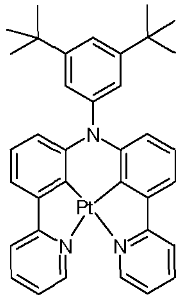
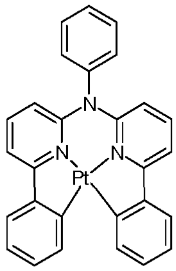
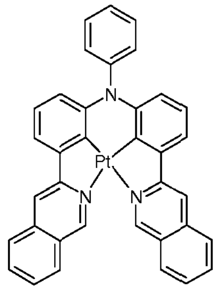
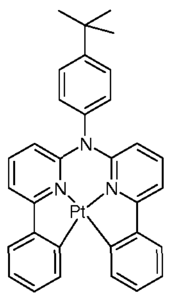
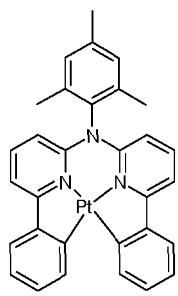
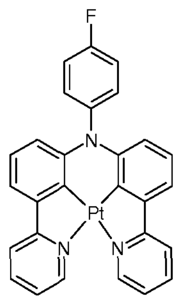
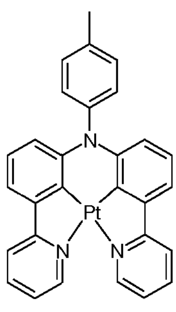
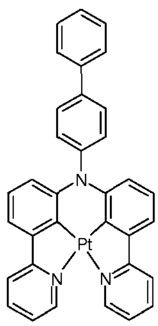
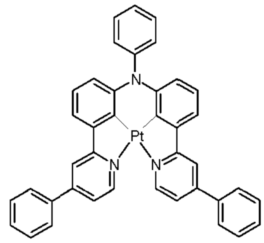

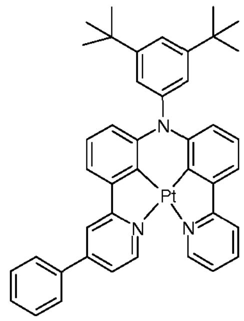

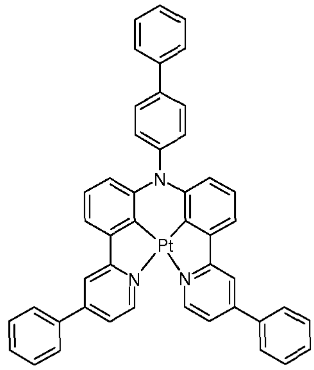

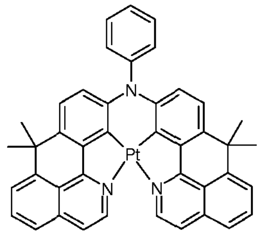

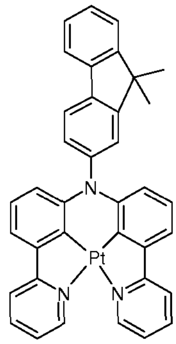

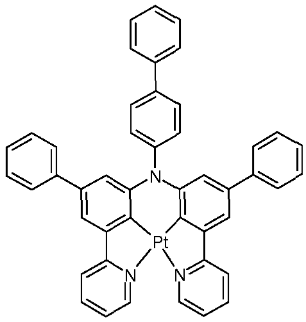

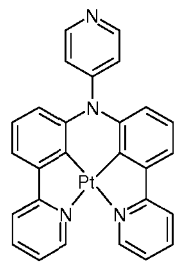

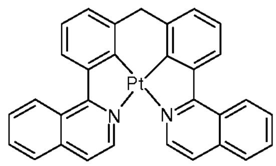

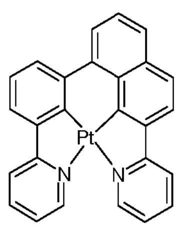

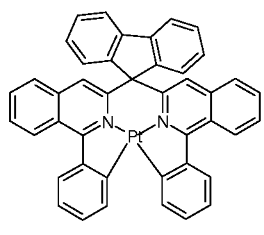

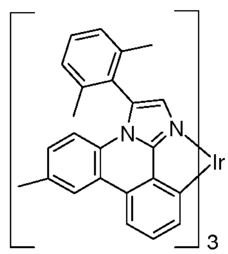

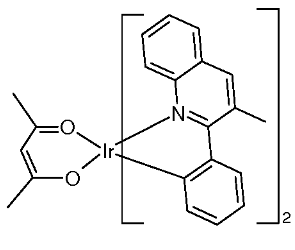

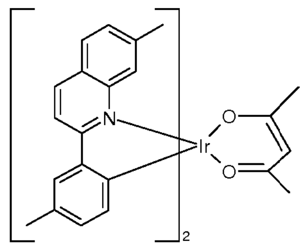

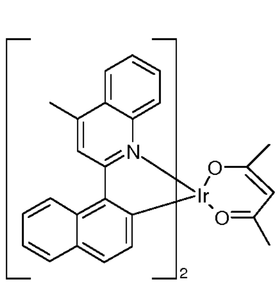

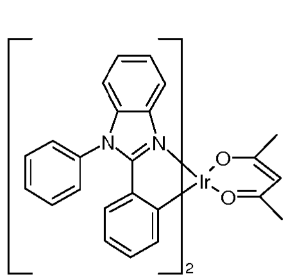

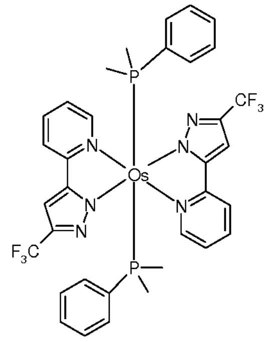

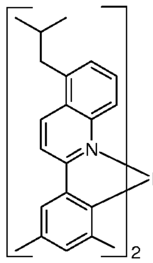

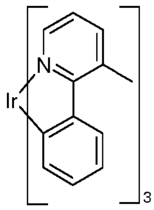

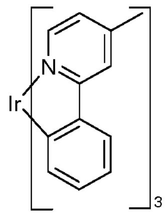

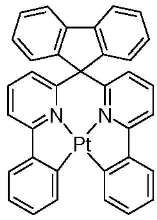

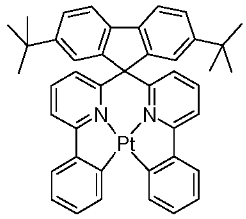

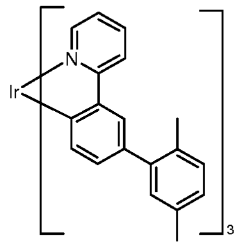

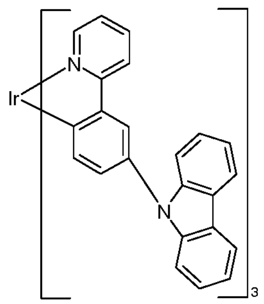

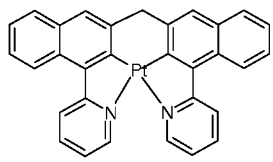

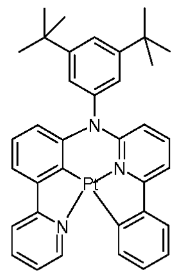

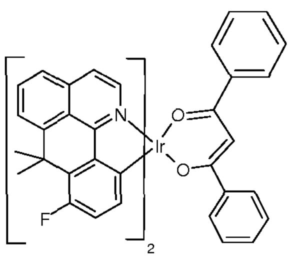

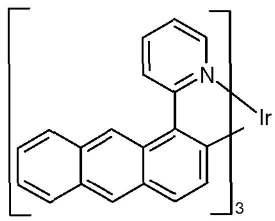

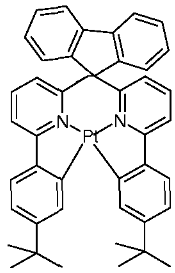

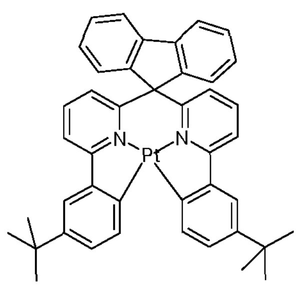

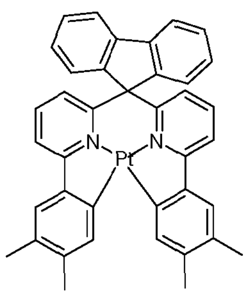

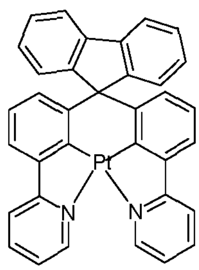

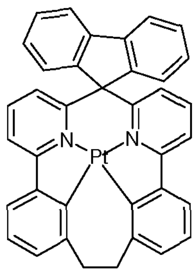
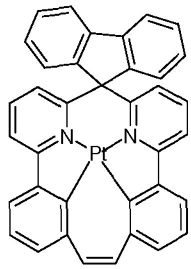
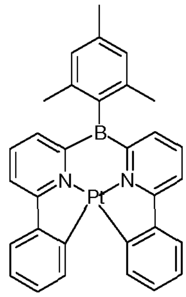

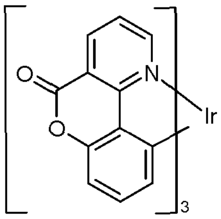
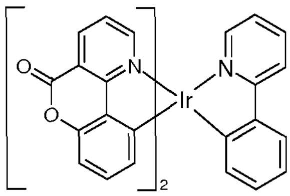
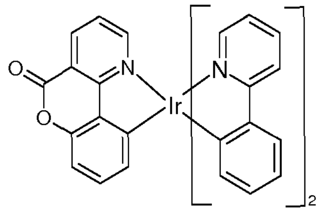

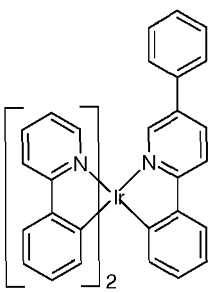
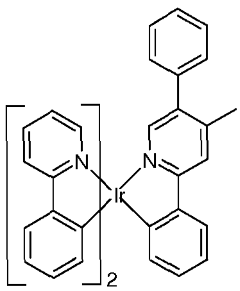
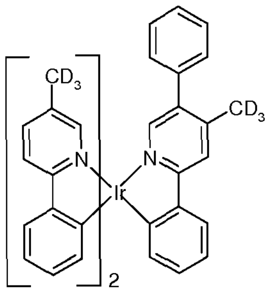

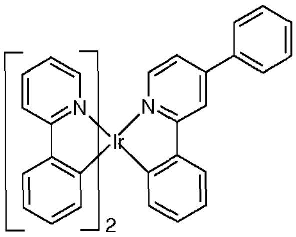
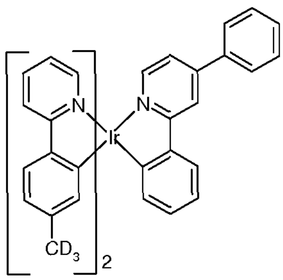
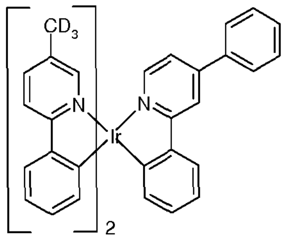

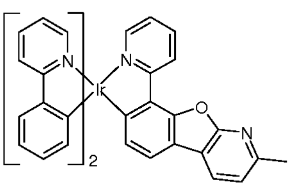
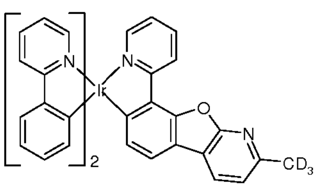
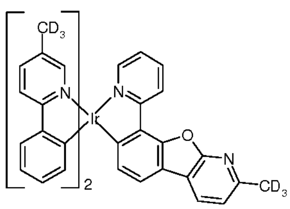

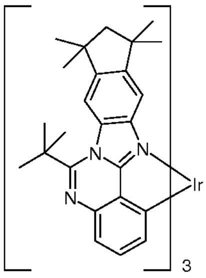
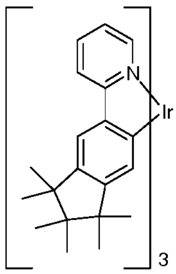
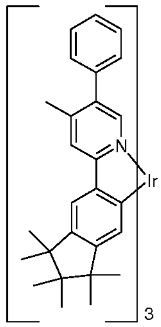

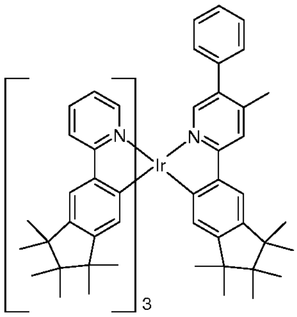
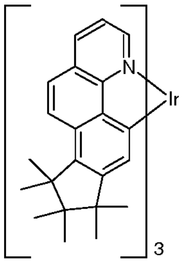
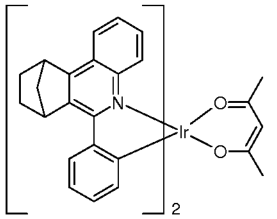

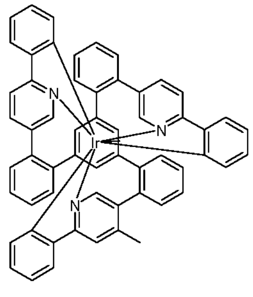
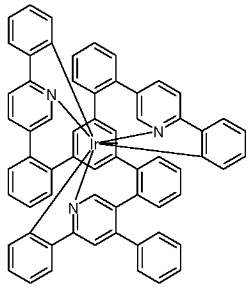
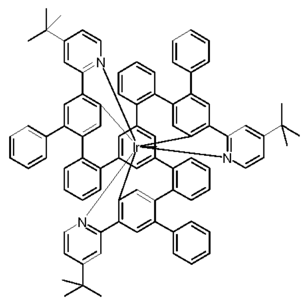

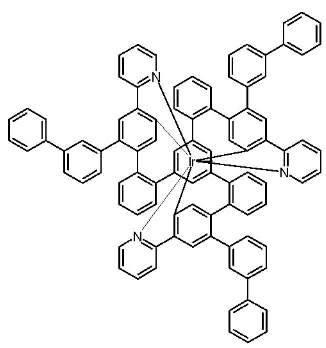
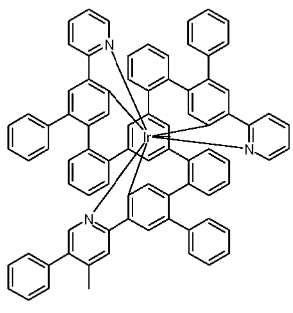
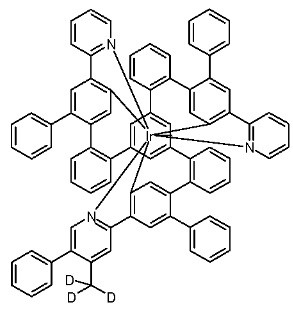

Die oben beschriebenen Verbindung, umfassend Strukturen der Formel (I) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formel (I), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formel (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $M0O_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonenblockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Weiterhin bevorzugt sind auch Tandem-OLEDs. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (I) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

In einer weiterhin bevorzugten Ausführungsform der Erfindung enthält die organische Elektrolumineszenzvorrichtung die efindungsgemäße Verbindung umfassend Strukturen gemäß Formel (I) bzw. die oben aufgeführten bevorzugten Ausführungsformen als Matrixmaterial für einen phosphoreszierenden Emitter.

Geeignete Matrixmaterialien, welche in Kombination mit den Verbindungen gemäß Formel (I) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011 /042107, WO 2011 /088877 oder WO 2012/143080, Triphenylenderivate, z. B. gemäß WO 2012/048781, Lactame, z. B. gemäß WO 2011/116865, WO 2011 /137951 oder WO 2013/064206, 4-SpirocarbazolDerivate, z. B. gemäß WO 2014/094963 oder WO 2015/192939, oder Dibenzofuran-Derivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame, Carbazolderivate und Biscarbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

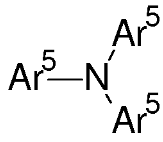

Formel (TA-1)

wobei $Ar^5$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, darstellt, wobei optional zwei oder mehr benachbarte Substituenten $R^2$ ein mono- oder polycyclisches, aliphatisches Ringsystem bilden können, das mit einem oder mehreren Resten $R^3$ substituiert sein kann, wobei das Symbol $R^2$ die zuvor, insbesondere für Formel (I) genannte Bedeutung aufweist. Vorzugsweise stellt $Ar^5$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^5$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^5$ gleich oder verschieden bei jedem Auftreten ausgewählt aus den oben genannten Gruppen $R^1$-1 bis $R^1$-92, besonders bevorzugt $R^1$-1 bis $R^1$-54.

In einer bevorzugten Ausführungsform der Verbindungen der Formel (TA-1) ist mindestens eine Gruppe $Ar^5$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^5$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^5$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^5$ ausgewählt aus einer Fluoren- oder Spirobifluorengruppe, insbesondere einer 4-Fluoren- bzw. 4-Spirobifluorengruppe, und eine Gruppe $Ar^5$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^5$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

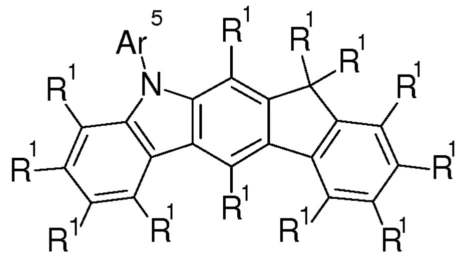

Formel (TA-2)

wobei $Ar^5$ und $R^1$ die oben insbesondere für Formeln (I) und/oder (TA-1) aufgeführten Bedeutungen aufweisen. Dabei sind bevorzugte Ausführungsformen der Gruppe $Ar^5$ die oben aufgeführten Strukturen $R^1$-1 bis $R^1$-92, besonders bevorzugt $R^1$-1 bis $R^1$-54.

Eine bevorzugte Ausführungsform der Verbindungen der Formel (TA-2) sind die Verbindungen der folgenden Formel (TA-2a),

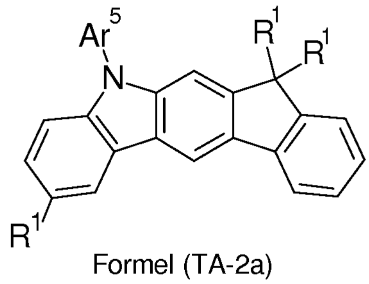

Formel (TA-2a)

wobei $Ar^5$ und $R^1$ die oben, insbesondere für Formeln (I) und/oder (TA-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

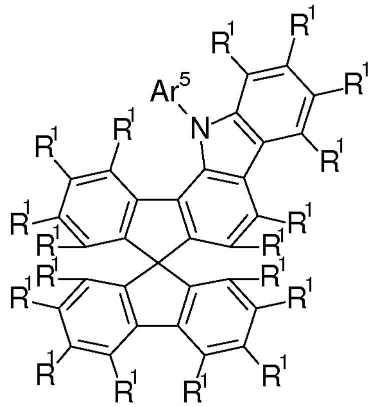

Formel (TA-3)

wobei $Ar^5$ und $R^1$ die oben, insbesondere für Formeln (I) und/oder (TA-1), aufgeführten Bedeutungen aufweisen. Dabei sind bevorzugte Ausführungsformen der Gruppe $Ar^5$ die oben aufgeführten Strukturen $R^1$-1 bis $R^1$-92, besonders bevorzugt $R^{1-}$1 bis $R^1$-54.

Eine bevorzugte Ausführungsform der Verbindungen der Formel (TA-3) sind die Verbindungen der folgenden Formel (TA-3a),

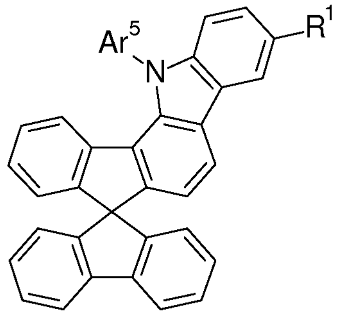

Formel (TA-3a)

wobei $Ar^5$ und $R^1$ die oben, insbesondere für Formeln (I) und/oder (TA-1), aufgeführten Bedeutungen aufweisen. Dabei sind bevorzugte Ausführungsformen der Gruppe $Ar^5$ die oben aufgeführten Strukturen $R^1$-1 bis $R^1$-92, besonders bevorzugt $R^1$-1 bis $R^1$-54.

Bevorzugte Biscarbazolderivate, die als Co-Host-Materialien zusammen mit den erfindungsgemäßen Verbindungen eingesetzt werden, sind ausgewählt aus den Verbindungen der folgenden Formel (TA-4),

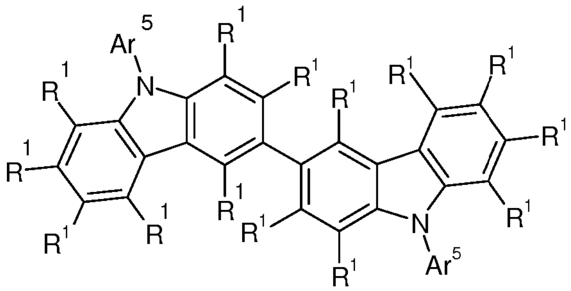

Formel (TA-4)

wobei $Ar^5$ und $R^1$ die oben, insbesondere für Formeln (I) und/oder (TA-1), aufgeführten Bedeutungen aufweisen. Dabei sind bevorzugte Ausführungsformen der Gruppe $Ar^5$ die oben aufgeführten Strukturen $R^1$-1 bis $R^1$-92, besonders bevorzugt $R^1$-1 bis $R^1$-54.

Eine bevorzugte Ausführungsform der Verbindungen der Formel (TA-4) sind die Verbindungen der folgenden Formel (TA-4a),

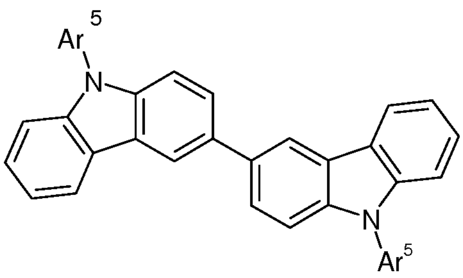

Formel (TA-4)

wobei $Ar^5$ die oben, insbesondere für Formel (TA-1), aufgeführten Bedeutungen aufweist. Dabei sind bevorzugte Ausführungsformen der Gruppe $Ar^5$ die oben aufgeführten Strukturen $R^1$-1 bis $R^1$-92, besonders bevorzugt $R^1$-1 bis $R^1$-54.

Beispiele für geeignete Biscarbazolderivate sind die in der folgenden Tabelle aufgeführten Materialien.

| | | |
|---|---|---|
| 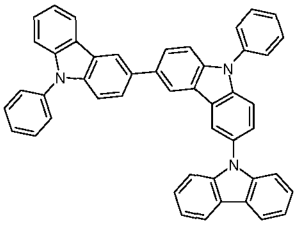 | 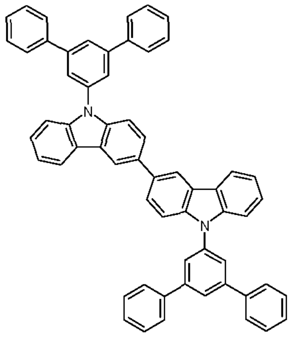 | 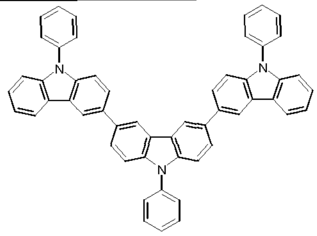 |
| 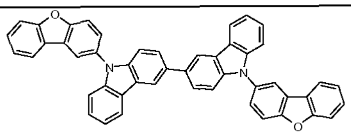 | 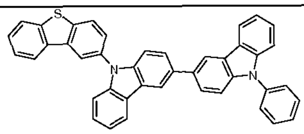 | 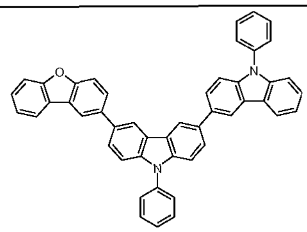 |
| 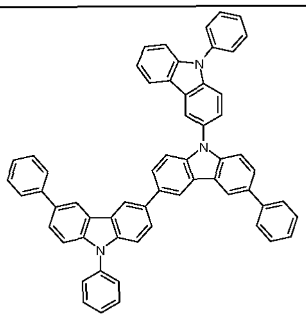 | 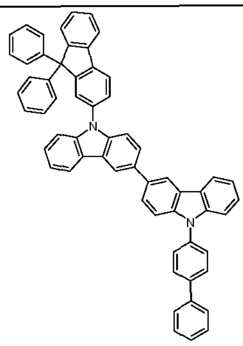 | 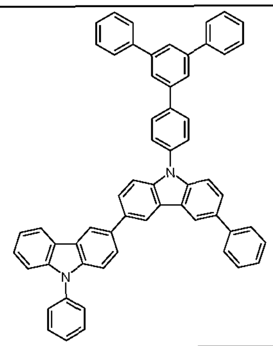 |
| 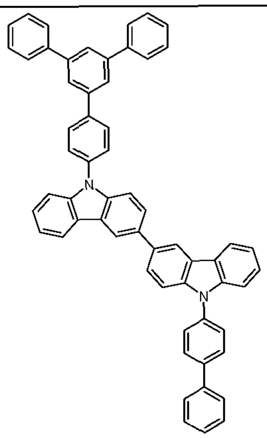 | 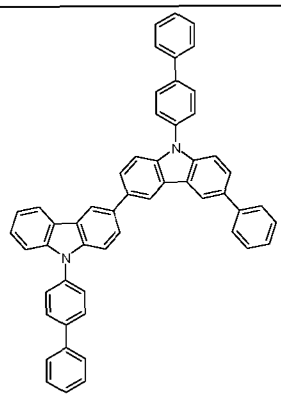 | 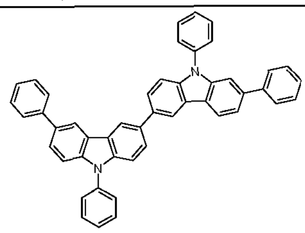 |
| 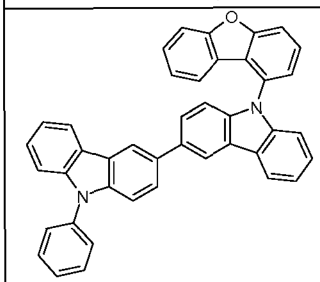 | 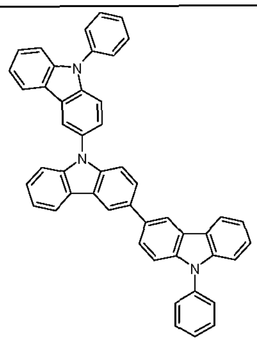 | 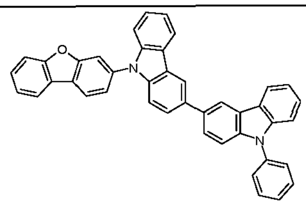 |

| | | |
|---|---|---|
| 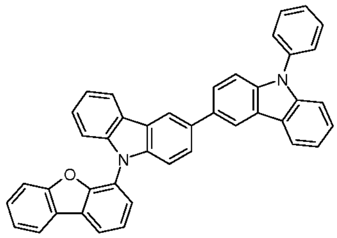 | 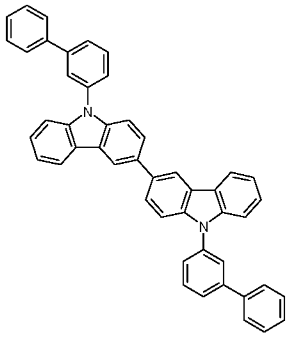 | 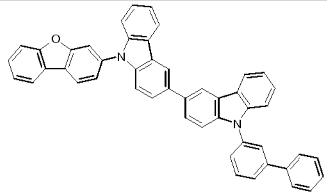 |
| 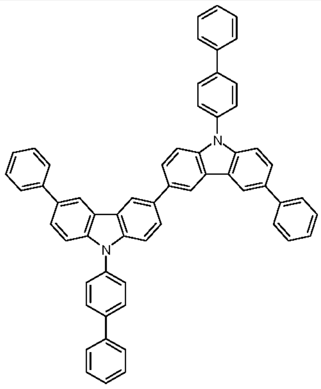 | 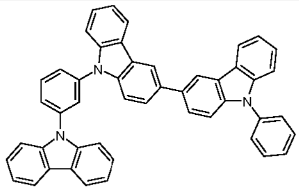 | 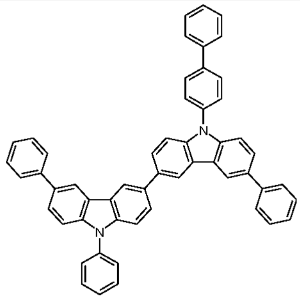 |
| 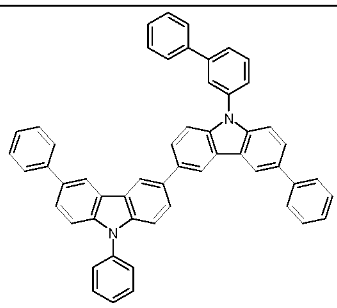 | 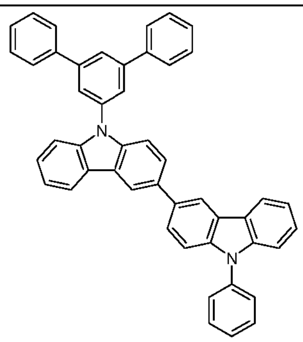 | 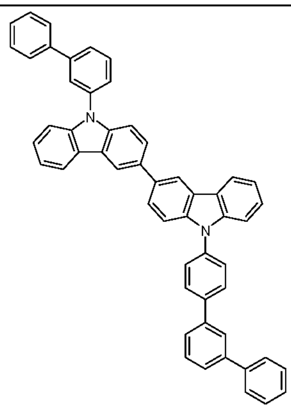 |
| 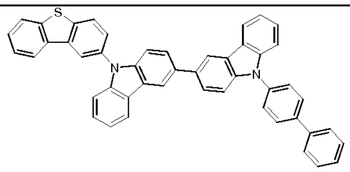 | 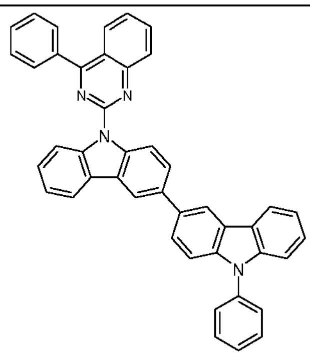 | 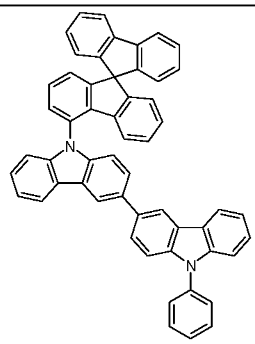 |

| | | |
|---|---|---|
| 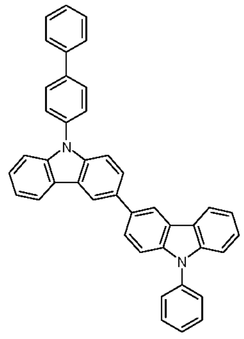 | 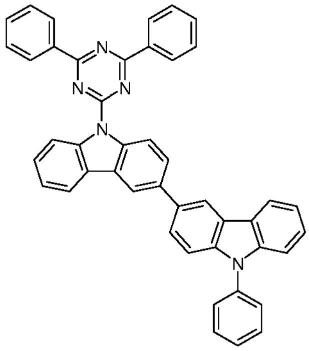 | 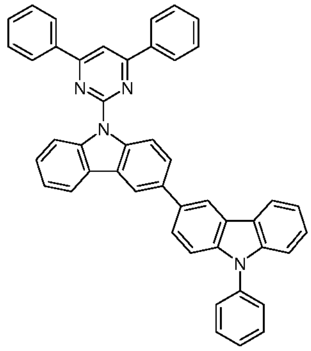 |

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

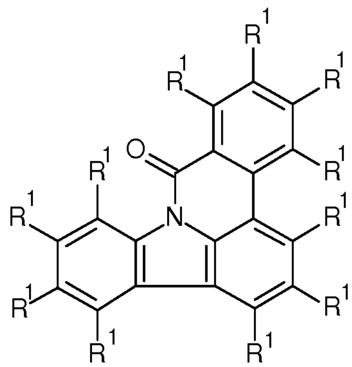

Formel (LAC-1)

wobei $R^1$ die oben, insbesondere für Formeln (I) aufgeführte Bedeutung aufweist.

Eine bevorzugte Ausführungsform der Verbindungen der Formel (LAC-1) sind die Verbindungen der folgenden Formel (LAC-1a),

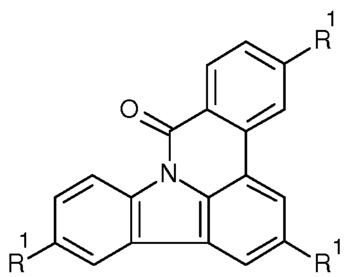

Formel (LAC-1a)

wobei $R^1$ die oben, insbesondere für Formel (I) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor, insbesondere für Formel (I) genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis $R^1$-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (I) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (I) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (I) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $CS_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die

Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $M0O_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung umfassend Strukturen gemäß Formel (I) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend erfindungsgemäße Verbindungen umfassend Strukturen gemäß Formel (I) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronische Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen, insbesondere als elektronenleitende Materialien und/oder Lochleitermaterialien oder als Matrixmaterialien, weisen eine sehr gute Lebensdauer auf. Dies gilt insbesondere für den Fall, dass die erfindungsgemäße Verbindung als Matrixmaterial für eine rot phosphoreszierende Verbindung, als Lochtransportmaterial oder als Elektronentransportmaterial eingesetzt wird.

2. Elektronische Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen insbesondere als Elektronentransportmaterialien, Lochleitermaterialien und/oder als Matrixmaterialien weisen eine hervorragende Effizienz auf. Insbesondere ist die Effizienz deutlich höher gegenüber analogen Verbindungen, die keine Struktureinheit gemäß Formel (I) enthalten. Hierbei bewirken die erfindungsgemäßen Verbindungen, Oligomere, Polymere oder Dendrimere mit Strukturen gemäß Formel (I) bzw. die zuvor und nachfolgend ausgeführten bevorzugten Ausführungsformen eine geringe Betriebsspannung bei Verwendung in elektronischen Vorrichtungen. Weiterhin bewirken diese Verbindungen insbesondere einen geringen

Roll-off, d.h. einen geringen Abfall der Leistungseffizienz der Vorrichtung bei hohen Leuchtdichten. Dies gilt insbesondere für den Fall, dass die erfindungsgemäße Verbindung als Matrixmaterial für eine rot phosphoreszierende Verbindung, als Lochtransportmaterial oder als Elektronentransportmaterial eingesetzt wird.

3. Elektronische Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen als Elektronentransportmaterialien, Lochleitermaterialien und/oder als Matrixmaterialien weisen eine hervorragende Farbreinheit auf.

4. Die erfindungsgemäßen Verbindungen, Oligomere, Polymere oder Dendrimere mit Strukturen gemäß Formel (I) bzw. die zuvor und nachfolgend ausgeführten bevorzugten Ausführungsformen zeigen eine sehr hohe thermische und photochemische Stabilität und führen zu Verbindungen mit einer sehr hohen Lebensdauer. Hierbei zeigen insbesondere Verbindungen mit einer Arylamin-, einer Fluoren- oder Spirobifluoreneinheit eine überraschend hohe Stabilität, so dass diese auch bei sehr effizienten und leistungsfähigen Verbindungen erhalten bleibt.

5. Mit Verbindungen, Oligomeren, Polymeren oder Dendrimeren mit Strukturen gemäß Formel (I) bzw. den zuvor und nachfolgend ausgeführten bevorzugten Ausführungsformen kann in elektronischen Vorrichtungen, insbesondere organische Elektrolumineszenzvorrichtungen, die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

6. Verbindungen, Oligomere, Polymere oder Dendrimere mit Strukturen gemäß Formel (I) bzw. die zuvor und nachfolgend ausgeführten bevorzugten Ausführungsformen weisen eine ausgezeichnete Glasfilmbildung auf.

7. Verbindungen, Oligomere, Polymere oder Dendrimere mit Strukturen gemäß Formel (I) bzw. die zuvor und nachfolgend ausgeführten bevorzugten Ausführungsformen bilden aus Lösungen sehr gute Filme.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als als Matrixmaterial, Lochleitermaterial, Elektroneninjektionsmaterial und/oder Elektronentransportmaterial, vorzugsweise als Matrixmaterial für eine rot phosphoreszierende Verbindung, Lochtransportmaterial oder Elektronentransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (I) bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbarte Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn, dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße Verbindungen und elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

## Synthesebeispiele

### a) Indolizino[3,4,5-ab]isoindol-2-boronsäure

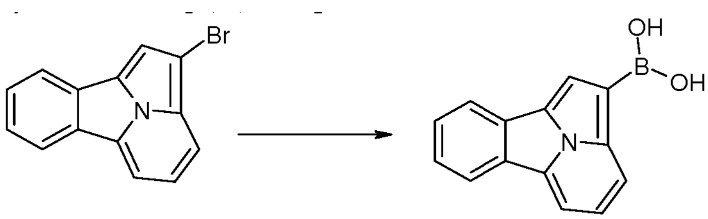

[811811-15-1 ]

197 g (729 mmol) 2-Brom-indolizino[3,4,5-*ab*]isoindol werden in 1500 mL trockenem THF gelöst und auf -78 °C gekühlt. Bei di eser Temperatur wird mit 305 ml (764 mmol / 2.5 M in Hexan) n-Butyllithium innerhalb von ca. 5 min. versetzt und anschließend für 2.5 h bei -78 °C nachgerührt. Bei dieser Temperatur wird mit 150 g (1455 mmol) Borsäuretrimethylester möglichst zügig versetzt und die Reaktion langsam auf Raumtemperatur kommen gelassen (ca. 18 h). Die Reaktionslösung wird mit Wasser gewaschen und der ausgefallene Feststoff und die organische Phase mit Toluol

azeotrop getrocknet. Das Rohprodukt wird aus Toluol/Methylenchlorid bei ca. 40 °C ausgerührt und abgesaugt. Aus beute: 148 g (529 mmol), 81 % d. Th..

**b) (2-Chlorphenyl)-indolizino[3,4,5-ab]isoindol-2-yl-amin**

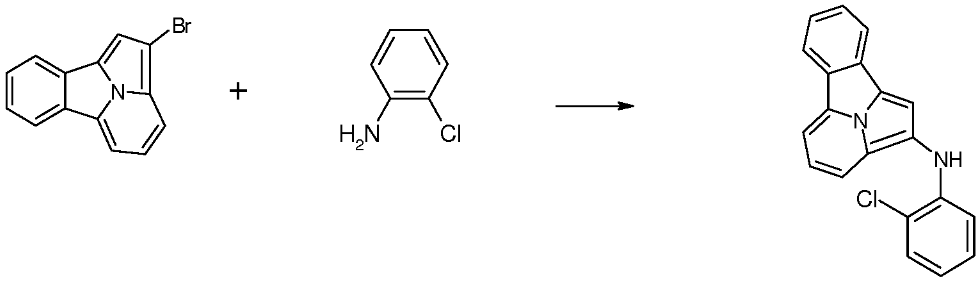

37 g (137 mmol) 2-Brom-indolizino[3,4,5-*ab*]isoindol, 17.5 g (137 mmol) 2-Chloranilin, 68.2 g (710 mmol) Natrium-tert-butylat, 613 mg (3 mmol) Palladium(II)acetat und 3.03 g (5 mmol) 1,1 '-Bis(diphenylphosphino)-ferrocen (dppf) werden in 1.3 L Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol/Heptan kristallisiert. Das Produkt wird als farbloser Feststoff isoliert. Ausbeute: 34 g (107 mmol), 80% d. Th..

Analog kann die folgende Verbindung hergestellt werden:

| | Edukt 1 | Edukt 2 | Produkt | Ausbeute |
|---|---|---|---|---|
| 1b | 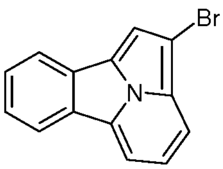 [811811-15-1 ] | 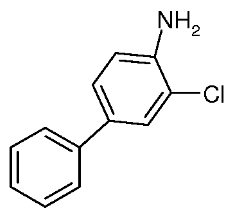 7285-66-7 | 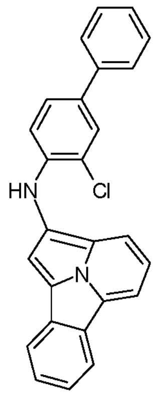 | 80% |

**c) Cyclisierung (Methode A)**

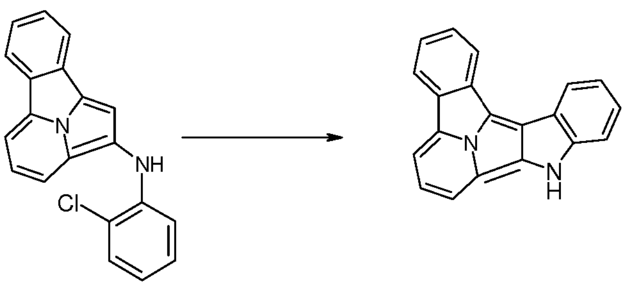

31.6 g (100 mmol) (2-Chlorphenyl)-indolizino[3,4,5-ab]isoindol-2-yl-amin, 56 g (409 mmol) Kaliumcarbonat, 4.5 g (12 mmol) Tricyclohexylphosphintetrafluoroborat und 1.38 g (6 mmol) Palladium(II)acetat werden in 500 mL Dimethylacetamid suspendiert und 6 h unter Rückfluss gerührt. Nach Erkalten wird die Reaktionsmischung mit 300 ml Wasser

und 400 mL $CH_2Cl_2$ erweitert. Man rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert. Ausbeute: 19 g (68 mmol), 68 % d. Th..

Analog kann die folgende Verbindung hergestellt werden:

| | Edukt | Produkt | Ausbeute |
|---|---|---|---|
| 1c | 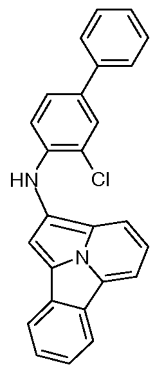 | 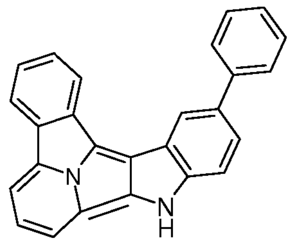 | 65% |

**d) 2-(2-Nitrophenyl)-indolizino[3,4,5-ab]isoindol**

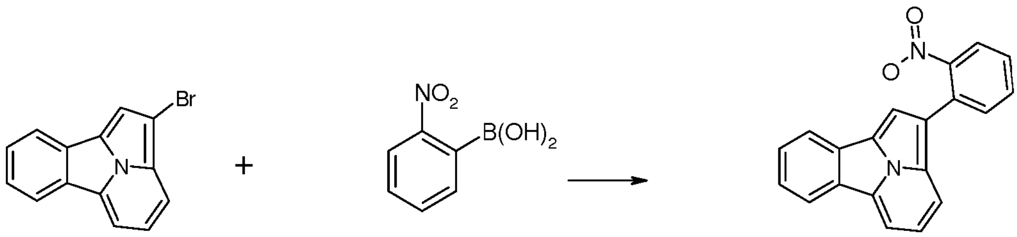

Eine gut gerührte, entgaste Suspension aus 30 g (184 mmol) B-(2-nitrophenyl)-boronsäure, 49 g (180 mmol) 2-Brom-indolizino[3,4,5-*ab*] isoindol und 66.5 g (212.7 mmol) Kaliumcarbonat in einem Gemisch aus 250 ml Wasser und 250 ml THF wird mit 1.7 g (1.49 mmol) $Pd(PPh_3)_4$ versetzt und 17 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und zur Trockene einrotiert. Der graue Rückstand wird aus Hexan umkristallisiert. Die ausgefallenen Kristalle werden abgesaugt, mit etwas MeOH gewaschen und im Vakuum getrocknet; Ausbeute: 47 g (150 mmol), 82% d. Th..

**e) Cyclisierung (Methode B)**

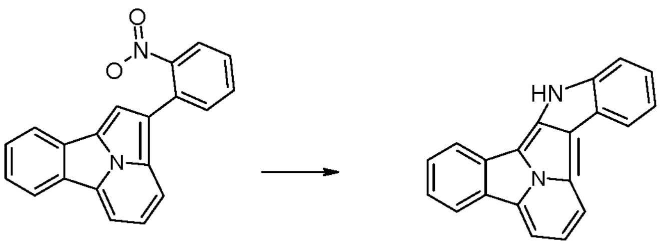

Eine Mischung aus 75 g (240 mmol) 2-(2-Nitro-phenyl)-indolizino[3,4,5-ab]isoindol und 290.3 ml (1669 mmol) Triethylphosphit wird 12 h unter Rückfluss erhitzt. Anschließend wird das restliche Triethylphosphit abdestilliert (72-76 °C / 9 mm Hg). Der Rückstand wir d mit Wasser/MeOH (1:1) versetzt, der Feststoff abfiltriert und aus Toluol umkristallisiert.

Ausbeute: 47g (167 mmol), 70 % d. Th..

**f) Nukleophile Substitution**

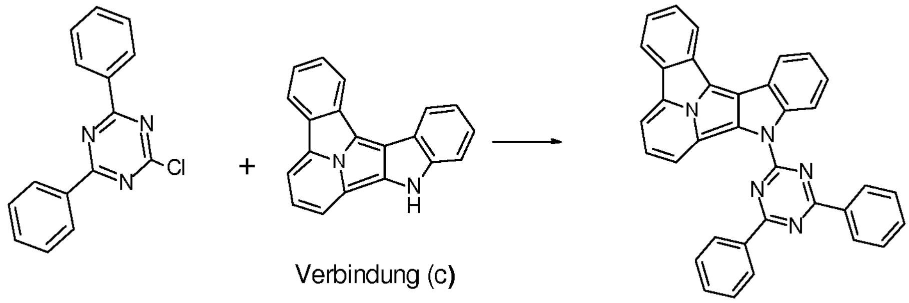

Verbindung (c)

4.2 g NaH (106 mmol, 60%ig in Mineralöl) werden in 300 mL Dimethylformamid unter Schutzatmosphäre gelöst. 29 g (106 mmol) von Verbindung **(c)** werden in 250 mL DMF gelöst und zu der Reaktionsmischung getropft. Nach 1 h bei Raumtemperatur wird eine Lösung von 2-Chlor-4,6-diphenyl-[1,3,5]triazin (34.5 g, 0.122 mol) in 200 mL THF zugetropft. Das Reaktionsgemisch wird 12 h bei Raumtemperatur gerührt und dann auf Eis gegossen. Der dabei ausgefallene Feststoff wird nach Erwärmen auf Raumtemperatur filtriert und mit Ethanol und Heptan gewaschen. Der Rückstand wird mit Toluol heiß extrahiert, aus Toluol/n-Heptan umkristallisiert und abschließend im Hochvakuum sublimiert. Die Reinheit beträgt 99.9%. Die Ausbeute beträgt 32 g (63 mmol; 60 % d. Th.).

Analog können die folgenden Verbindungen hergestellt werden:

| | Edukt1 | Edukt 2 | Produkt | Ausbeute |
|---|---|---|---|---|
| 1f | 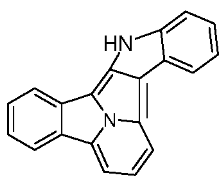 | 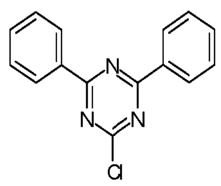 3842-55-5 | 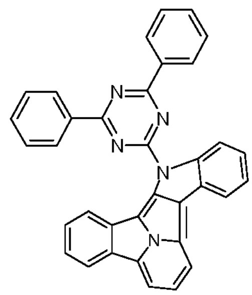 | 61% |
| 2f | 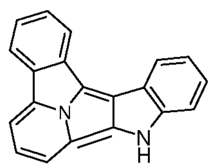 | 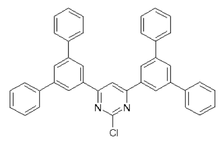 1384480-21-0 | 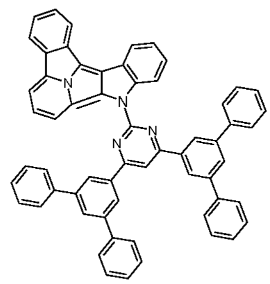 | 60% |

(fortgesetzt)

| | Edukt1 | Edukt 2 | Produkt | Ausbeute |
|---|---|---|---|---|
| 3f | 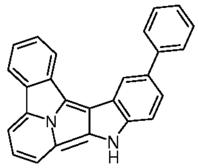 | 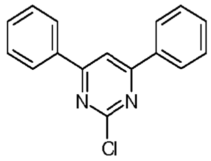 2915-16-4 | 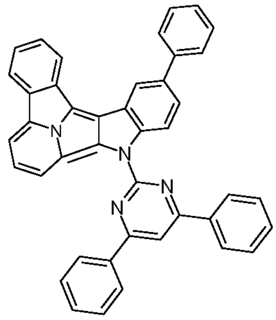 | 57% |
| 4f | 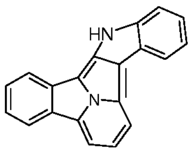 | 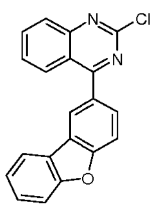 [1616499-38-7] | 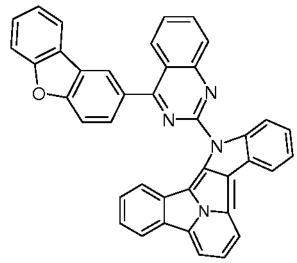 | 61% |
| 5f | 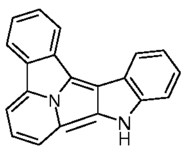 | 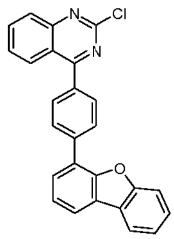 [1403252-58-3] | 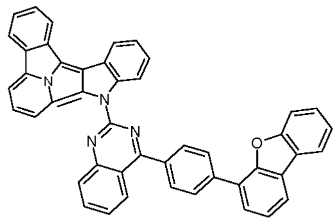 | 55% |
| 6f | 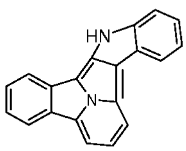 | 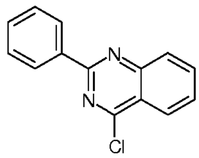 [6484-25-9] | 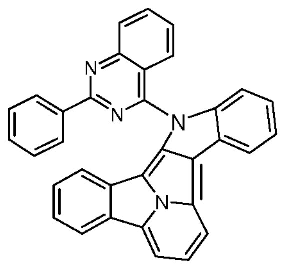 | 58% |
| 7f | 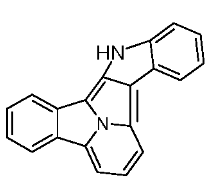 | 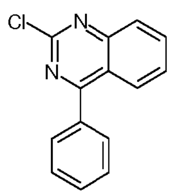 [29874-83-7] | 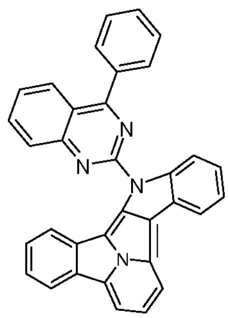 | 54% |

(fortgesetzt)

| | Edukt1 | Edukt 2 | Produkt | Ausbeute |
|---|---|---|---|---|
| 8f | 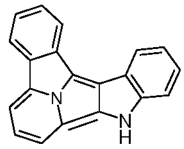 | 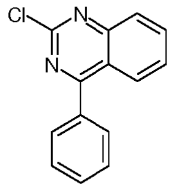 [29874-83-7] | 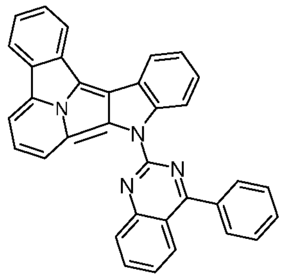 | 62% |
| 9f | 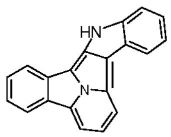 | 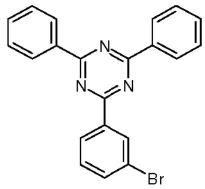 864377-31-1 | 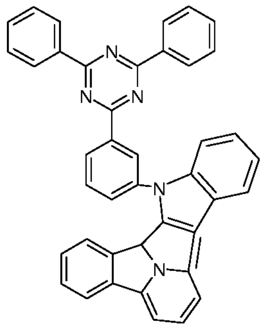 | 60% |

**g) Buchwald-Kupplung**

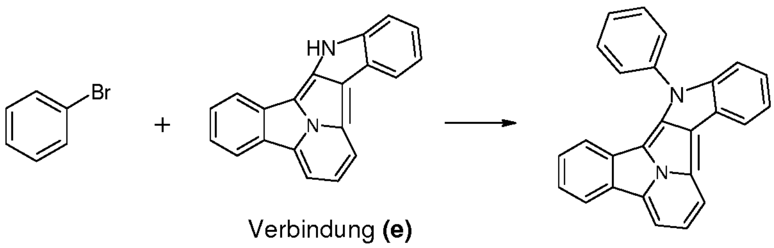

Verbindung **(e)**

15.1 g (50 mmol) Verbindung **(e)** und 8.4 g (54 mmol) Brombenzol werden in 400 ml Toluol unter Argonatmosphäre gelöst. 1.0 g (5 mmol) Tri-tert-butylphosphin werden zugegeben und unter Argonatmosphäre gerührt. 0.6 g (2 mmol) $Pd(OAc)_2$ werden zugegeben und unter Argonatmosphäre gerührt, wonach 9.5 g (99 mmol) Natium-tert-butanolat zugegeben werden. Das Reaktionsgemisch wird unter Rückfluss 24 h gerührt. Nach dem Abkühlen wird die organische Phase getrennt, mit 200 ml Wasser dreimal gewaschen, über $MgSO_4$ getrocknet, filtriert und das Lösungsmittel im Vakuum entfernt. Der Rückstand wird durch Säulenchromatographie über Kieselgel (Eluent: DCM/Heptan (1:3)) gereinigt. Der Rückstand wird mit Toluol heiß extrahiert, aus Toluol/n-Heptan umkristallisiert und abschließend im Hochvakuum sublimiert. Die Ausbeute beträgt 16.9 g (47 mmol), 88 % d. Th..

Analog können die folgenden Verbindungen hergestellt werden:

| | Edukt 1 | Edukt 2 | Produkt | Ausbeute |
|---|---|---|---|---|
| 1g | 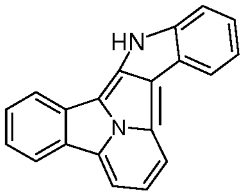 | 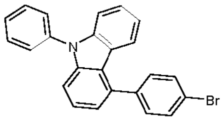 [1505512-86-6 ] | 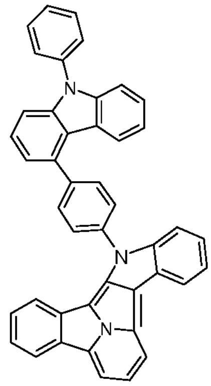 | 71% |
| 2g | 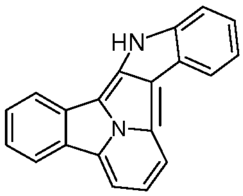 | 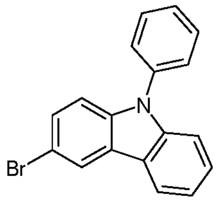 1153-85-1 | 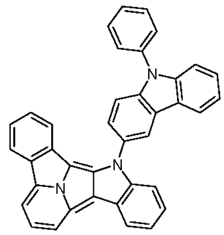 | 75% |
| 3g | 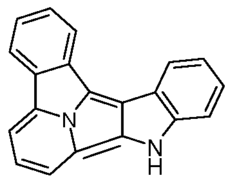 | 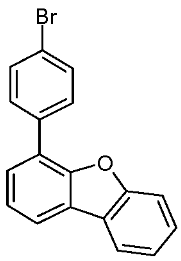 [1225053-54-2] | 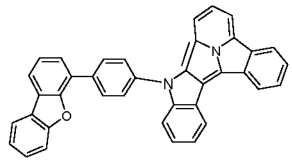 | 74% |
| 4g | 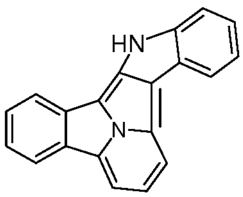 | 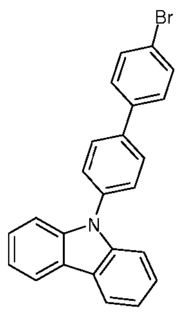 212385-73-4 | 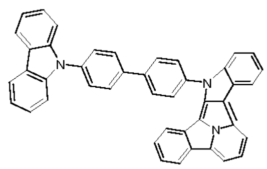 | 72% |
| 5g | 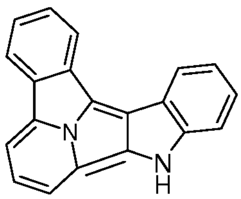 | 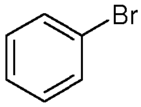 | 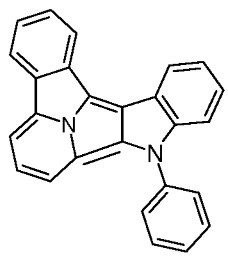 | 78% |

(fortgesetzt)

| | Edukt 1 | Edukt 2 | Produkt | Ausbeute |
|---|---|---|---|---|
| 6g | 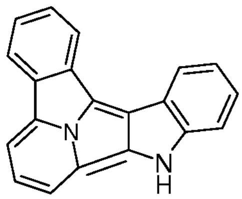 | 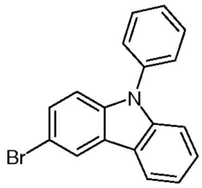 | 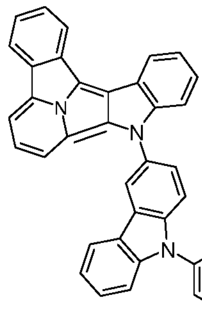 | 70% |
| 7g | 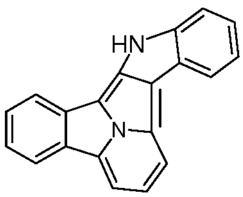 | 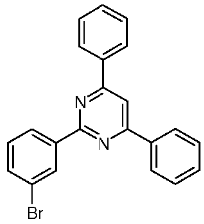 [864377-22-0 ] | 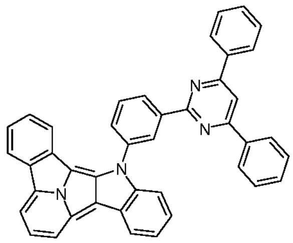 | 71% |

## h) Bromierung

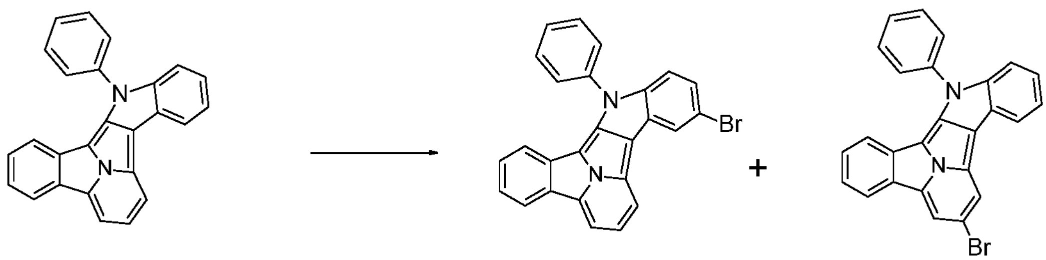

Verbindung g Verbindung ha Verbindung hb

67 g (187 mmol) Verbindung **g** werden in 2000 mL Essigsäure (100%) und 2000 mL Schwefelsäure (95-98%) suspendiert. Zu dieser Suspension werden portionsweise 34 g (190 mmol) NBS zugegeben und 2 h in Dunkelheit gerührt. Danach wird mit Wasser/Eis versetzt, der Feststoff abgetrennt und mit Ethanol gewaschen. Die isomeren Verbindungen ha und hb werden durch Umkristallisation getrennt. Die Ausbeute beträgt 51 g (117 mmol), 62% (ha) und 25 g (58 mmol), 31% (hb), entsprechend 93 % der Theorie.

Analog können die folgenden Verbindungen hergestellt werden:

| | Edukt 1 | Produkt 1 | Produkt 2 | Ausbeute (Verhältnis 1:2) |
|---|---|---|---|---|
| 1h | 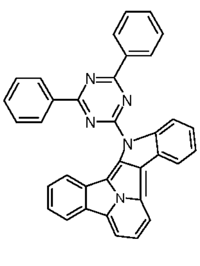 | 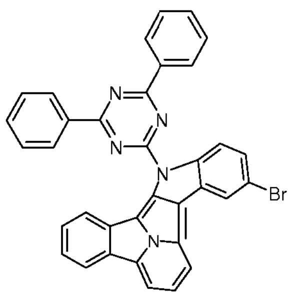 | 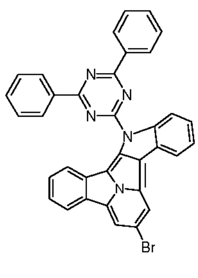 | 68%: 24% |

(fortgesetzt)

| | Edukt 1 | Produkt 1 | Produkt 2 | Ausbeute (Verhältnis 1:2) |
|---|---|---|---|---|
| 2h | 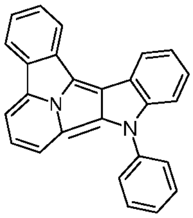 | 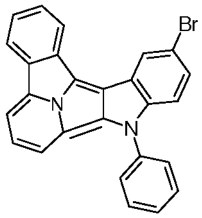 | 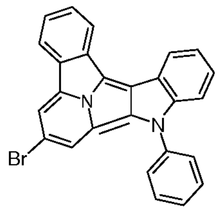 | 70%: 20% |

**j) Suzuki-Kupplung**

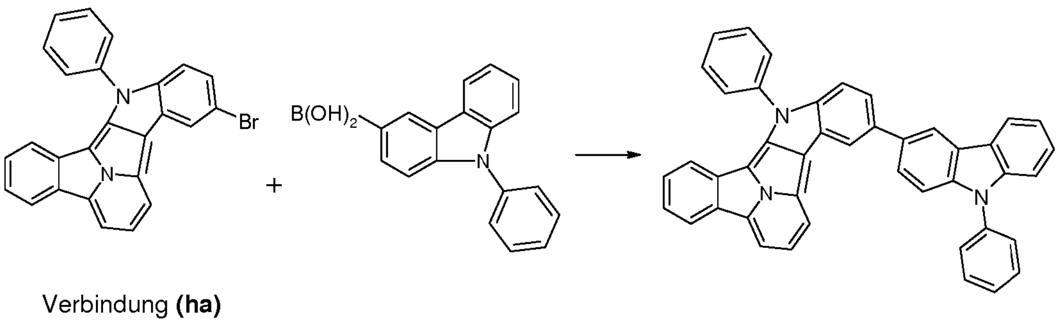

Verbindung **(ha)**

67 g (155 mmol) Verbindung **(ha),** 50 g (172 mmol) N-Phenyl-carbazol-3-boronsäure und 36 g (340 mmol) Natriumcarbonat werden in 1000 ml Ethylenglycoldimethylether und 280 ml Wasser suspendiert. Zu dieser Suspension werden 1.8 g (1.5 mmol) Tetrakis(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Die Ausbeute beträgt 56 g (94 mmol), 61 % d. Th..

Analog können die folgenden Verbindungen hergestellt werden:

| | Edukt 1 | Edukt 2 | Produkt | Ausbeute |
|---|---|---|---|---|
| 1j | 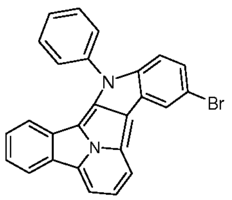 | 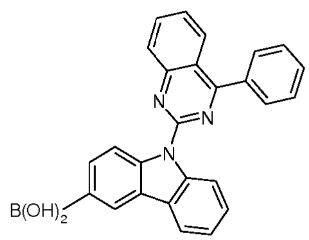 [1642121-58-1 ] | 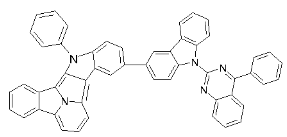 | 57% |

(fortgesetzt)

| | Edukt 1 | Edukt 2 | Produkt | Ausbeute |
|---|---|---|---|---|
| 2j | 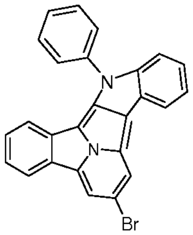 | 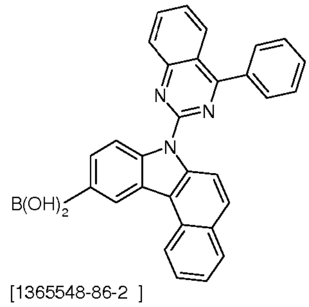 [1365548-86-2 ] | 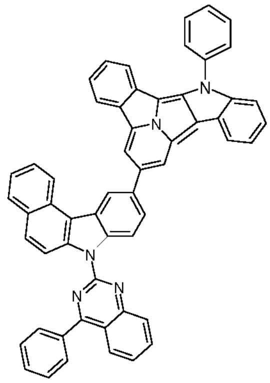 | 60% |
| 3j | 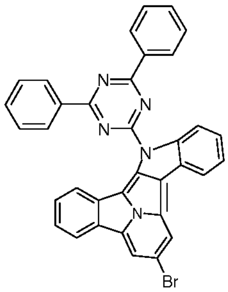 | 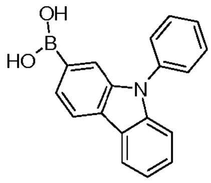 [1001911-63-2 ] | 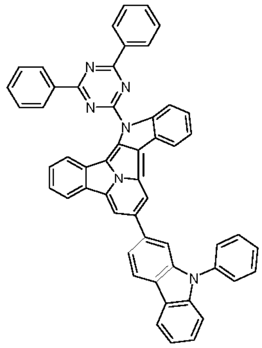 | 64% |
| 4j | 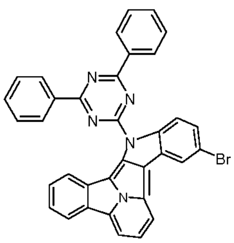 | 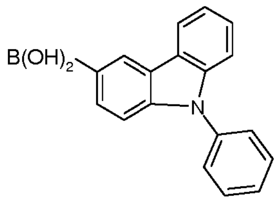 | 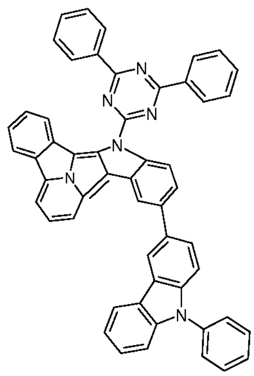 | 59% |
| 5j | 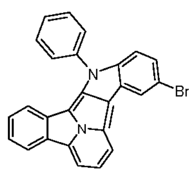 | 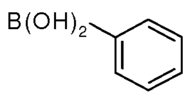 | 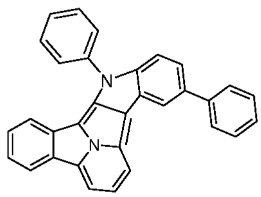 | 79% |
| 6j | 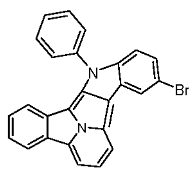 | 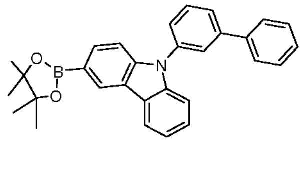 [1416814-68-0 ] | 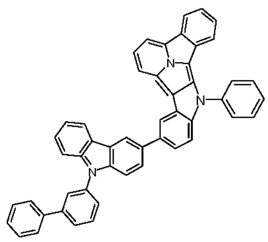 | 56% |

(fortgesetzt)

| | Edukt 1 | Edukt 2 | Produkt | Ausbeute |
|---|---|---|---|---|
| 7j | 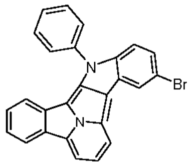 | 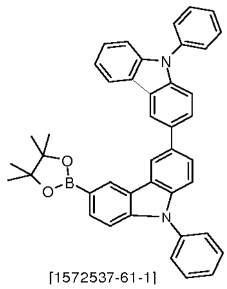 [1572537-61-1] | 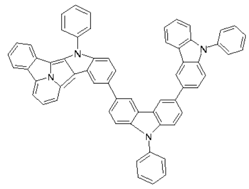 | 72% |
| 8j | 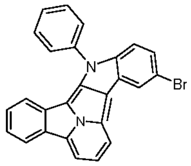 | 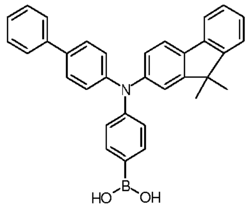 [1265177-27-2 ] | 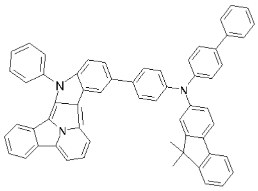 | 69% |
| 9j | 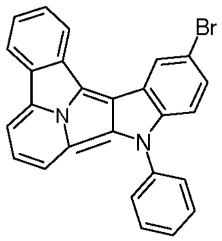 | 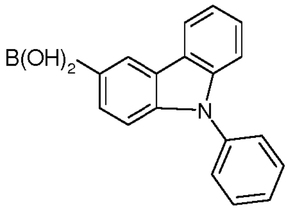 | 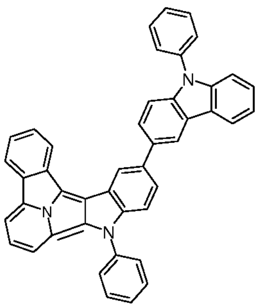 | 65% |
| 10j | 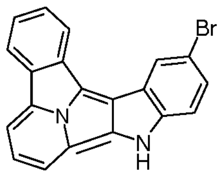 | 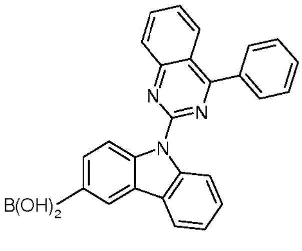 [1642121-58-1 ] | 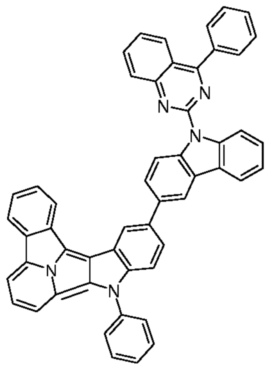 | 61% |
| 11j | 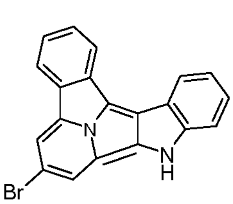 | 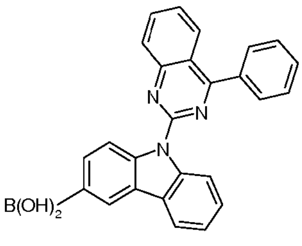 [1642121-58-1 ] | 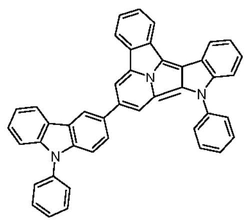 | 57% |

(fortgesetzt)

| | Edukt 1 | Edukt 2 | Produkt | Ausbeute |
|---|---|---|---|---|
| 12j | 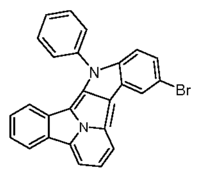 | 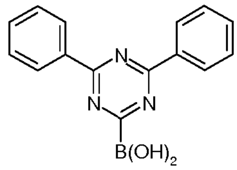 [1251825-65-6 ] | 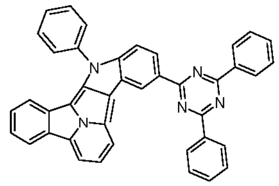 | 61% |

## Herstellung der OLEDs

In den folgenden Beispielen E1 bis E7 (siehe Tabelle 1) wird der Einsatz der erfindungsgemäßen Materialien in OLEDs vorgestellt.

**Vorbehandlung für die Beispiele E1-E7:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1 :IC2:TER1 (50%:45%:5%) bedeutet hierbei, dass das Material IC1 in einem Volumenanteil von 50%, IC2 in einem Volumenanteil von 45% und TER1 in einem Volumenanteil von 5% in der Schicht vorliegt. Analog kann auch die Elektronentransportschicht aus einer Mischung von zwei Materialien bestehen. Die OLEDs werden standardmäßig charakterisiert. Die Elektrolumineszenzspektren werden bei einer Leuchtdichte von 1000 cd/$m^2$ bestimmt und daraus die CIE 1931 x und y Farbkoordinaten berechnet.

## Verwendung von erfindungsgemäßen Mischungen in OLEDs

Die erfindungsgemäßen Materialien können in der Emissionsschicht in phosphoreszierenden roten OLEDs eingesetzt werden. Die erfindungsgemäßen Verbindungen 1f bis 12j werden in den Beispielen E1 bis E7 als Matrixmaterial in der Emissionsschicht eingesetzt. Die Farbkoordinaten der Elektrolumineszenzspektren der OLEDs liegen bei CIEx=0.67 und CIEy= 0.33. Diese Beispiele zeigen, dass sich die erfindungsgemäßen Materialien für den Einsatz in der Emissionsschicht von roten OLEDs eignen.

Des Weiteren lassen sich die erfindungsgemäßen Materialien in der Elektronenblockierschicht (EBL) erfolgreich einsetzen. Dies ist in Beispiel E5 gezeigt. Auch hier liegen die Farbkoordinaten des Spektrums der OLED jeweils bei CIEx=0.67 und CIEy= 0.33.

Tabelle 1: Aufbau der OLEDs

| Bsp. | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 125nm | SpMA2 10nm | 1f:TER1 (95%:5%) 40nm | --- | ST1:LiQ (50%: 50%) 35nm | --- |
| E2 | HATCN 5nm | SpMA1 125nm | SpMA2 10nm | 7f:TER1 (95%:5%) 40nm | --- | ST1:LiQ (50%: 50%) 35nm | --- |
| E3 | HATCN 5nm | SpMA1 125nm | SpMA2 10nm | 7g:TER1 (95%:5%) 40nm | --- | ST1:LiQ (50%: 50%) 35nm | --- |

(fortgesetzt)

| Bsp. | HIL Dicke | HTL Dicke | EBL Dicke | EML Dicke | HBL Dicke | ETL Dicke | EIL Dicke |
|---|---|---|---|---|---|---|---|
| E4 | HATCN 5nm | SpMA1 125nm | SpMA2 10nm | IC1 :2g:TER1 (50%:45%: 5%) 40nm | --- | ST1:LiQ (50%: 50%) 35nm | --- |
| E5 | HATCN 5nm | SpMA1 125nm | 2g 10nm | IC1 :2g:TER1 (50%:45%: 5%) 40nm | --- | ST1:LiQ (50%: 50%) 35nm | --- |
| E6 | HATCN 5nm | SpMA1 125nm | SpMA2 10nm | 12j:TER1 (95%:5%) 40nm | --- | ST1:LiQ (50%: 50%) 35nm | --- |
| E7 | HATCN 5nm | SpMA1 125nm | SpMA2 10nm | IC2:12j:TER1 (50%:45%: 5%) 40nm | --- | ST1:LiQ (50%: 50%) 35nm | --- |

Tabelle 2: Strukturformeln der Materialien für die OLEDs

| | |
|---|---|
| 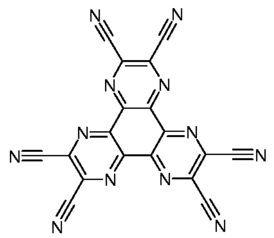 | 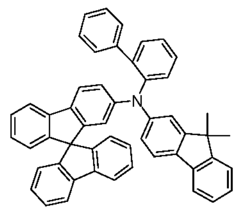 |
| HATCN | SpMA1 |
| 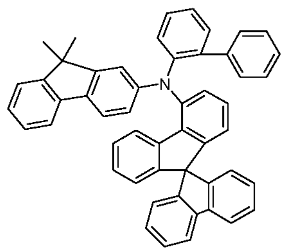 | 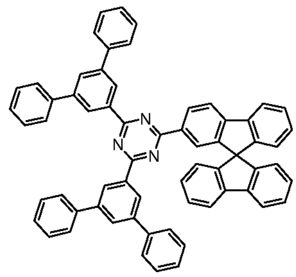 |
| SpMA2 | ST1 |
| 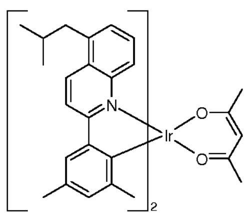 | 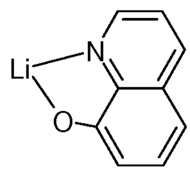 |
| TER1 | LiQ |
| 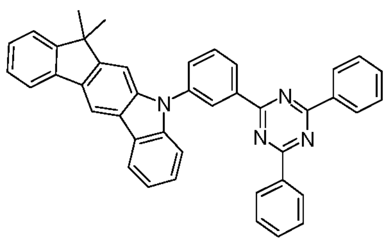 | 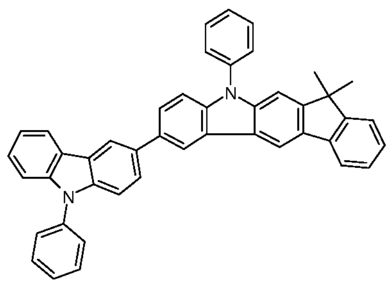 |
| IC1 | IC2 |

(fortgesetzt)

| | |
|---|---|
| 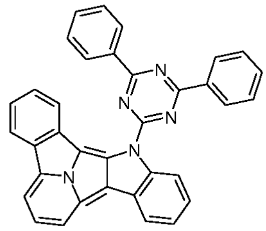 | 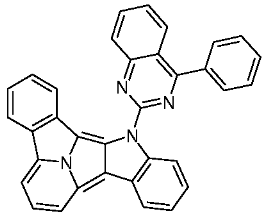 |
| 1f | 7f |
| 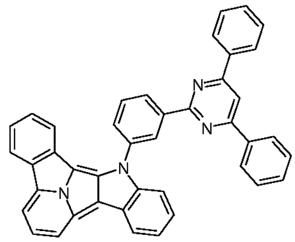 | 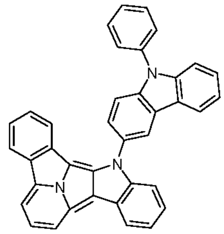 |
| 7g | 2g |
| 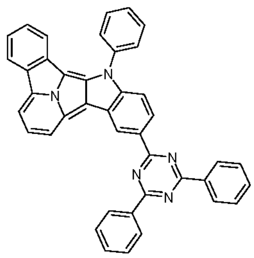 | |
| 12j | |

**Patentansprüche**

1. Verbindung, umfassend mindestens eine Struktur der Formel (I),

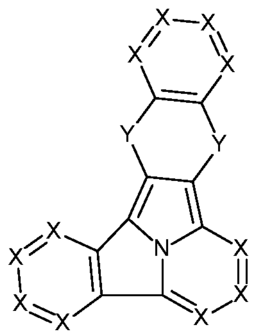

Formel (I)

wobei für die verwendeten Symbole gilt:

Y ist bei jedem Auftreten gleich oder verschieden eine Bindung oder $NR^1$, NAr, O, S, $C(R^1)_2$, $CArR^1$, $C(Ar)_2$, $Si(Ar)_2$, $SiArR^1$ oder $Si(R^1)_2$, wobei mindestens eine Gruppe Y ausgewählt ist aus $NR^1$, NAr, O, S, $C(R^1)_2$, $CArR^1$, $C(Ar)_2$, $Si(Ar)_2$, $SiArR^1$ oder $Si(R^1)_2$, vorzugsweise aus $NR^1$ oder NAr;

X ist bei jedem Auftreten gleich oder verschieden N oder $CR^1$;

Ar ist bei jedem Auftreten gleich oder verschieden ein aromatisches oder heteroaromatisches Ringsystem mit 5 bis 60 aromatischen Ringatomen, das mit einem oder mehreren Resten $R^1$ substituiert sein kann, dabei können zwei Reste Ar, welche an dasselbe C-Atom oder Si-Atom binden, auch durch eine Einfachbindung oder eine Brücke, ausgewählt aus $B(R^1)$, $C(R^1)_2$, $Si(R^1)_2$, C=O, $C=NR^1$, $C=C(R^1)_2$, O, S, S=O, $SO_2$, $N(R^1)$, $P(R^1)$ und $P(=O)R^1$, miteinander verbrückt sein;

$R^1$ ist bei jedem Auftreten gleich oder verschieden H, D, OH, $OR^2$, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder einer Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C≡C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen, die mit einem oder mehreren Resten $R^2$ substituiert sein kann, oder eine Kombination dieser Systeme; dabei können zwei oder mehrere, vorzugsweise benachbarte Reste $R^1$ miteinander ein Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^2$ substituiert sein kann, dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, $NO_2$, $C(=O)R^3$, $CR^3=C(R^3)2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C≡C-, $Si(R^3)_2$, $Ge(R^3)_2$, $Sn(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere, vorzugsweise benachbarte Substituenten $R^2$ auch miteinander ein Ringsystem bilden;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehrere, vorzugsweise benachbarte Substituenten $R^3$ auch miteinander ein Ringsystem bilden.

**2.** Verbindung gemäß Anspruch 1, umfassend mindestens eine Struktur der Formel (IIa) oder (IIb),

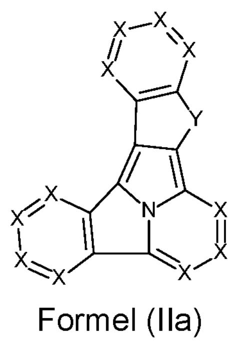

Formel (IIa)

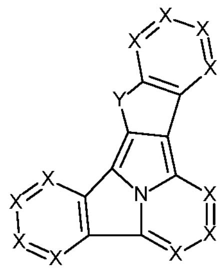

Formel (IIb)

wobei die verwendeten Symbole Y und X die in Anspruch 1 genannte Bedeutung aufweisen.

**3.** Verbindung gemäß mindestens einem der vorhergehenden Ansprüche, umfassend mindestens eine Struktur der Formel (IIIa), (IIIb), (IVa), (IVb), (Va) oder (Vb),

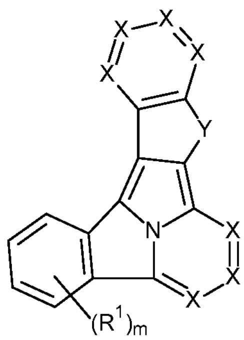

Formel (IIIa)

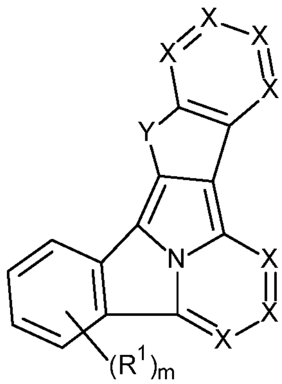

Formel (IIIb)

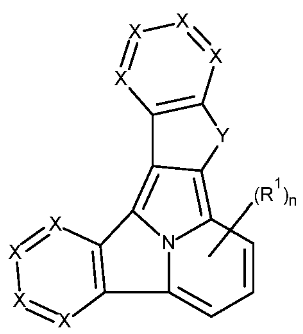

Formel (IVa)

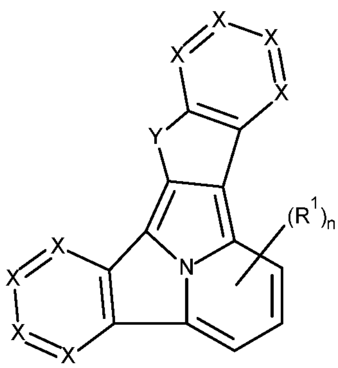

Formel (IVb)

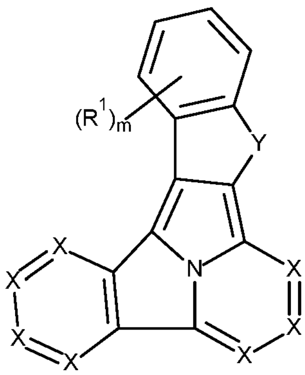

Formel (Va)

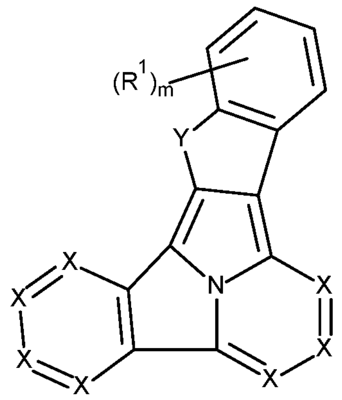

Formel (Vb)

wobei die Symbole $R^1$, Y und X die in Anspruch 1 genannte Bedeutung aufweisen, m 0, 1, 2, 3, oder 4 ist und n 0, 1, 2 oder 3 ist.

**4.** Verbindung gemäß mindestens einem der vorhergehenden Ansprüche, umfassend mindestens eine Struktur der Formel (VIa) oder (VIb),

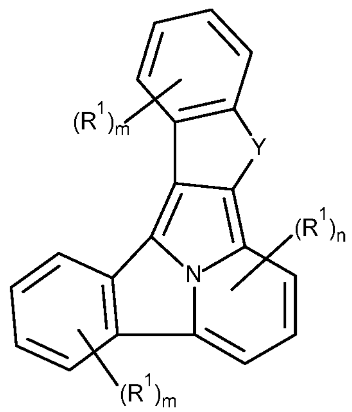

Formel (VIa)

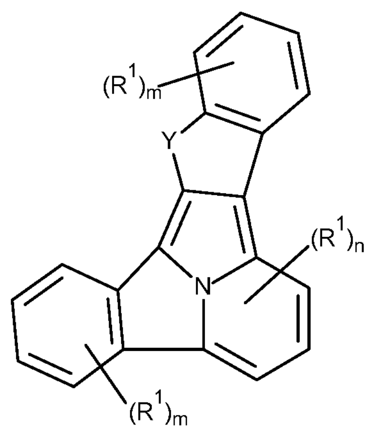

Formel (VIb)

wobei die Symbole $R^1$ und Y die in Anspruch 1 genannte Bedeutung aufweisen, m gleich oder verschieden bei jedem Auftreten 0, 1, 2, 3, oder 4 und n 0, 1, 2 oder 3 ist.

5. Verbindung gemäß mindestens einem der vorhergehenden Ansprüche, **dadurch gekennzeichnet, dass** die Verbindung eine Lochtransportgruppe umfasst, wobei vorzugsweise die Gruppe Ar, die in einer Gruppe Y enthalten ist, oder eine Gruppe $R^1$, die am Grundgerüst gebunden ist, eine Lochtransportgruppe umfasst, vorzugsweise darstellt.

6. Verbindung gemäß Anspruch 5, **dadurch gekennzeichnet, dass** die Lochtransportgruppe eine Gruppe umfasst, bevorzugt für eine Gruppe steht, die ausgewählt ist aus den Formeln (H-1) bis (H-3),

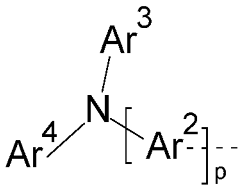

Formel (H-1)

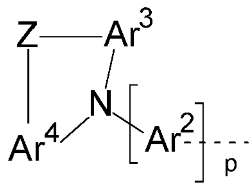

Formel (H-2)

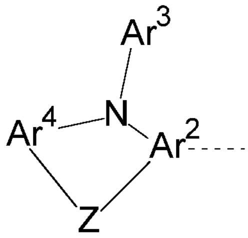

Formel (H-3)

wobei die gestrichelte Bindung die Anbindungsposition markiert und weiterhin gilt:

$Ar^2$, $Ar^3$, $Ar^4$ ist jeweils unabhängig ein aromatisches Ringsystem mit 6 bis 40 C-Atomen oder ein heteroaromatisches Ringsystem mit 3 bis 40 C-Atomen ist, welches jeweils durch einen oder mehrere Reste $R^1$ substituiert sein kann;
p ist 0 oder 1 ;
Z steht für $C(R^1)_2$, $Si(R^1)_2$, C=O, N-$Ar^1$, $BR^1$, $PR^1$, $POR^1$, SO, $SO_2$, Se, O oder S, wobei die Symbole $Ar^1$ und $R^1$ die in Anspruch 1 genannte Bedeutung aufweisen, wobei das Vorhandensein einer N-N-Bindung ausgeschlossen ist, so dass im Falle Y = NAr der Index p = 1 ist.

7. Verbindung gemäß Anspruch 5 oder 6, **dadurch gekennzeichnet, dass** die Lochtransportgruppe eine Gruppe umfasst, bevorzugt für eine Gruppe steht, die ausgewählt ist aus den Formeln (H-4) bis (H-26),

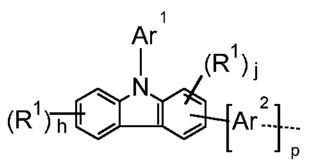

Formel (H-4)

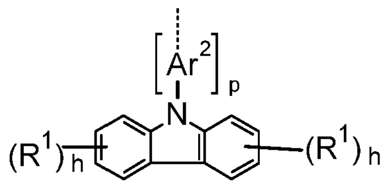

Formel (H-5)

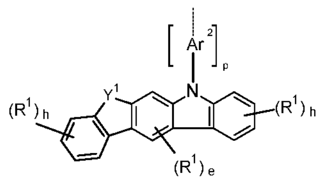

Formel (H-6)

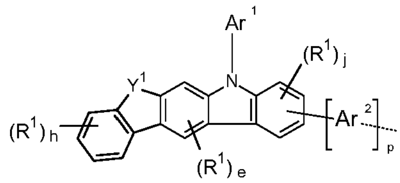

Formel (H-7)

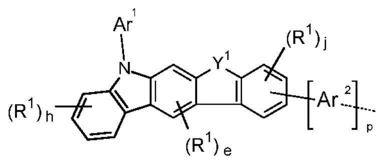

Formel (H-8)

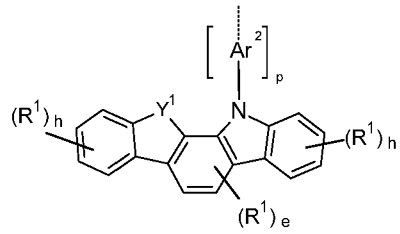

Formel (H-9)

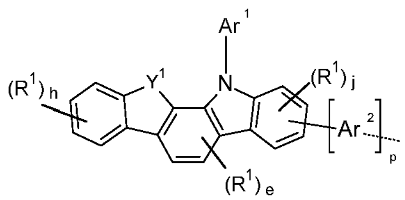

Formel (H-10)

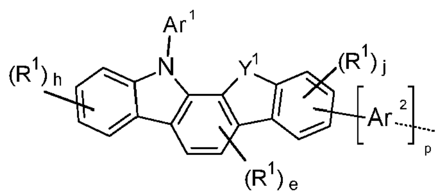

Formel (H-11)

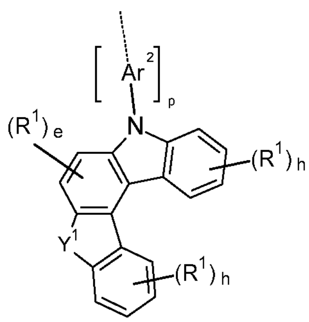

Formel (H-12)

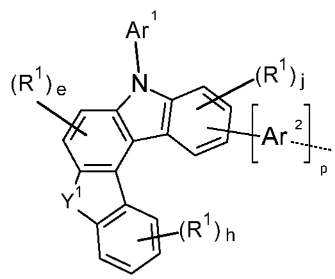

Formel (H-13)

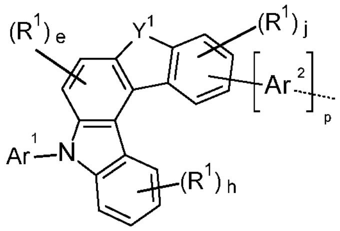

Formel (H-14)

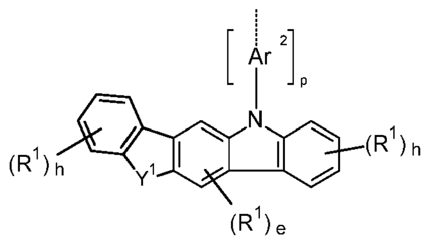

Formel (H-15)

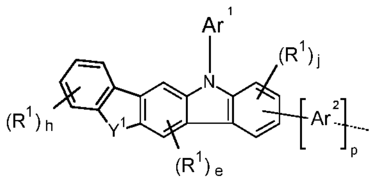

Formel (H-16)

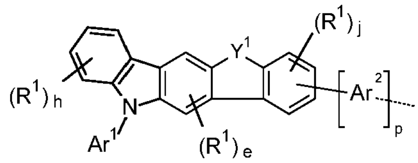

Formel (H-17)

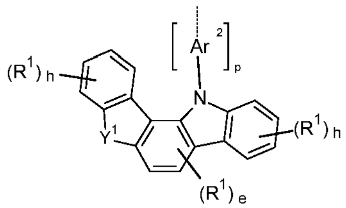

Formel (H-18)

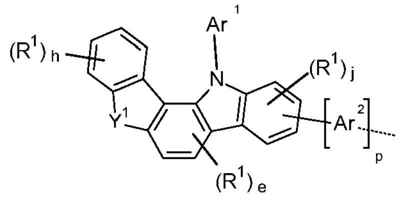

Formel (H-19)

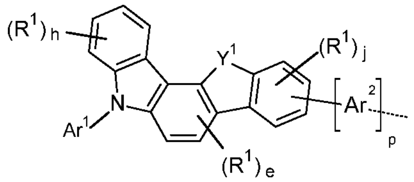

Formel (H-20)

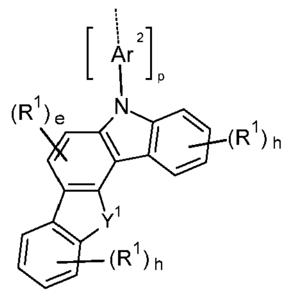

Formel (H-21)

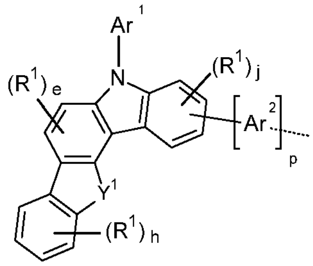

Formel (H-22)

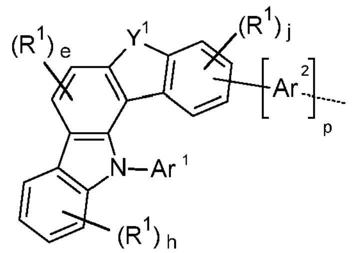

Formel (H-23)

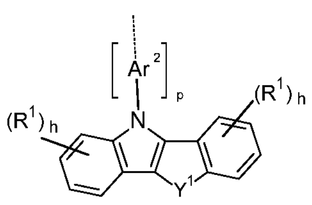

Formel (H-24)

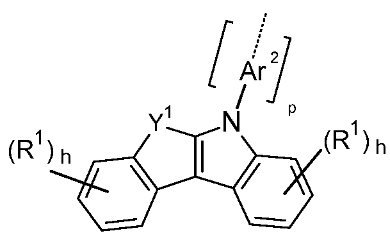

Formel (H-25)

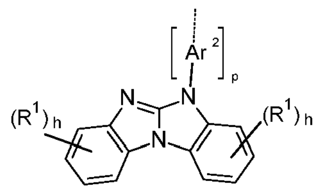

Formel (H-26)

wobei $Y^1$ O, S, $C(R^1)_2$ oder $NAr^1$ darstellt, die gestrichelte Bindung die Anbindungsposition markiert, e 0, 1 oder 2 ist, j 0, 1, 2 oder 3 ist, h 0, 1, 2, 3 oder 4 ist, p 0 oder 1 ist, $Ar^1$ und $R^1$ die in Anspruch 1 und $Ar^2$ die in Anspruch 6 genannte Bedeutung aufweist, wobei das Vorhandensein einer N-N-Bindung ausgeschlossen ist, so dass im Falle Y = NAr in den Formeln (H-5), (H-6), (H-9), (H-12), (H-15), (H-18), (H-21), (H-24), (H-25) und (H-26) der Index p = 1 ist.

**8.** Verbindung gemäß mindestens einem der vorhergehenden Ansprüche, **dadurch gekennzeichnet, dass** die Verbindung eine Elektronentransportgruppe umfasst, wobei vorzugsweise die Gruppe Ar, die in einer Gruppe Y enthalten ist, eine Elektronentransportgruppe umfasst, vorzugsweise darstellt.

**9.** Verbindung gemäß Anspruch 8, **dadurch gekennzeichnet, dass** die Gruppe Ar, die in einer Gruppe Y enthalten ist, oder eine Gruppe $R^1$, die am Grundgerüst gebunden ist, für eine Gruppe steht, die durch die Formel (QL) darstellbar ist,

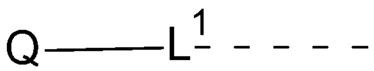

Formel (QL)

worin $L^1$ eine Bindung oder ein aromatisches oder heteroaromatisches Ringsystem mit 5 bis 40 aromatischen Ringatomen darstellt, welches durch einen oder mehrere Reste $R^1$ substituiert sein kann, und Q eine Elektronentransportgruppe ist, wobei $R^1$ die in Anspruch 1 genannte Bedeutung aufweist

**10.** Verbindung gemäß Anspruch 8 oder 9, **dadurch gekennzeichnet, dass** die Elektronentransportgruppe ausgewählt ist aus Strukturen der Formeln (Q-1), (Q-2), (Q-3), (Q-4), (Q-5), (Q-6), (Q-7), (Q-8), (Q-9) und/oder (Q-10),

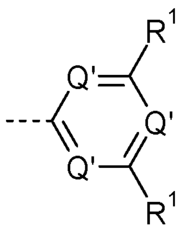

Formel (Q-1)

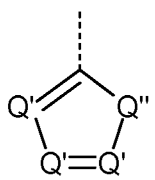

Formel (Q-2)

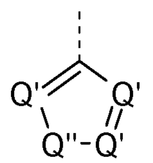

Formel (Q-3)

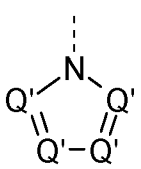

Formel (Q-4)

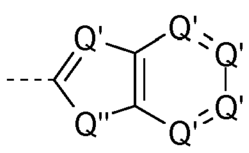

Formel (Q-5)

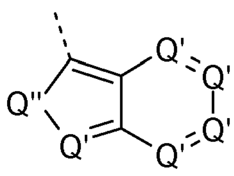

Formel (Q-6)

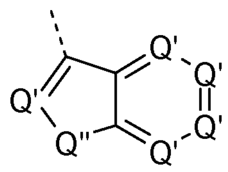

Formel (Q-7)

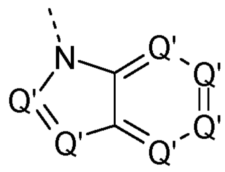

Formel (Q-8)

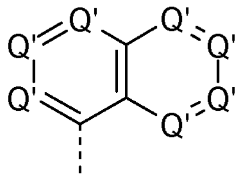

Formel (Q-9)

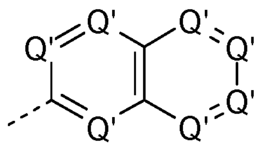

Formel (Q-10),

wobei die gestrichelte Bindung die Anbindungsposition markiert,

Q' bei jedem Auftreten gleich oder verschieden $CR^1$ oder N darstellt, wobei wenigstens ein Q' gleich N ist;
Q" $NR^1$, O oder S darstellt; und
$R^1$ wie in Anspruch 1 definiert ist.

**11.** Oligomer, Polymer oder Dendrimer enthaltend eine oder mehrere Verbindungen nach einem der Ansprüche 1 bis 10, wobei statt eines Wasserstoffatoms oder eines Substituenten ein oder mehrere Bindungen der Verbindungen zum Polymer, Oligomer oder Dendrimer vorhanden sind.

**12.** Zusammensetzung enthaltend wenigstens eine Verbindung nach einem oder mehreren der Ansprüche 1 bis 10 oder ein Oligomer, Polymer oder Dendrimer nach Anspruch 11 und wenigstens eine weitere Verbindung ausgewählt aus der Gruppe bestehend aus fluoreszierenden Emittern, phosphoreszierenden Emittern, Matrixmaterialien, Elektronentransportmaterialien, Elektroneninjektionsmaterialien, Lochleitermaterialien, Lochinjektionsmaterialien, Elektronenblockiermaterialien und Lochblockiermaterialien.

**13.** Formulierung, enthaltend mindestens eine Verbindung nach einem oder mehreren der Ansprüche 1 bis 10 oder ein Oligomer, Polymer oder Dendrimer nach Anspruch 11 oder eine Zusammensetzung nach Anspruch 12 und mindestens ein Lösemittel.

**14.** Verwendung einer Verbindung nach einem oder mehreren der Ansprüche 1 bis 10, eines Oligomers, Polymers oder Dendrimers nach Anspruch 11 oder einer Zusammensetzung nach Anspruch 12 in einer elektronischen Vorrichtung.

**15.** Verfahren zur Herstellung einer Verbindung nach einem oder mehreren der Ansprüche 1 bis 10 oder eines Oligomers, Polymers und/oder Dendrimers nach Anspruch 11, **dadurch gekennzeichnet, dass** in einer Kupplungsreaktion eine Verbindung, umfassend mindestens eine stickstoffhaltige heterocyclische Gruppe, mit einer Verbindung, umfassend mindestens eine aromatische oder heteroaromatische Gruppe, verbunden wird.

**16.** Elektronische Vorrichtung, enthaltend mindestens eine Verbindung nach einem oder mehreren der Ansprüche 1 bis 10, ein Oligomer, Polymer oder Dendrimer nach Anspruch 11 oder eine Zusammensetzung gemäß Anspruch 12.

**17.** Elektronische Vorrichtung nach Anspruch 16, wobei es sich um eine organische Elektrolumineszenzvorrichtung handelt, **dadurch gekennzeichnet, dass** die Verbindung nach einem oder mehreren der Ansprüche 1 bis 10 oder das Oligomer, Polymer oder Dendrimer nach Anspruch 11 als Matrixmaterial in einer emittierenden Schicht, als Lochtransportmaterial oder als Elektronentransportmaterial enthalten ist.

## Claims

**1.** Compound comprising at least one structure of the formula (I)

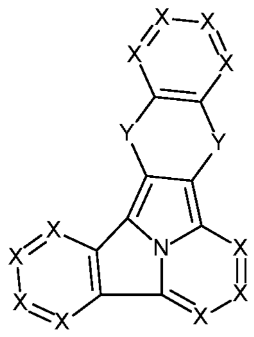

Formula (I)

where the symbols used are as follows:

Y is the same or different at each instance and is a bond or $NR^1$, NAr, 0, S, $C(R^1)_2$, $CArR^1$, $C(Ar)_2$, $Si(Ar)_2$, $SiArR^1$ or $Si(R^1)_2$, where at least one Y group is selected from $NR^1$, NAr, 0, S, $C(R^1)_2$, $CArR^1$, $C(Ar)_2$, $Si(Ar)_2$, $SiArR^1$ or $Si(R^1)_2$, preferably from $NR^1$ or NAr;
X is the same or different at each instance and is N or $CR^1$;
Ar is the same or different at each instance and is an aromatic or heteroaromatic ring system which has 5 to 60 aromatic ring atoms and may be substituted by one or more $R^1$ radicals; at the same time, two Ar radicals bonded to the same carbon atom or silicon atom may also be joined to one another by a single bond or a bridge selected from $B(R^1)$, $C(R^1)_2$, $Si(R^1)_2$, C=O, $C=NR^1$, $C=C(R^1)_2$, 0, S, S=O, $SO_2$, $N(R^1)$, $P(R^1)$ and $P(=O)R^1$;
$R^1$ is the same or different at each instance and is H, D, OH, $OR^2$, F, Cl, Br, I, CN, $NO_2$, $N(Ar^1)_2$, $N(R^2)_2$, $C(=)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, a straight-chain alkyl, alkoxy or thioalkoxy group having 1 to 40 carbon atoms or a branched or cyclic alkyl, alkoxy or thioalkoxy group having 3 to 40 carbon atoms or an alkenyl or alkynyl group having 2 to 40 carbon atoms, each of which may be substituted by one or more $R^2$ radicals, where one or more nonadjacent $CH_2$ groups may be replaced by $-R^2C=CR^2-$, -C≡C-, $Si(R^2)_2$, $Ge(R^2)_2$, $Sn(R^2)_2$, C=O, C=S, C=Se, $C=NR^2$, -C(=O)O-, $- C(=O)NR^2-$, $NR^2$, $P(=O)(R^2)$, -O-, -S-, SO or $SO_2$ and where one or more hydrogen atoms may be replaced by D, F, Cl, Br, I, CN or $NO_2$, or an aromatic or heteroaromatic ring system which has 5 to 40 aromatic ring atoms and may be substituted in each case by one or more $R^2$ radicals, or an aryloxy or heteroaryloxy group which has 5 to 40 aromatic ring atoms and may be substituted by one or more $R^2$ radicals, or an aralkyl or heteroaralkyl group which has 5 to 40 aromatic ring atoms and may be substituted by one or more $R^2$ radicals, or a combination of these systems; at the same time, two or more, preferably adjacent $R^1$ radicals may form a ring system with one another;
$Ar^1$ is the same or different at each instance and is an aromatic or heteroaromatic ring system which has 5 to 30 aromatic ring atoms and may be substituted by one or more nonaromatic $R^2$ radicals; at the same time, two $Ar^1$ radicals bonded to the same silicon atom, nitrogen atom, phosphorus atom or boron atom may also be joined to one another by a single bond or a bridge selected from $B(R^2)$, $C(R^2)_2$, $Si(R^2)_2$, C=O, $C=NR^2$, $C=C(R^2)_2$, 0, S, S=O, $SO_2$, $N(R^2)$, $P(R^2)$ and $P(=O)R^2$;
$R^2$ is the same or different at each instance and is H, D, F, Cl, Br, I, CN, $B(OR^3)_2$, $NO_2$, $C(=O)R^3$, $CR^3=C(R^3)_2$, $C(=O)OR^3$, $C(=O)N(R^3)2$, Si $(R^3)_3$, $P(R^3)2$, $B(R^3)2$, $N(R^3)_2$, $NO_2$, $P(=O)(R^3)_2$, $OSO_2R^3$, $OR^3$, $S(=O)R^3$, $S(=O)_2R^3$, a straight-chain alkyl, alkoxy or thioalkoxy group having 1 to 40 carbon atoms or a branched or cyclic alkyl, alkoxy or thioalkoxy group having 3 to 40 carbon atoms, each of which may be substituted by one or more $R^3$ radicals, where one or more nonadjacent $CH_2$ groups may be replaced by $- R^3C=CR^3-$, -C≡C-, Si $(R^3)_2$, $Ge(R^3)_2$, $Sn(R^3)_2$, C=O, C=S, $C=NR^3$, -C(=O)O-, $-C(=O)NR^3-$, $NR^3$, $P(=O)(R^3)$, -O-, -S-, SO or $SO_2$ and where one or more hydrogen atoms may be replaced by D, F, Cl, Br, I, CN or $NO_2$, or an aromatic or heteroaromatic ring system which has 5 to 40 aromatic ring atoms and may be substituted in each case by one or more $R^3$ radicals, or an aryloxy or heteroaryloxy group which has 5 to 40 aromatic ring atoms and may be substituted by one or more $R^3$ radicals, or a combination of these systems; at the same time, two or more, preferably adjacent $R^2$ substituents may also form a ring system with one another;
$R^3$ is the same or different at each instance and is selected from the group consisting of H, D, F, CN, an aliphatic hydrocarbyl radical having 1 to 20 carbon atoms or an aromatic or heteroaromatic ring system which has 5 to 30 aromatic ring atoms and in which one or more hydrogen atoms may be replaced by D, F, Cl, Br, I or CN and which may be substituted by one or more alkyl groups each having 1 to 4 carbon atoms; at the same time, two or more, preferably adjacent $R^3$ substituents may also form a ring system with one another.

**2.** Compound according to Claim 1, comprising at least one structure of the formula (IIa) or (IIb)

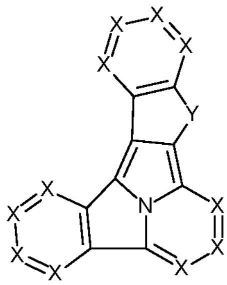

Formula (IIa)

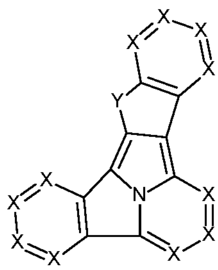

Formula (IIb)

where the symbols Y and X used have the definition given in Claim 1.

**3.** Compound according to at least one of the preceding claims, comprising at least one structure of the formula (IIIa), (IIIb), (IVa), (IVb), (Va) or (Vb)

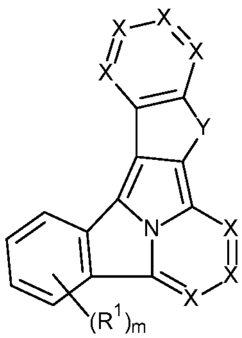

Formula (IIIa)

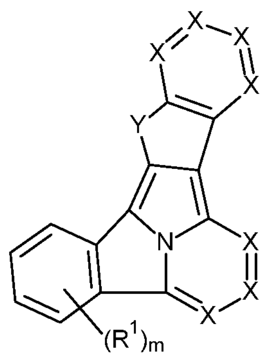

Formula (IIIb)

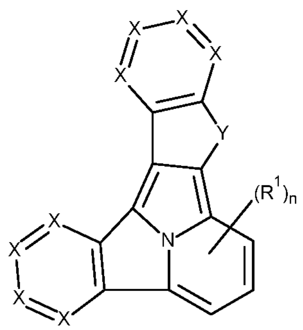

Formula (IVa)

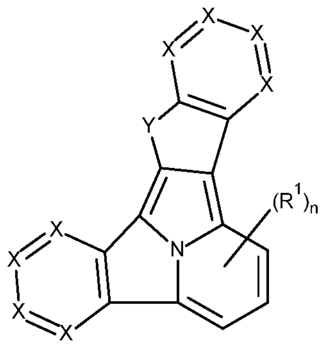

Formula (IVb)

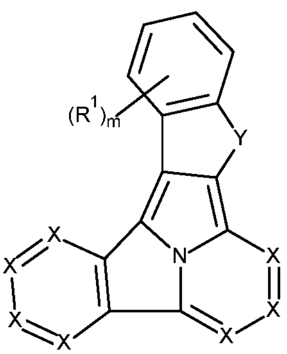

Formula (Va)

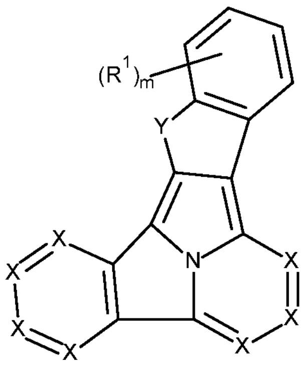

Formula (Vb)

where the symbols $R^1$, Y and X have the definition given in Claim 1, m is 0, 1, 2, 3 or 4, and n is 0, 1, 2 or 3.

4. Compound according to at least one of the preceding claims, comprising at least one structure of the formula (VIa) or (VIb)

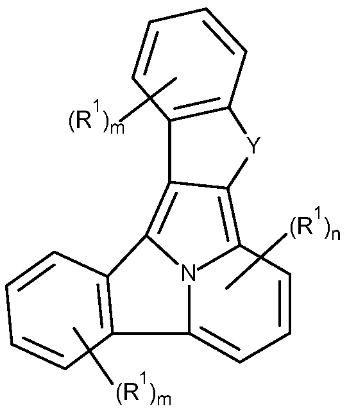

Formula (VIa)

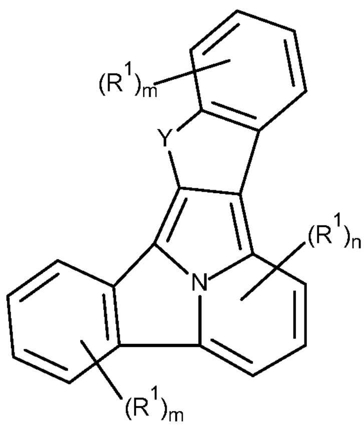

Formula (VIb)

where the symbols $R^1$ and Y have the definition given in Claim 1, m is the same or different at each instance and is 0, 1, 2, 3 or 4, and n is 0, 1, 2 or 3.

5. Compound according to at least one of the preceding claims, **characterized in that** the compound comprises a

hole transport group, it being preferable that the Ar group present in a Y group, or an $R^1$ group bonded to the base skeleton, comprises and preferably represents a hole transport group.

**6.** Compound according to Claim 5, **characterized in that** the hole transport group comprises a group and preferably is a group selected from the formulae (H-1) to (H-3)

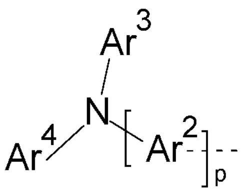

Formula (H-1)

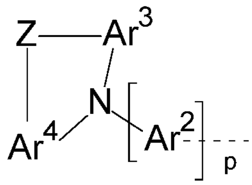

Formula (H-2)

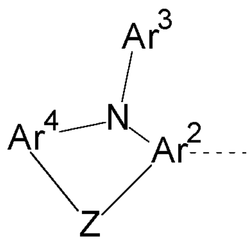

Formula (H-3)

where the dotted bond marks the position of attachment and in addition:

$Ar^2$, $Ar^3$, $Ar^4$ is in each case independently an aromatic ring system having 6 to 40 carbon atoms or a heteroaromatic ring system having 3 to 40 carbon atoms, each of which may be substituted by one or more $R^1$ radicals;
p is 0 or 1;
Z is $C(R^1)_2$, $Si(R^1)_2$, C=0, N-$Ar^1$, $BR^1$, $PR^1$, $POR^1$, SO, $SO_2$, Se, 0 or S, where the symbols $Ar^1$ and $R^1$ have the definition given in Claim 1, where the presence of an N-N bond is ruled out, such that, in the case that Y = NAr, the index p = 1.

**7.** Compound according to Claim 5 or 6, **characterized in that** the hole transport group comprises a group and preferably is a group selected from the formulae (H-4) to (H-26)

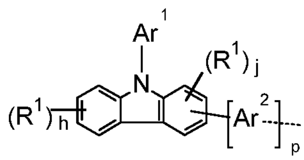

Formula (H-4)

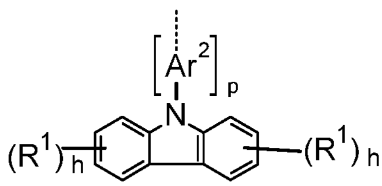

Formula (H-5)

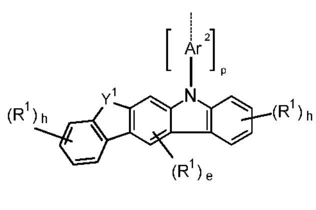

Formula (H-6)

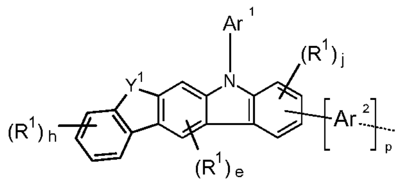

Formula (H-7)

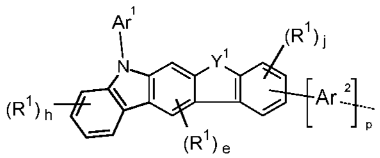

Formula (H-8)

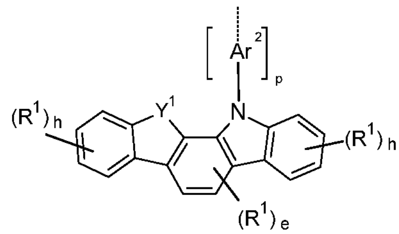

Formula (H-9)

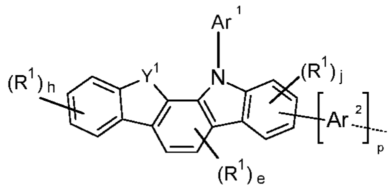

Formula (H-10)

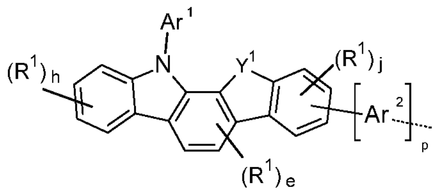

Formula (H-11)

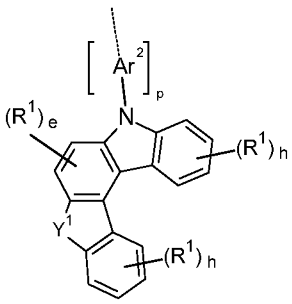

Formula (H-12)

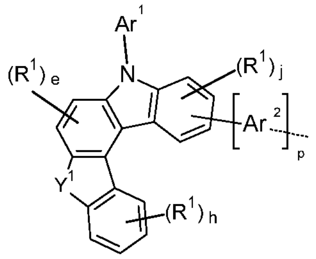

Formula (H-13)

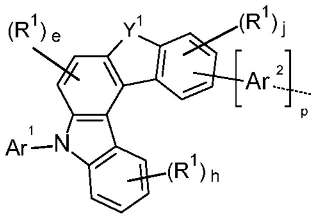

Formula (H-14)

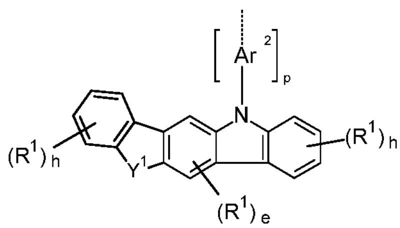

Formula (H-15)

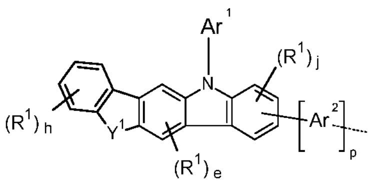

Formula (H-16)

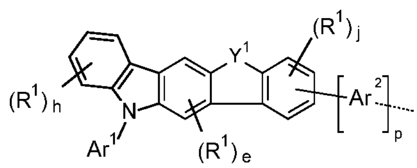

Formula (H-17)

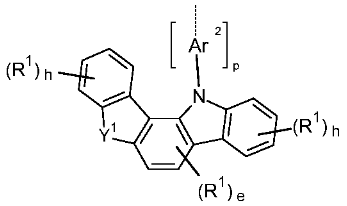

Formula (H-18)

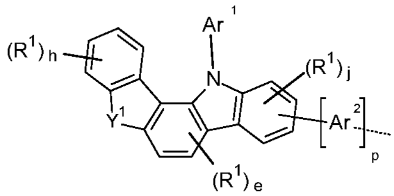

Formula (H-19)

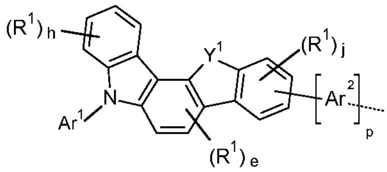

Formula (H-20)

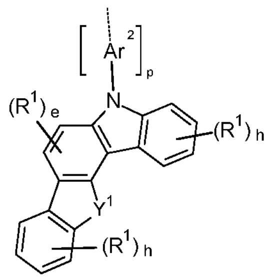

Formula (H-21)

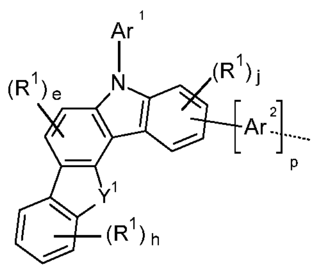

Formula (H-22)

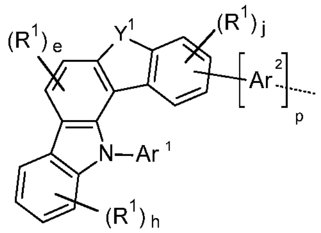

Formula (H-23)

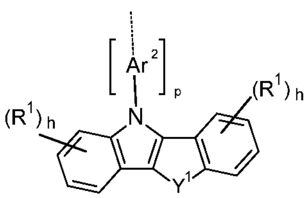

Formula (H-24)

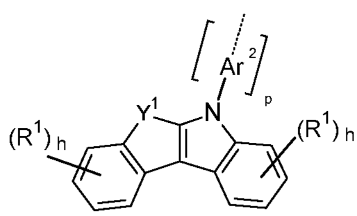

Formula (H-25)

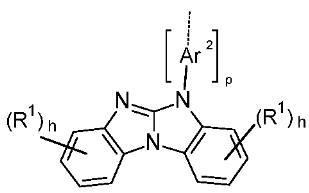

Formula (H-26)

where $Y^1$ represents 0, S, $C(R^1)_2$ or $NAr^1$, the dotted bond marks the position of attachment, e is 0, 1 or 2, j is 0, 1, 2 or 3, h is 0, 1, 2, 3 or 4, p is 0 or 1, $Ar^1$ and $R^1$ have the definition given in Claim 1 and $Ar^2$ has that given in Claim 6, where the presence of an N-N bond is ruled out, such that, in the case that Y = NAr in the formulae (H-5), (H-6), (H-9), (H-12), (H-15), (H-18), (H-21), (H-24), (H-25) and (H-26), the index p = 1.

**8.** Compound according to at least one of the preceding claims, **characterized in that** the compound comprises an electron transport group, it being preferable that the Ar group present in a Y group comprises and preferably represents an electron transport group.

**9.** Compound according to Claim 8, **characterized in that** the Ar group present in a Y group, or an $R^1$ group bonded to the base skeleton, is a group representable by the formula (QL)

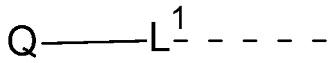

Formula (QL)

in which $L^1$ represents a bond or an aromatic or heteroaromatic ring system which has 5 to 40 aromatic ring atoms and may be substituted by one or more $R^1$ radicals, and Q is an electron transport group, where $R^1$ has the definition given in Claim 1.

**10.** Compound according to Claim 8 or 9, **characterized in that** the electron transport group is selected from structures of the formulae (Q-1), (Q-2), (Q-3), (Q-4), (Q-5), (Q-6), (Q-7), (Q-8), (Q-9) and/or (Q-10)

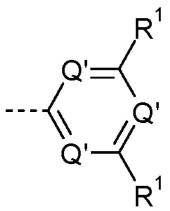

Formula (Q-1)

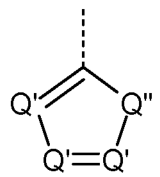

Formula (Q-2)

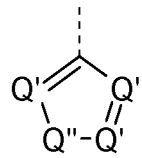

Formula (Q-3)

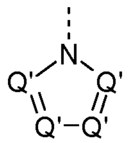

Formula (Q-4)

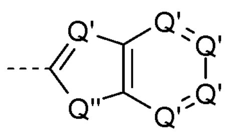

Formula (Q-5)

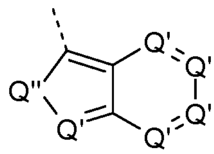

Formula (Q-6)

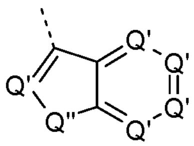

Formula (Q-7)

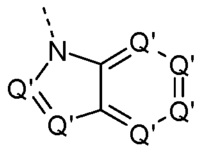

Formula (Q-8)

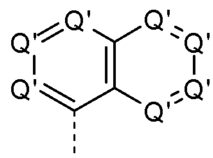

Formula (Q-9)

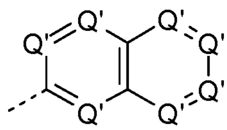

Formula (Q-10)

where the dotted bond marks the position of attachment,

Q' is the same or different at each instance and is $CR^1$ or N, where at least one Q' is N;
Q" is $NR^1$, 0 or S; and
$R^1$ is as defined in Claim 1.

**11.** Oligomer, polymer or dendrimer containing one or more compounds according to any of Claims 1 to 10, wherein, rather than a hydrogen atom or a substituent, there are one or more bonds of the compounds to the polymer, oligomer or dendrimer.

**12.** Composition comprising at least one compound according to one or more of Claims 1 to 10 or an oligomer, polymer or dendrimer according to Claim 11 and at least one further compound selected from the group consisting of fluorescent emitters, phosphorescent emitters, matrix materials, electron transport materials, electron injection materials, hole conductor materials, hole injection materials, electron blocker materials and hole blocker materials.

**13.** Formulation comprising at least one compound according to one or more of Claims 1 to 10 or an oligomer, polymer or dendrimer according to Claim 11 or a composition according to Claim 12 and at least one solvent.

**14.** Use of a compound according to one or more of Claims 1 to 10, of an oligomer, polymer or dendrimer according to Claim 11 or of a composition according to Claim 12 in an electronic device.

**15.** Process for preparing a compound according to one or more of Claims 1 to 10 or an oligomer, polymer and/or dendrimer according to Claim 11, **characterized in that**, in a coupling reaction, a compound comprising at least one nitrogen-containing heterocyclic group is joined to a compound comprising at least one aromatic or heteroaromatic group.

**16.** Electronic device comprising at least one compound according to one or more of Claims 1 to 10, an oligomer, polymer or dendrimer according to Claim 11 or a composition according to Claim 12.

**17.** Electronic device according to Claim 16 which is an organic electroluminescent device, **characterized in that** the compound according to one or more of Claims 1 to 10 or the oligomer, polymer or dendrimer according to Claim 11 is present as matrix material in an emitting layer, as hole transport material or as electron transport material.

**Revendications**

**1.** Composé, comprenant au moins une structure de formule (I),

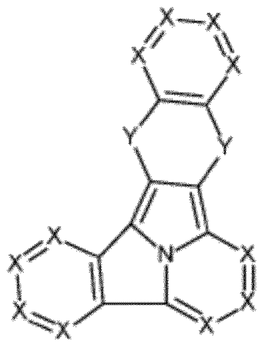

formule (I)

ce qui suit s'appliquant aux symboles utilisés :

Y, identique ou différent en chaque occurrence, est une liaison ou $NR^1$, NAr, 0, S, $C(R^1)_2$, $CArR^1$, $C(Ar)_2$, $Si(Ar)_2$, $SiArR^1$ ou $Si(R^1)_2$, au moins un groupe Y étant choisi parmi $NR^1$, NAr, 0, S, $C(R^1)_2$, $CArR^1$, $C(Ar)_2$, $Si(Ar)_2$, $SiArR^1$ et $Si(R^1)_2$, de préférence parmi $NR^1$ et NAr ;

X, identique ou différent en chaque occurrence, est N ou $CR^1$ ;

Ar, identique ou différent en chaque occurrence, est un système cyclique aromatique ou hétéroaromatique ayant 5 à 60 atomes de cycle aromatique, qui peut être substitué par un ou plusieurs radicaux $R^1$, deux radicaux Ar qui sont liés au même atome de C ou au même atome de Si, peuvent à cet égard également être pontés l'un avec l'autre par une simple liaison ou un pont choisi parmi $B(R^1)$, $C(R^1)_2$, $Si(R^1)_2$, C=O, $C=NR^1$, $C=C(R^1)_2$, O, S, S=O, $SO_2$, $N(R^1)$, $P(R^1)$ et $P(=O)R^1$ ;

$R^1$, identique ou différent en chaque occurrence, est H, D, OH, $OR^2$, F, Cl, Br, I, CN, $NO_2$, $N(Ar^1)_2$, $N(R^2)_2$,

$C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, un groupe alkyle, alcoxy ou thioalcoxy à chaîne droite comportant 1 à 40 atomes de C ou un groupe alkyle, alcoxy ou thioalcoxy ramifié ou cyclique ayant 3 à 40 atomes de C ou un groupe alcényle ou alcynyle comportant 2 à 40 atomes de C, qui peut à chaque fois être substitué par un ou plusieurs radicaux $R^2$, un ou plusieurs groupes $CH_2$ non adjacents pouvant être remplacés par $-R^2C=CR^2-$, -C≡C-, Si $(R^2)_2$, $Ge(R^2)_2$, $Sn(R^2)_2$, C=O, C=S, C=Se, $C=NR^2$, -C (=0) 0-, $-C(=O)NR^2-$, $NR^2$, $P(=O)(R^2)$, -0-, -S-, SO ou $SO_2$ et un ou plusieurs atomes de H pouvant être remplacés par D, F, Cl, Br, I, Cl ou $NO_2$, ou un système cyclique aromatique ou hétéroaromatique comportant 5 à 40 atomes de cycle aromatique, qui peut à chaque fois être substitué par un ou plusieurs radicaux $R^2$, ou un groupe aryloxy ou hétéroaryloxy comportant 5 à 40 atomes de cycle aromatique, qui peut être substitué par un ou plusieurs radicaux $R^2$, ou un groupe aralkyle ou hétéroaralkyle comportant 5 à 40 atomes de cycle aromatique, qui peut être substitué par un ou plusieurs radicaux $R^2$, ou une combinaison de ces systèmes ; deux radicaux $R^1$, de préférence voisins, ou plus peuvent à cet égard former ensemble un système cyclique ;

$Ar^1$, identique ou différent en chaque occurrence, est un système cyclique aromatique ou hétéroaromatique comportant 5 à 30 atomes de cycle aromatique, qui peut être substitué par un ou plusieurs radicaux $R^2$ non aromatiques, deux radicaux $Ar^1$ qui sont liés au même atome de Si, au même atome de N, au même atome de P ou au même atome de B peuvent à cet égard également être pontés l'un avec l'autre par une simple liaison ou un pont choisi parmi $B(R^2)$, $C(R^2)_2$, $Si(R^2)_2$, C=O, $C=NR^2$, $C=C(R^2)_2$, 0, S, S=O, $SO_2$, $N(R^2)$, $P(R^2)$ et $P(=O)R^2$ ;

$R^2$, identique ou différent en chaque occurrence, est H, D, F, Cl, Br, I, CN, $B(OR^3)_2$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)2$, $OSO_2R^3$, $OR^3$, $S(=O)R^3$, $S(=O)_2R^3$, un groupe alkyle, alcoxy ou thioalcoxy à chaîne droite comportant 1 à 40 atomes de C ou un groupe alkyle, alcoxy ou thioalcoxy ramifié ou cyclique ayant 3 à 40 atomes de C, qui peut à chaque fois être substitué par un ou plusieurs radicaux $R^3$, un ou plusieurs groupes $CH_2$ non adjacents pouvant être remplacés par $-R^3C=CR^3-$, -C≡C-, $Si(R^3)_2$, $Ge(R^3)_2$, $Sn(R^3)_2$, C=O, C=S, $C=NR^3$, -C(=O)O-, $-C(=O)NR^3-$, $NR^3$, $P(=O)(R^3)$, -O-, -S-, SO ou $SO_2$ et un ou plusieurs atomes de H pouvant être remplacés par D, F, Cl, Br, I, Cl ou $NO_2$, ou un système cyclique aromatique ou hétéroaromatique comportant 5 à 40 atomes de cycle aromatique, qui peut à chaque fois être substitué par un ou plusieurs radicaux $R^3$, ou un groupe aryloxy ou hétéroaryloxy comportant 5 à 40 atomes de cycle aromatique, qui peut être substitué par un ou plusieurs radicaux $R^3$, ou une combinaison de ces systèmes ; deux substituants $R^2$, de préférence voisins, ou plus peuvent à cet égard former ensemble un système cyclique ;

$R^3$, identique ou différent en chaque occurrence, est choisi dans le groupe constitué par H, D, F, CN, un radical hydrocarboné aliphatique comportant 1 à 20 atomes de C ou un système cyclique aromatique ou hétéroaromatique comportant 5 à 30 atomes de cycle aromatique dans lequel un ou plusieurs atomes de H peuvent être remplacés par D, F, Cl, Br, I ou CN et qui peut être substitué par un ou plusieurs groupes alkyle comportant à chaque fois 1 à 4 atomes de carbone ; deux substituants $R^3$, de préférence voisins, ou plus peuvent à cet égard former ensemble un système cyclique.

2. Composé selon la revendication 1, comprenant au moins une structure de formule (IIa) ou (IIb),

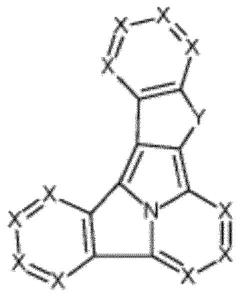

formule (IIa)

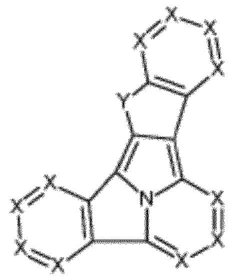

formule (IIb)

les symboles utilisés Y et X présentant la signification mentionnée dans la revendication 1.

**3.** Composé selon au moins l'une des revendications précédentes, comprenant au moins une structure de formule (IIIa), (IIIb), (IVa), (IVb), (Va) ou (Vb),

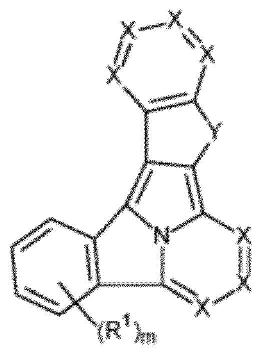

formule (IIIa)

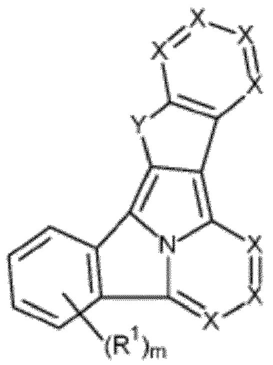

formule (IIIb)

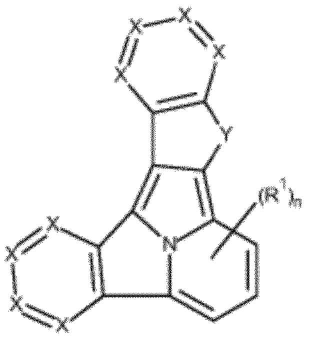

formule (IVa)

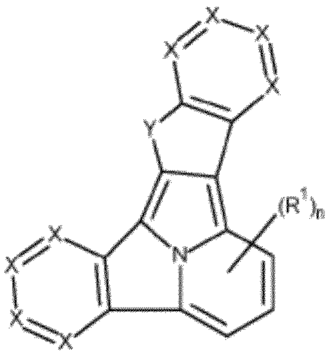

formule (IVb)

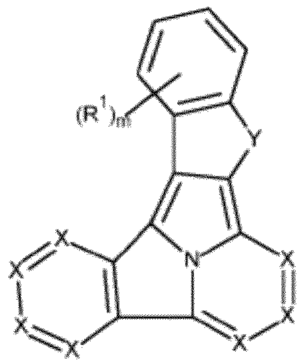

formule (Va)

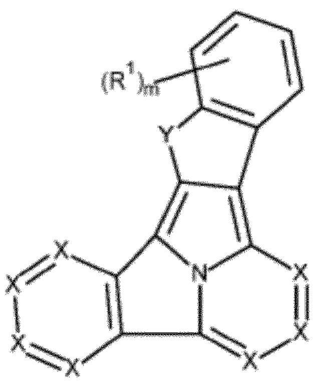

formule (Vb)

les symboles $R^1$, Y et X présentant la signification mentionnée dans la revendication 1, m étant 0, 1, 2, 3 ou 4 et n étant 0, 1, 2 ou 3.

4. Composé selon au moins l'une des revendications précédentes, comprenant au moins une structure de formule (VIa) ou (VIb),

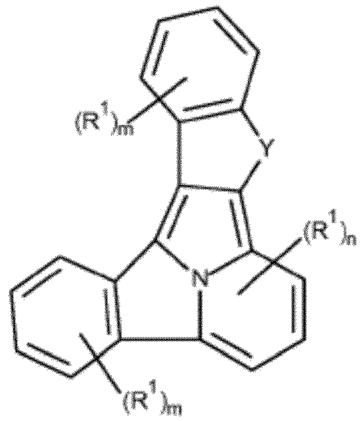

formule (VIa)

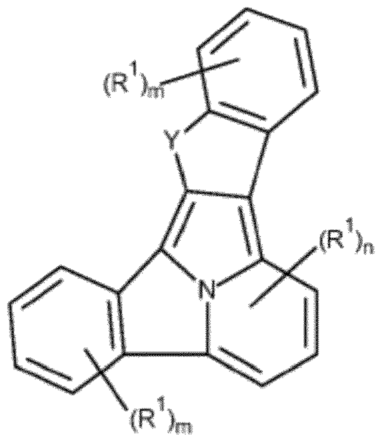

formule (VIb)

les symboles $R^1$ et Y présentant la signification mentionnée dans la revendication 1, m, identique ou différent en chaque occurrence, étant 0, 1, 2, 3 ou 4 et n étant 0, 1, 2 ou 3.

5. Composé selon au moins l'une des revendications précédentes, **caractérisé en ce que** le composé comprend un groupe de transport de trous, de préférence le groupe Ar qui est contenu dans un groupe Y, ou un groupe $R^1$ qui est relié au squelette de base, comprenant, de préférence représentant, un groupe de transport de trous.

6. Composé selon la revendication 5, **caractérisé en ce que** le groupe de transport de trous comprend, préférablement représente un groupe, qui est choisi parmi les formules (H-1) à (H-3),

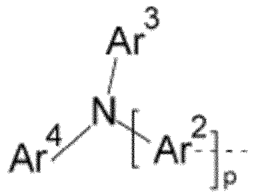

formule (H-1)

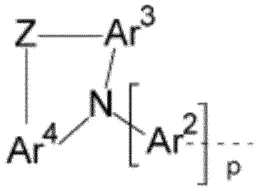

formule (H-2)

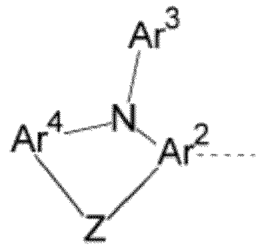

formule (H-3)

la liaison en pointillés marquant la position de connexion et en outre ce qui suit s'appliquant :

$Ar^2$, $Ar^3$, $Ar^4$ sont à chaque fois indépendamment un système cyclique aromatique comportant 6 à 40 atomes de C ou un système cyclique hétéroaromatique comportant 3 à 40 atomes de C, qui peut à chaque fois être substitué par un ou plusieurs radicaux $R^1$ ; p est 0 ou 1 ;
Z représente $C(R^1)_2$, $Si(R^1)_2$, C=O, N-$Ar^1$, $BR^1$, $PR^1$, $POR^1$, SO, $SO_2$, Se, 0 ou S, les symboles $Ar^1$ et $R^1$ présentant la signification mentionnée dans la revendication 1, la présence d'une liaison N-N étant exclue, de sorte que dans le cas où Y = NAr, l'indice p = 1.

**7.** Composé selon la revendication 5 ou 6, **caractérisé en ce que** le groupe de transport de trous comprend un groupe, préférablement représente un groupe, qui est choisi parmi les formules (H-4) à (H-26),

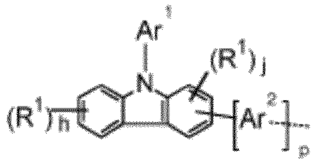

formule (H-4)

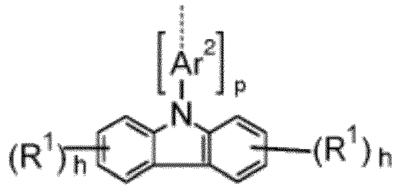

formule (H-5)

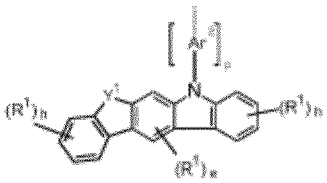

formule (H-6)

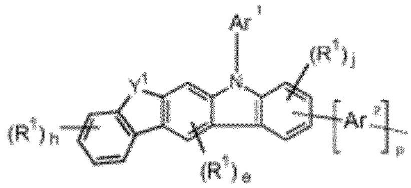

formule (H-7)

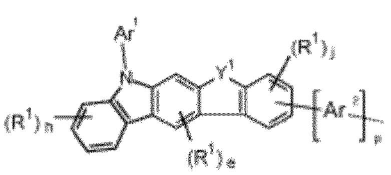

formule (H-8)

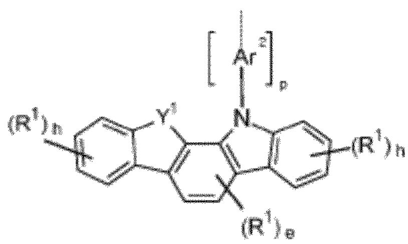

formule (H-9)

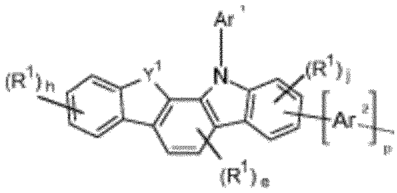

formule (H-10)

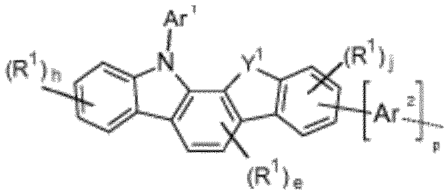

formule (H-11)

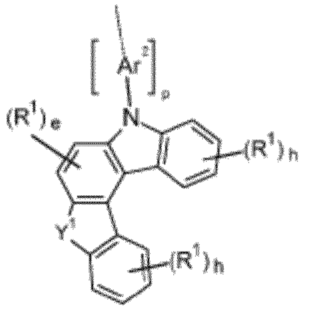

formule (H-12)

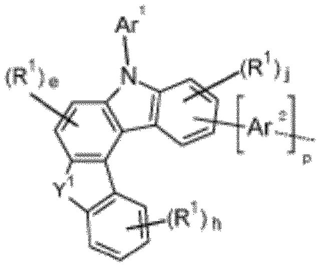

formule (H-13)

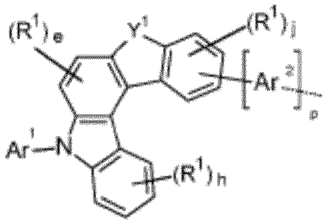

formule (H-14)

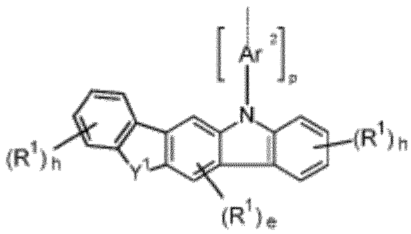

formule (H-15)

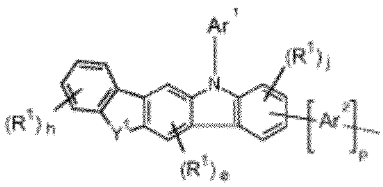

formule (H-16)

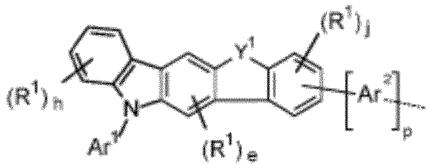

formule (H-17)

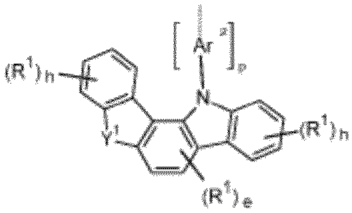

formule (H-18)

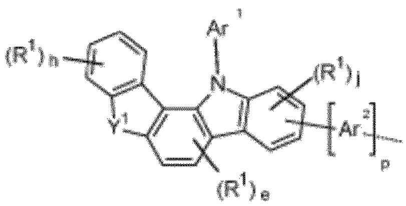

formule (H-19)

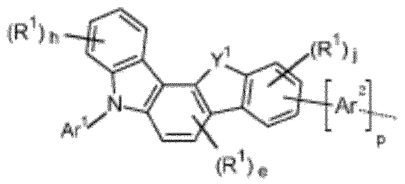

formule (H-20)

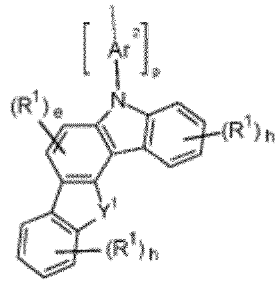

formule (H-21)

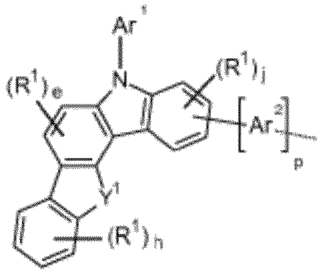

formule (H-22)

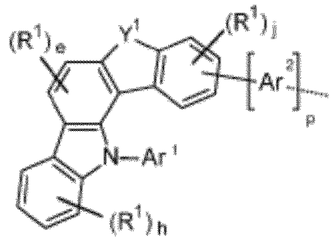

formule (H-23)

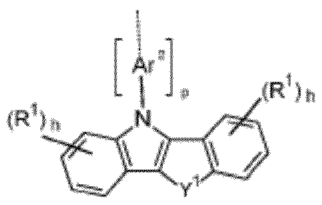

formule (H-24)

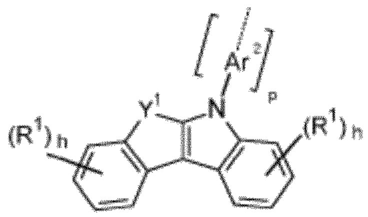

formule (H-25)

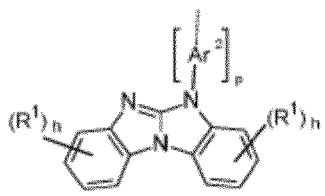

formule (H-26)

$Y^1$ représentant 0, S, $C(R^1)_2$ ou $NAr^1$, la liaison en pointillés marquant la position de connexion, e étant 0, 1 ou 2, j étant 0, 1, 2 ou 3, h étant 0, 1, 2, 3 ou 4, p étant 0 ou 1, $Ar^1$ et $R^1$ présentant la signification mentionnée dans la revendication 1 et $Ar^2$ présentant la signification mentionnée dans la revendication 6, la présence d'une liaison N-N étant exclue, de sorte que dans le cas où Y = NAr dans les formules (H-5), (H-6), (H-9), (H-12), (H-15), (H-18), (H-21), (H-24), (H-25) et (H-26), l'indice p = 1.

8. Composé selon au moins l'une des revendications précédentes, **caractérisé en ce que** le composé comprend un groupe de transport d'électrons, de préférence le groupe Ar qui est contenu dans un groupe Y comprenant, de préférence représentant, un groupe de transport d'électrons.

9. Composé selon la revendication 8, **caractérisé en ce que** le groupe Ar qui est contenu dans un groupe Y, ou un groupe $R^1$ qui est lié au squelette de base, représente un groupe qui peut être représenté par la formule (QL),

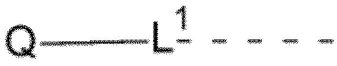

formule (QL)

dans laquelle $L^1$ représente une liaison ou un système cyclique aromatique ou hétéroaromatique comportant 5 à 40 atomes de cycle aromatique qui peut être substitué par un ou plusieurs radicaux $R^1$, et Q est un groupe de transport d'électrons, $R^1$ présentant la signification mentionnée dans la revendication 1.

**10.** Composé selon la revendication 8 ou 9, **caractérisé en ce que** le groupe de transport d'électrons est choisi parmi des structures de formule (Q-1), (Q-2), (Q-3), (Q-4), (Q-5), (Q-6), (Q-7), (Q-8), (Q-9) et/ou (Q-10),

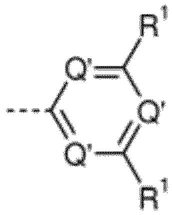

formule (Q-1)

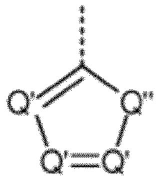

formule (Q-2)

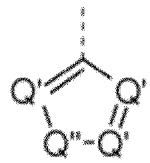

formule (Q-3)

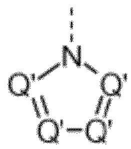

formule (Q-4)

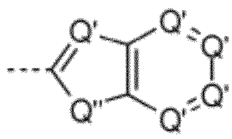

formule (Q-5)

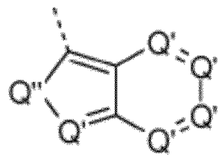

formule (Q-6)

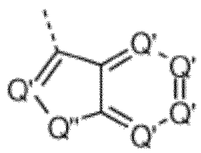

formule (Q-7)

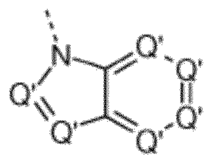

formule (Q-8)

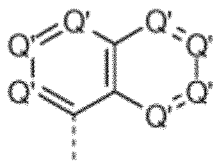

formule (Q-9)

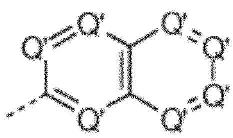

formule (Q-10),

la liaison en pointillés marquant la position de connexion,

Q', identique ou différent en chaque occurrence, représentant $CR^1$ ou N, au moins un Q' étant égal à N ;
Q" représentant $NR^1$, 0 ou S ; et
$R^1$ étant défini comme dans la revendication 1.

**11.** Oligomère, polymère ou dendrimère contenant un ou plusieurs composés selon l'une quelconque des revendications 1 à 10, une ou plusieurs liaisons des composés au polymère, à l'oligomère ou au dendrimère étant présente(s) à la place d'un atome d'hydrogène ou d'un substituant.

**12.** Composition contenant au moins un composé selon l'une ou plusieurs des revendications 1 à 10 ou un oligomère,

un polymère ou un dendrimère selon la revendication 11 et au moins un composé supplémentaire choisi dans le groupe constitué par des émetteurs fluorescents, des émetteurs phosphorescents, des matériaux de matrice, des matériaux de transport d'électrons, des matériaux d'injection d'électrons, des matériaux conducteurs de trous, des matériaux d'injection de trous, des matériaux de blocage d'électrons et des matériaux de blocage de trous.

**13.** Formulation, contenant au moins un composé selon l'une ou plusieurs des revendications 1 à 10 ou un oligomère, un polymère ou un dendrimère selon la revendication 11 ou une composition selon la revendication 12 et au moins un solvant.

**14.** Utilisation d'un composé selon l'une ou plusieurs des revendications 1 à 10, d'un oligomère, d'un polymère ou d'un dendrimère selon la revendication 11 ou d'une composition selon la revendication 12 dans un dispositif électronique.

**15.** Procédé pour la préparation d'un composé selon l'une ou plusieurs des revendications 1 à 10, ou d'un oligomère, d'un polymère et/ou d'un dendrimère selon la revendication 11, **caractérisé en ce qu'**un composé, comprenant au moins un groupe hétérocyclique contenant de l'azote, est relié, dans une réaction de couplage, avec un composé comprenant au moins un groupe aromatique ou hétéroaromatique.

**16.** Dispositif électronique, contenant au moins un composé selon l'une ou plusieurs des revendications 1 à 10, un oligomère, un polymère ou un dendrimère selon la revendication 11 ou une composition selon la revendication 12.

**17.** Dispositif électronique selon la revendication 16, qui est un dispositif d'électroluminescence organique, **caractérisé en ce que** le composé selon l'une ou plusieurs des revendications 1 à 10 ou l'oligomère, le polymère ou le dendrimère selon la revendication 11 est contenu en tant que matériau de matrice dans une couche émettrice, en tant que matériau de transport de trous ou en tant que matériau de transport d'électrons.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2013183327 A
- WO 2005048315 A
- WO 2016149975 A
- KR 20140070450
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016015815 A
- WO 2016124304 A
- WO 2017032439 A
- WO 2018011186 A
- EP 16186313
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- WO 2015192939 A
- WO 2015169412 A
- WO 2016015810 A
- WO 2016023608 A
- WO 2017148564 A
- WO 2017148565 A
- WO 2010108579 A

- WO 2005053051 A
- WO 2009030981 A

**In der Beschreibung aufgeführte Nicht-Patentliteratur**

- *J. Am. Chem. Soc.,* 2004, vol. 126 (51), 16793-16803
- *J. Mater. Chem.,* 2009, vol. 19, 5826-5836
- *Eur. J. Org. Chem.,* 2007, 3718-3726
- *Organic and BioOrganic Chemistry,* 1988, vol. 5, 1209-17
- *Sulfur Letters,* 1998, vol. 21 (5), 199-203
- *Bioorganic & Medicinal Chemistry Letters,* 2007, vol. 17 (11), 3014-3017